# EUROPEAN PATENT APPLICATION

(11) **EP 4 553 079 A1**
(43) Date of publication of application: **14.05.2025**
(21) Application number: 23834800.7
(22) Date of filing: 03.07.2023
(51) Int. Cl.: C07F 5/02, A61K 31/69, A61P 31/04

(54) **CLASS OF 7-((FIVE-MEMBERED HETEROCYCLIC)METHYL)-8-CARBOXYLIC ACID-BENZO CYCLIC BORATE DERIVATIVES AND USES THEREOF**

(30) Priority: 05.07.2022 CN 202210784531; 21.06.2023 CN 202310747738
(71) Applicant: Fuan Pharmaceutical Group Chongqing Sunhorizon Pharm-Tech Co., Ltd, Chongqing 401147 (CN)
(72) Inventor: LI, Guobo, Chengdu, Sichuan 610065 (CN)
(74) Representative: V.O.
(86) International application number: PCT/CN2023/105485
(87) International publication number: WO 2024/008033

(57) **Abstract**

Provided are a class of 7-((five-membered heterocyclic)methyl)-8-carboxylic acid-benzo cyclic borate derivatives represented by formula (I) and uses thereof. The derivative has good broad-spectrum inhibitory activity on clinically common metallo-β-lactamase (MBL) and serine-β-lactamase (SBL), can be used as an inhibitor of MBL and/or SBL, and is used for preparing antibacterial drugs, especially drugs against drug-resistant bacteria. In addition, the derivative is combined with β-lactam antibiotics, has good antibacterial activity on various β-lactam antibiotic-resistant bacteria, has a very great potential in preparing MBL and SBL dual broad-spectrum inhibitors and drugs for overcoming β-lactam antibiotic-resistant bacteria, provides a new choice for the use of the antibacterial drugs, and has a good application prospect.

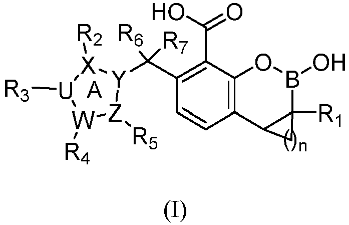

## Description

This application claims the priority of Chinese Patent Application No. 202210784531.9, filed with the China National Intellectual Property Administration on July 05, 2022, and titled with "CLASS OF 7-((FIVE-MEMBERED HETEROCYCLIC)METHYL)-8-CARBOXYLIC ACID-BENZO CYCLIC BORATE DERIVATIVES AND USES THEREOF", and Chinese Patent Application No. 202310747738.3, filed with the China National Intellectual Property Administration on June 21, 2023, and titled with "CLASS OF 7-((FIVE-MEMBERED HETEROCYCLIC)METHYL)-8-CARBOXYLIC ACID-BENZO CYCLIC BORATE DERIVATIVES AND USES THEREOF", which are hereby incorporated by reference in entirety.

### FIELD

The present disclosure relates to the technical field of pharmaceuticals, and in particular to a 7-((five-membered heterocyclic)methyl)-8-carboxylic acid-benzo cyclic borate derivative and use thereof as a metallo-beta-lactamase and serine beta-lactamase inhibitor.

### BACKGROUND

At present, the most widely used antibiotics are β-lactam antibiotics, such as penicillins, cephalosporins, carbapenems, and monocyclics. Among them, carbapenems, as atypical β-lactam antibiotics, have the broadest antimicrobial spectrum and the most potent activity against bacteria, which are important drugs for the treatment of serious bacterial infections in the clinic, and were once known as the "last line of defense" against bacterial infections. However, certainly, the situation regarding β-lactam antibiotic resistance is very serious. In the list of drug-resistant bacteria published by the World Health Organization (WHO) in 2017, carbapenem-resistant Acinetobacter baumannii, Pseudomonas aeruginosa, and Enterobacteriaceae were classified as the most critical superbugs. There is a lack of safe and effective treatment drugs against these drug-resistant bacteria, which have sparked global concern and vigilance. The primary mechanism leading to resistance to β-lactam antibiotics, including carbapenems, is that pathogens themselves produce β-lactamases. These β-lactamases hydrolyze the β-lactam ring, which is the core pharmacophore of these antibiotics, thus causing β-lactam antibiotics to lose their antimicrobial activity. Based on their structure and catalytic mechanism, β-lactamases are classified into metallo-β-lactamases (MBLs) and serine-β-lactamases (SBLs). As drug-resistant genes are exchanged and transferred between the same or different pathogenic bacteria species, drug-resistant bacteria co-expressing MBLs and SBLs continue to appear and spread, posing a significant threat to human health.

The combination of β-lactam antibiotics with β-lactamase inhibitors is one of the most important strategies in clinical practice for treating a disease related to drug-resistant bacterial infections. Currently, 6 serine-β-lactamase (SBL) inhibitors, including clavulanic acid, sulbactam, tazobactam, avibactam, vaborbactam, and relebactam, have been approved for clinical use. These inhibitors have shown good clinical efficacy against SBL-producing resistant bacteria. However, these approved SBL inhibitors are ineffective against metallo-β-lactamase (MBL)-producing resistant bacteria, and there are currently no drugs available clinically to combat MBL-producing resistant bacteria. The dual MBL/SBL inhibitor can block the hydrolysis of β-lactam antibiotics by MBLs and SBLs. Such inhibitors cannot only restore the effectiveness of β-lactam antibiotics against MBL- or SBL-producing resistant bacteria, but also be effective against resistant bacteria that produce both MBLs and SBLs. However, the development of MBL/SBL dual inhibitors presents a significant challenge due to the different structures and catalytic mechanisms of MBLs and SBLs. To date, no such inhibitors have been approved for clinical use. Therefore, there is an urgent need to develop more highly potent and druggable broad-spectrum MBL/SBL dual inhibitors, and to provide candidate compounds for drug development overcoming resistance to β-lactam antibiotics, including carbapenems.

### SUMMARY

One object of the present disclosure is to provide a class of 7-((five-membered heterocyclic)methyl)-8-carboxylic acid-benzo cyclic borate derivatives and use thereof as MBL and SBL inhibitors.

The present disclosure provides a compound represented by formula I, or a salt, a conformational isomer or an optical isomer thereof, 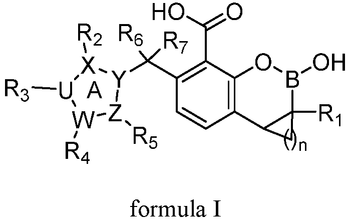 wherein,
n is an integer of 0 to 5;
R₁ is selected from the group consisting of hydrogen, substituted or unsubstituted C₁-C₈ alkyl, C₁-C₈ alkoxy, halogen, hydroxyl, amino, nitro, carboxyl, cyano and SR₉;
A ring is a five-membered unsaturated heterocyclic ring, wherein X, Y, Z, W and U are independently selected from the group consisting of C, CR₈ and N;
R₂, R₃, R₄ and R₅ are substituents on ring A, and R₂, R₃, R₄ and R₅ are independently selected from the group consisting of absence, hydrogen, halogen, hydroxyl, amino, nitro, carboxyl, cyano, amido, quaternary ammonium salt, substituted or unsubstituted C₁-C₈ alkyl, substituted or unsubstituted C₂-C₈ alkynyl, C₁-C₈ alkoxy, substituted or unsubstituted 3- to 8-membered cycloalkyl, substituted or unsubstituted 4- to 8-membered heterocycloalkyl, substituted or unsubstituted 5- to 8-membered aryl, substituted or unsubstituted 5- to 8-membered heteroaryl, and -COR₁₀;
alternatively, any two adjacent positions in R₂, R₃, R₄, and R₅ are connected to form substituted or unsubstituted 3- to 8-membered cycloalkyl, substituted or unsubstituted 4- to 8-membered heterocycloalkyl, substituted or unsubstituted 5- to 8-membered aryl, or substituted or unsubstituted 5- to 8-membered heteroaryl;
R₆ and R₇ are independently selected from the group consisting of hydrogen, substituted or unsubstituted C₁-C₈ alkyl, C₁-C₈ alkoxy, halogen, hydroxyl, amino, nitro, carboxyl, and cyano;
R₈ is selected from the group consisting of hydrogen, halogen, hydroxyl, amino, nitro, carboxyl, cyano, amido, quaternary ammonium salt, substituted or unsubstituted C₁-C₈ alkyl, C₁-C₈ alkoxy, substituted or unsubstituted 3- to 8-membered cycloalkyl, substituted or unsubstituted 4- to 8-membered heterocycloalkyl, substituted or unsubstituted 5- to 8-membered aryl, and substituted or unsubstituted 5- to 8-membered heteroaryl;
R₉ is a five-membered unsaturated heterocyclic group;
R₁₀ is selected from the group consisting of amino, 4- to 8-membered heterocycloalkyl and amino-substituted 4- to 8-membered heterocycloalkyl;
a substituent on the alkyl is selected from the group consisting of halogen, hydroxyl, amino, nitro, carboxyl, cyano, guanidino, quaternary ammonium salt, sulfonamido, and substituted or unsubstituted 4- to 8-membered heterocycloalkyl;
a substituent on the alkynyl, the cycloalkyl, the heterocycloalkyl, the aryl, and the heteroaryl is selected from the group consisting of C₁-C₈ alkyl, C₁-C₈ aliphatic amine group, C₁-C₈ alkoxy, halogen, hydroxyl, amino, nitro, carboxyl, cyano, guanidino, and sulfonamido;
a heteroatom on the heterocycloalkyl and the heteroaryl is selected from the group consisting of N, O and S, and a number of the heteroatom is selected from the group consisting of 1, 2, 3, 4 and 5;
one or more hydrogen atoms on the amino can be further substituted with a substituent selected from the group consisting of C₁-C₈ alkyl, C₁-C₈ aliphatic amine group and sulfonamido.

Further,
n is selected from the group consisting of 0, 1, 2, 3, 4 and 5;
R₁ is selected from the group consisting of hydrogen, C₁-C₄ alkyl, C₁-C₄ alkoxy, halogen, hydroxyl, amino, nitro, carboxyl, cyano and SR₉;
A ring is a five-membered unsaturated heterocyclic ring, wherein X is selected from the group consisting of C, CR₈ and N, Y is N, Z is selected from the group consisting of C, CR₈ and N, W is selected from the group consisting of C and N, and U is selected from the group consisting of C and N;
R₂, R₃, R₄ and R₅ are substituents on ring A, and R₂, R₃, R₄ and R₅ are independently selected from the group consisting of absence, hydrogen, halogen, hydroxyl, amino, nitro, carboxyl, cyano, amido, quaternary ammonium salt, substituted or unsubstituted C₁-C₄ alkyl, substituted or unsubstituted C₂-C₄ alkynyl, C₁-C₄ alkoxy, substituted or unsubstituted 3- to 6-membered cycloalkyl, substituted or unsubstituted 4- to 6-membered heterocycloalkyl, substituted or unsubstituted 5- to 6-membered aryl, substituted or unsubstituted 5- to 6-membered heteroaryl, and -COR₁₀;
alternatively, any two adjacent positions in R₂, R₃, R₄, and R₅ are connected to form substituted or unsubstituted 3- to 6-membered cycloalkyl, substituted or unsubstituted 4- to 6-membered heterocycloalkyl, substituted or unsubstituted 5- to 6-membered aryl, or substituted or unsubstituted 5- to 6-membered heteroaryl;
R₆ and R₇ are independently selected from the group consisting of hydrogen, substituted or unsubstituted C₁-C₄ alkyl, C₁-C₄ alkoxy, halogen, hydroxyl, amino, nitro, carboxyl, and cyano;
R₈ is selected from the group consisting of hydrogen, halogen, hydroxyl, amino, nitro, carboxyl, cyano, amido, quaternary ammonium salt, substituted or unsubstituted C₁-C₄ alkyl, C₁-C₄ alkoxy, substituted or unsubstituted 3- to 6-membered cycloalkyl, substituted or unsubstituted 4- to 6-membered heterocycloalkyl, substituted or unsubstituted 5- to 6-membered aryl, and substituted or unsubstituted 5- to 6-membered heteroaryl;
R₉ is a five-membered unsaturated heterocyclic group, wherein a heteroatom on the heterocyclic group is selected from the group consisting of N, O, S, and a combination thereof.
R₁₀ is selected from the group consisting of amino, 4- to 5-membered heterocycloalkyl and amino-substituted 4- to 5-membered heterocycloalkyl;
a substituent on the alkyl is selected from the group consisting of halogen, hydroxyl, amino, nitro, carboxyl, cyano, guanidino, quaternary ammonium salt, sulfonamido, and substituted or unsubstituted 4- to 6-membered heterocycloalkyl.
a substituent on the alkynyl, the cycloalkyl, the heterocycloalkyl, the aryl, and the heteroaryl is selected from the group consisting of C₁-C₄ alkyl, C₁-C₄ aliphatic amine group, C₁-C₄ alkoxy, halogen, hydroxyl, amino, nitro, carboxyl, cyano, guanidino, and sulfonamido;
a heteroatom on the heterocycloalkyl and the heteroaryl is selected from the group consisting of N, O and S, and a number of the heteroatom is 1 or 2;
one or more hydrogen atoms of the amino can be further substituted with a substituent selected from the group consisting of C₁-C₄ alkyl, C₁-C₄ aliphatic amine group and sulfonamido.

Further, R₉ has a structure of

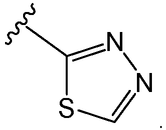

Further, R₁₀ is selected from the group consisting of amino, 4- or 5-membered N-containing heterocycloalkyl, and amino-substituted 4- to 5-membered N-containing heterocycloalkyl.

Further, R₁₀ is selected from the group consisting of amino, 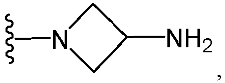 and

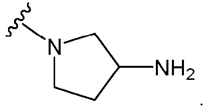

Further, A ring is selected from the group consisting of:

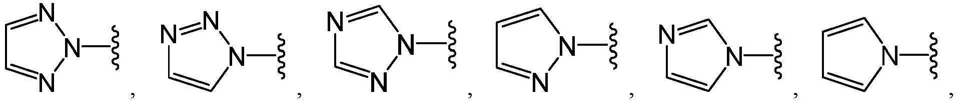

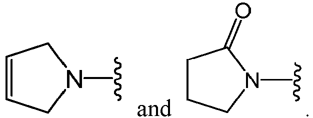

Further, the compound has a structure represented by formula II: 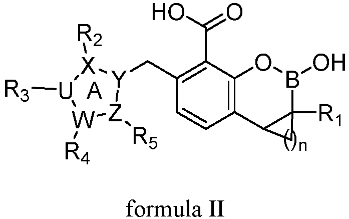 wherein n, A ring, X, Y, Z, W, U, R₁, R₂, R₃, R₄ and R₅ are each selected as defined above.

Further, the compound has a structure represented by formula III: 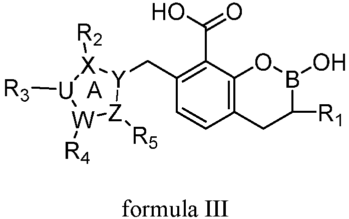 wherein A ring, X, Y, Z, W, U, R₁, R₂, R₃, R₄ and R₅ are each selected as defined above.

Alternatively, the compound has a structure represented by formula IV: 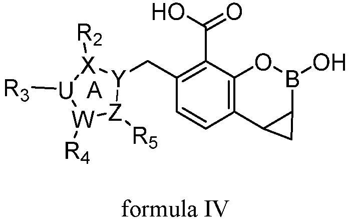 wherein A ring, X, Y, Z, W, U, R₂, R₃, R₄ and R₅ are each selected as defined above.

Further, the compound has a structure represented by formula V: 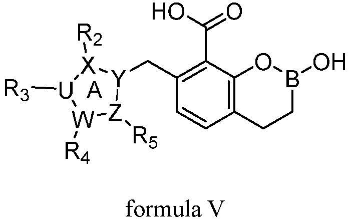 wherein A ring, X, Y, Z, W, U, R₂, R₃, R₄ and R₅ are each selected as defined above.

Further, the compound has a structure represented by formula VI-1: 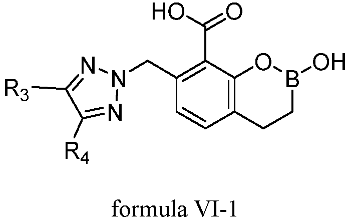 wherein, R₃ and R₄ are each selected as defined above.

Alternatively, the compound has a structure represented by formula VI-2: 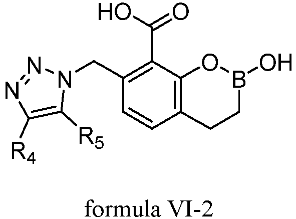 wherein, R₄ and R₅ are each selected as defined above.

Alternatively, the compound has a structure represented by formula VI-3: 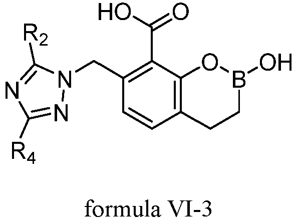 wherein, R₂ and R₄ are each selected as defined in above.

Alternatively, the compound has a structure represented by formula VI-4: 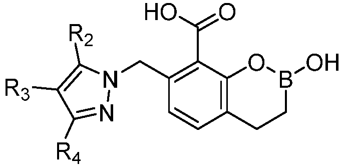 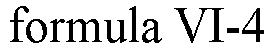 wherein, R₂, R₃ and R₄ are each selected as defined above.

Alternatively, the compound has a structure represented by formula VI-5: 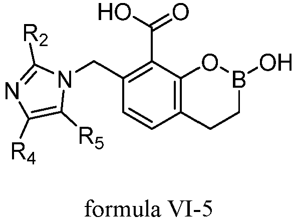 wherein, R₂, R₄ and R₅ are each selected as defined above.

Alternatively, the compound has a structure represented by formula VI-6: 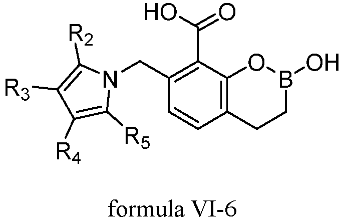 wherein, R₂, R₃, R₄ and R₅ are each selected as defined above.

Alternatively, the compound has a structure represented by formula VI-7:

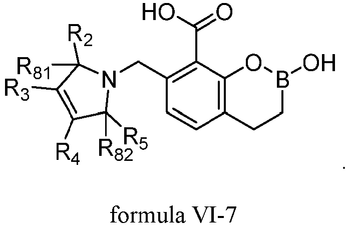

Alternatively, the compound has a structure represented by formula VI-8: 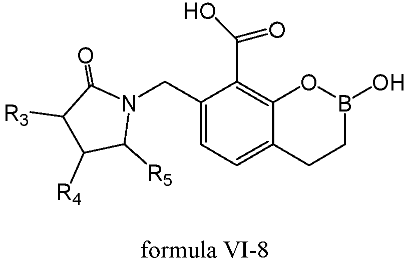
wherein, R₂, R₃, R₄ and R₅ are each selected as defined above;
R₈₁ and R₈₂ are independently selected from the group consisting of hydrogen, halogen, hydroxyl, amino, nitro, carboxyl, cyano, amido, quaternary ammonium salt, substituted or unsubstituted C₁-C₄ alkyl, C₁-C₄ alkoxy, substituted or unsubstituted 3- to 6-membered cycloalkyl, substituted or unsubstituted 4- to 6-membered heterocycloalkyl, substituted or unsubstituted 5- to 6-membered aryl, and substituted or unsubstituted 5- to 6-membered heteroaryl;
a substituent on the alkyl is selected from the group consisting of halogen, hydroxyl, amino, nitro, carboxyl, cyano, guanidino, quaternary ammonium salt, and substituted or unsubstituted 4- to 6-membered heterocycloalkyl;
a substituent on the cycloalkyl, the heterocycloalkyl, the aryl, and the heteroaryl is selected from the group consisting of C₁-C₄ alkyl, C₁-C₄ alkoxy, halogen, hydroxyl, amino, nitro, carboxyl, and cyano;
a heteroatom on the heterocycloalkyl and the heteroaryl is selected from the group consisting of N, O and S, and a number of the heteroatom is 1 or 2.

Further, any two adjacent positions in R₂, R₃, R₄, and R₅ are connected to form substituted or unsubstituted phenyl, tetrahydropyrrolyl, pyridyl or pyrimidinyl.

Further, any two adjacent positions in R₂, R₃, R₄, and R₅ are connected and are fused with A ring to form the following moiety: 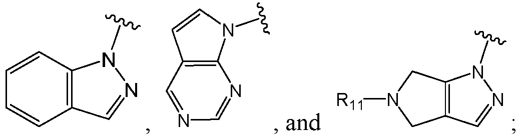
R₁₁ is selected from the group consisting of hydrogen, sulfonamido, amido, C₁-C₈ aliphatic amine group, C₁-C₈ alkyl, and C₁-C₈ alkoxy;
alternatively, R₁₁ and N atom to which it is attached together form guanidino.

Further, R₁₁ is selected from hydrogen, sulfonamido, amide, C₁-C₄ aliphatic amine group, C₁-C₄ alkyl, and C₁-C₄ alkoxy;
alternatively, R₁₁ and N atom to which it is attached together form guanidino.

Further, the compound is selected from the group consisting of:

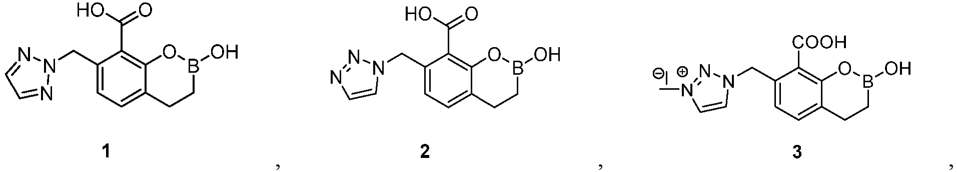

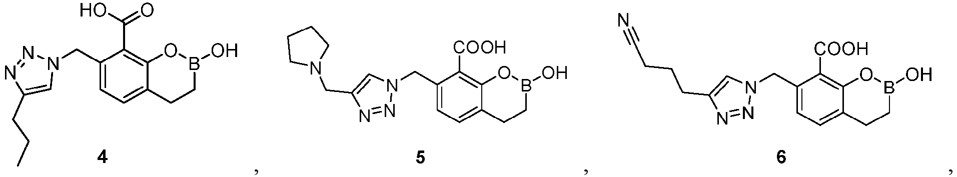

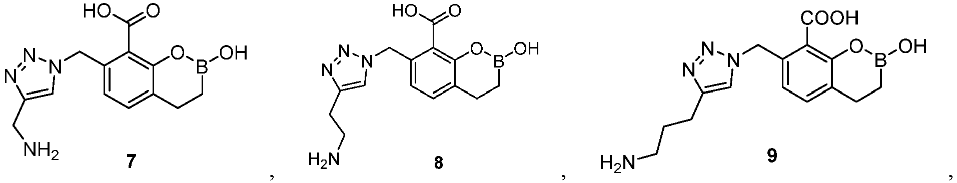

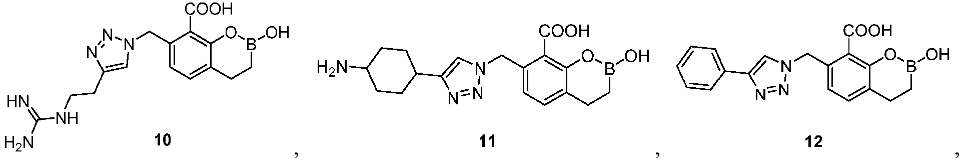

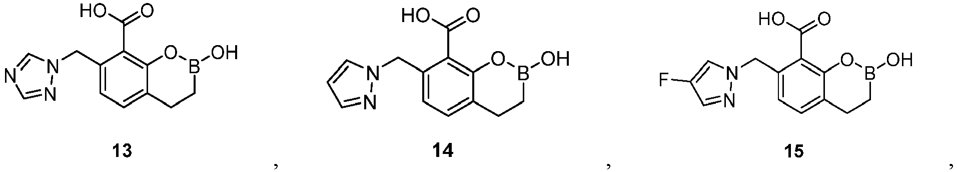

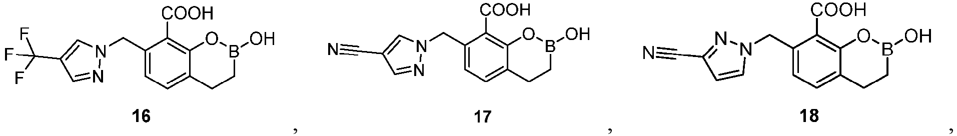

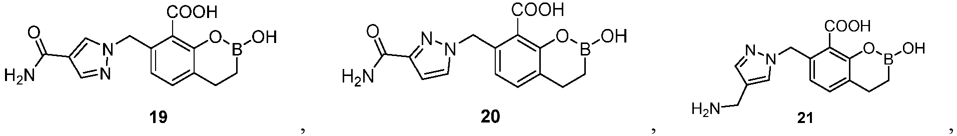

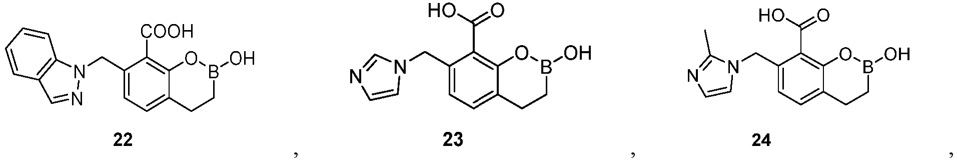

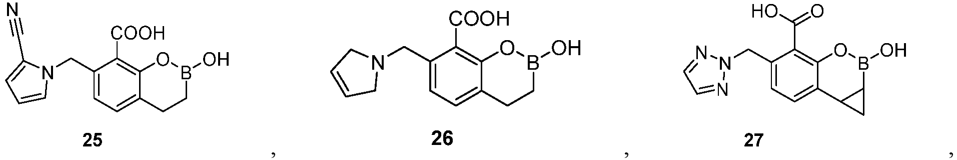

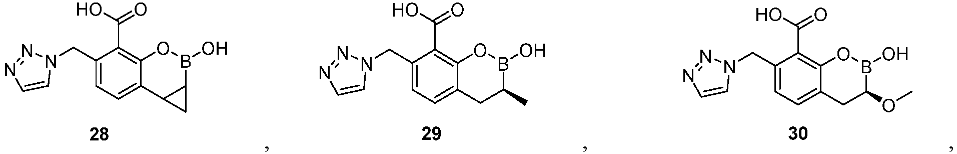

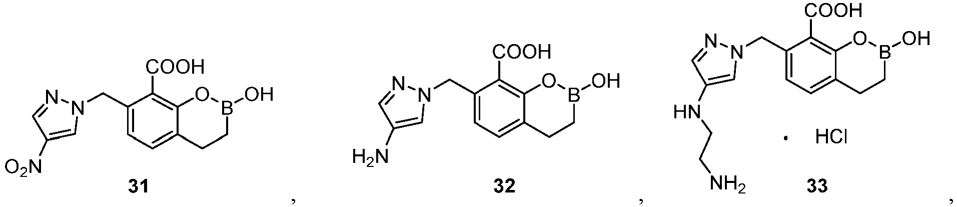

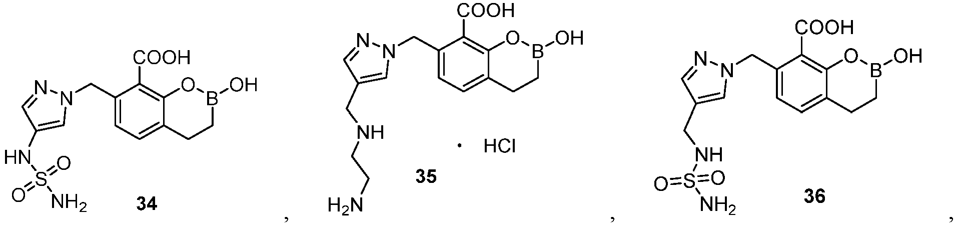

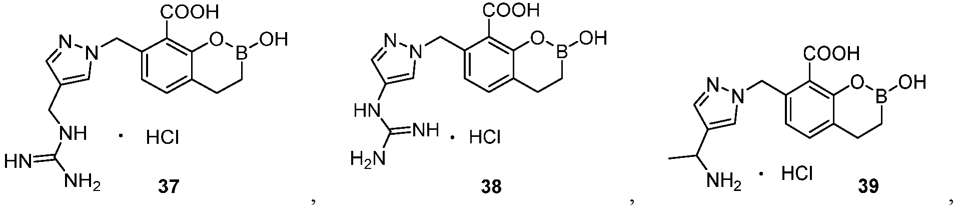

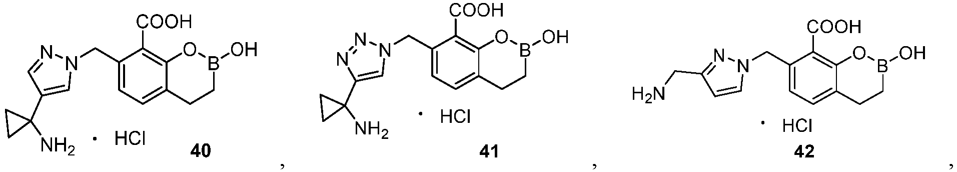

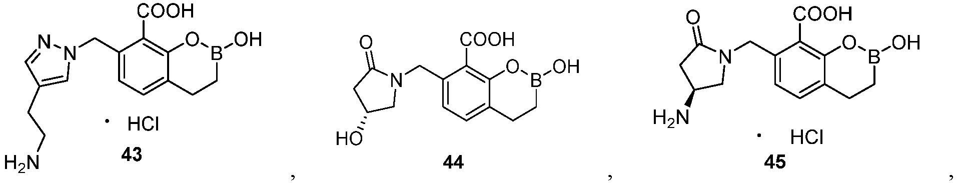

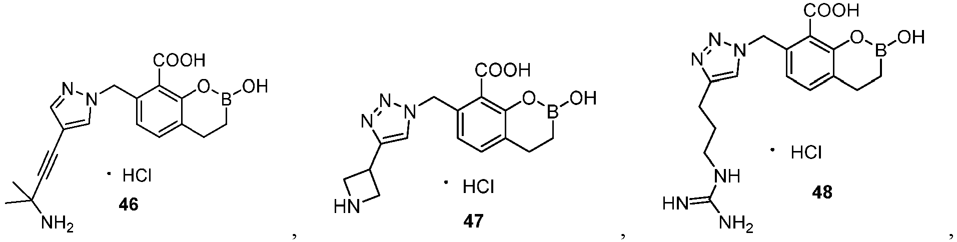

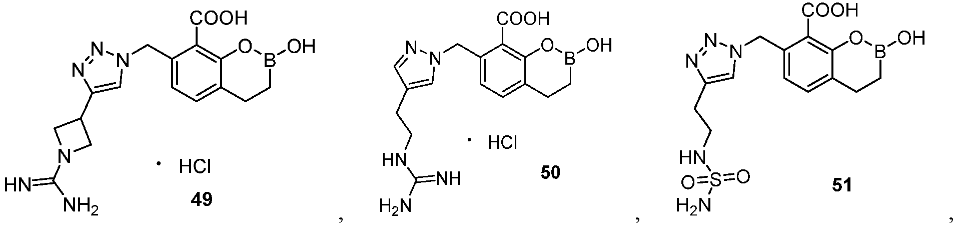

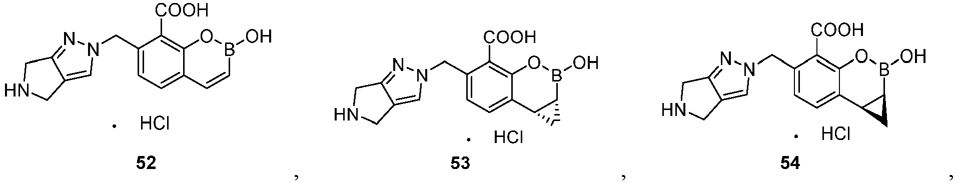

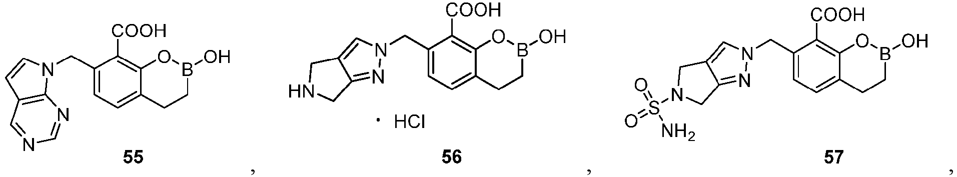

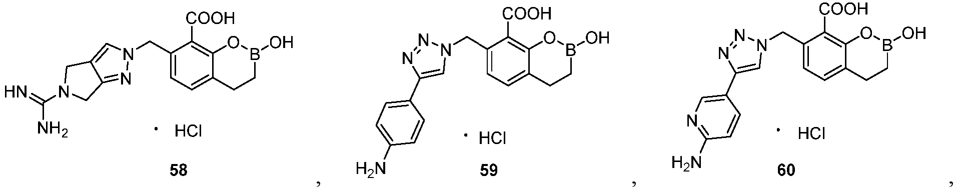

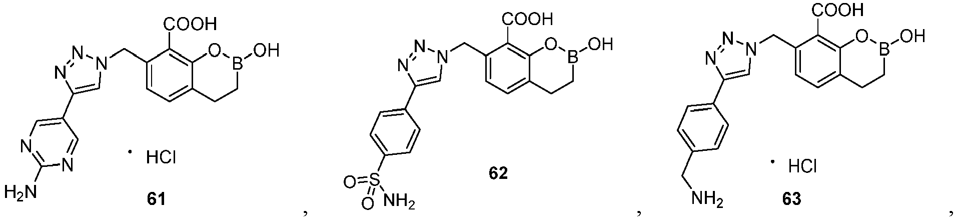

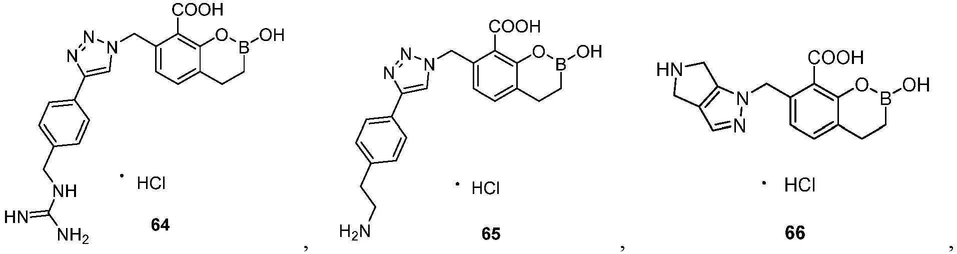

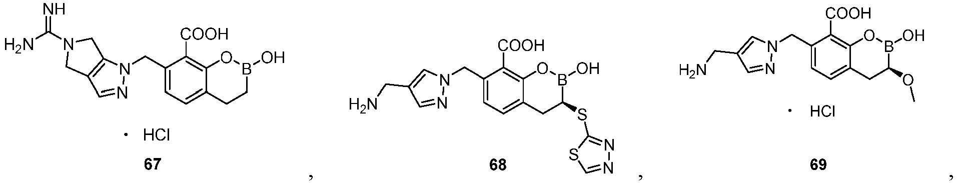

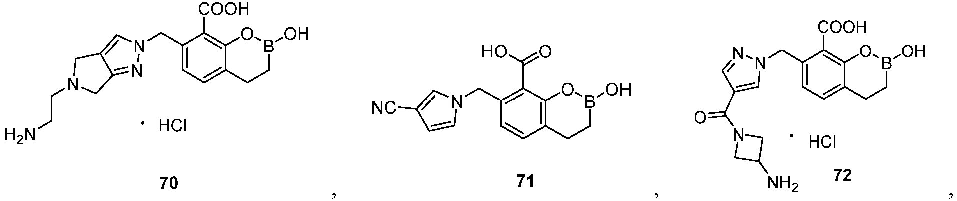

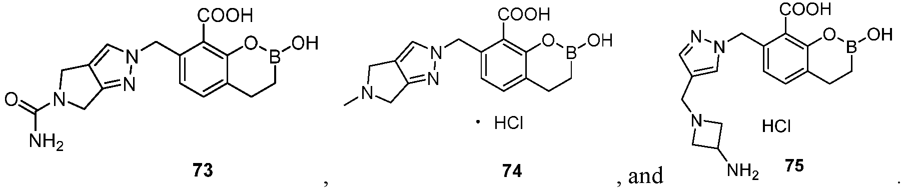

The present disclosure further provides use of the compound, or the salt, the conformational isomer or the optical isomer thereof as described above in the manufacture of a metallo-β-lactamase and/or serine-β-lactamase inhibitor.

The present disclosure further provides use of the compound, or the salt, the conformational isomer or the optical isomer thereof as described above in the manufacture of an antibacterial medicament.

Preferably, the medicament is a medicament against drug-resistant bacteria.

More preferably, the medicament against drug-resistant bacteria is a medicament against β-lactam antibiotic-resistant bacteria.

The present disclosure further provides a medicament, which is a formulation obtained by using the compound, or the salt, the conformational isomer or the optical isomer thereof as described above as an active ingredient, and a pharmaceutically acceptable excipient or an auxiliary ingredient.

The present disclosure further provides a pharmaceutical composition comprising the compound, or the salt, the conformational isomer or the optical isomer thereof as described above, and an antibiotic.

Preferably, the antibiotic is a β-lactam antibiotic.

More preferably, the antibiotic is meropenem.

The present disclosure further provides use of the compound, or the salt, the conformational isomer or the optical isomer thereof as described above in combination with an antibiotic in the manufacture of an antibacterial medicament.

Preferably, the medicament is a medicament against drug-resistant bacteria, and/or the antibiotic is a β-lactam antibiotic.

More preferably, the medicament against drug-resistant bacteria is a medicament against β-lactam antibiotic-resistant bacteria, and/or the antibiotic the group consisting of meropenem, imipenem, a fourth-generation cephalosporin including cefepime, and a combination thereof.

The metallo-β-lactamases include clinically relevant metallo-β-lactamases such as NDM-1, NDM-5, IMP-1, IMP-4, VIM-1 and VIM-2, and the serine β-lactamases include clinically relevant serine β-lactamases such as KPC-2, TEM-1, SHV-12, CTX-M-14, AmpC and OXA-48.

The compounds and derivatives provided in the present disclosure can be named according to the IUPAC (International Union of Pure and Applied Chemistry) or CAS (Chemical Abstracts Service, Columbus, OH) nomenclature system.

Definitions of terms used in the present disclosure: Unless otherwise stated, the initial definitions provided for groups or terms herein apply to the groups or terms throughout the specification; for terms that are not specifically defined herein, the meaning that a person skilled in the art can give them should be given based on the disclosure and context.

The structures of the compounds described in the present disclosure all refer to structures that can exist stably.

As used herein, the term "substitution" refers to the replacement of hydrogen atoms in a molecule with other different atoms or molecules.

As used herein, the minimum and maximum number of the carbon atom in a hydrocarbon group is indicated by a prefix. For example, a prefixed (Cₐ-C_{b})alkyl indicates any alkyl group containing "a" to "b" carbon atoms. Thus, for example, C₁-C₄ alkyl refers to an alkyl group containing 1-4 carbon atoms; and C₁-C₄ alkoxy refers to an alkoxy group containing 1-4 carbon atoms.

As used herein, the term "alkyl" refers to an aliphatic hydrocarbon group, that is, a saturated hydrocarbon group. The alkyl moiety may be a linear alkyl group or a branched alkyl group. Exemplary alkyl groups include, but are not limited to, methyl, ethyl, propyl, isopropyl, butyl, isobutyl, tert-butyl, pentyl, hexyl, and the like.

As used herein, the term "halogen" means fluorine, chlorine, bromine and iodine.

As used herein, the term "amido" includes formamido, acetamido, propionamido and the like.

As used herein, the term "quaternary ammonium salt" refers to

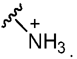

As used herein, the term "cycloalkyl" refers to a saturated or partially saturated non-aromatic cyclic group having 3-8 carbon atoms and no cyclic heteroatoms, and having a single ring or a plurality of rings (including fused ring, bridged ring and spiro ring). Examples of cycloalkyl groups include, for example, adamantyl, cyclopropyl, cyclobutyl, cyclohexyl, cyclopentyl, and cyclooctyl. Examples of cycloalkyl groups containing polydicycloalkyl cyclic system include bicyclohexyl, bicyclopentyl, bicyclooctyl, and the like, and two such dicycloalkyl polycyclic structures are exemplified and named below: 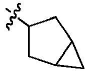 dicyclohexyl and 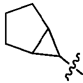 dicyclohexyl.

As used herein, the term "heterocycloalkyl" refers to a saturated or partially saturated non-aromatic cyclic group with a single ring or multiple rings (including fused, bridged, and spiro rings) containing at least one heteroatom; wherein the heteroatom refers to a nitrogen atom, an oxygen atom, or a sulfur atom. Examples of heterocycloalkyl groups include, for example,

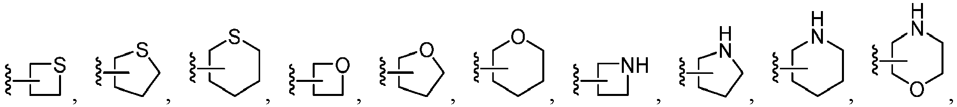

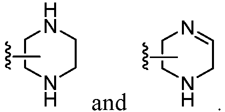

As used herein, "aryl" refers to an aromatic unsaturated group with a single ring or multiple rings (including fused, bridged, and spiro rings) having 5 to 8 carbon atoms and no ring heteroatoms, such as phenyl, anthryl, naphthyl, and

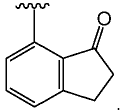

As used herein, "heteroaryl" refers to an aromatic unsaturated ring with a single ring or multiple rings (including fused, bridged, and spiro rings) containing at least one heteroatom; wherein the heteroatom refers to a nitrogen atom, an oxygen atom, and a sulfur atom, for example, furanyl, thienyl, pyrrolyl, pyridinyl, pyrazinyl, pyrazolyl, indazolyl, and further including 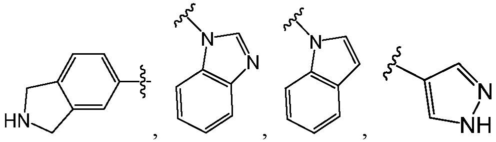 and the like.

As used herein, an unsaturated heterocyclic ring is a heterocyclic ring containing at least one double bond, either a heteroaryl or a heterocycloalkyl.

In a structure of 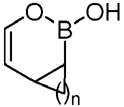 herein, when n is 0, the structure is 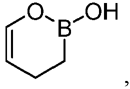 when n is 1, the structure is 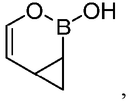 when n is 2, the structure is 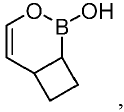 and so on.

As used herein, the salt refers to a pharmaceutically acceptable salt.

The present disclosure provides a class of 7-((five-membered heterocyclic)methyl)-8-carboxylic acid-benzo cyclic borate derivatives. The derivative provided by the present disclosure has a good and broad-spectrum inhibitory activity on clinically common metallo-β-lactamases (MBLs) and serine-β-lactamases (SBLs), and can be used as an inhibitor of MBL and/or SBL for preparing antimicrobial drugs, especially drugs against drug-resistant bacteria. In addition, the derivative of the present disclosure is combined with β-lactam antibiotics, and has good antimicrobial activity against on various β-lactam antibiotic-resistant bacteria. The derivative of the present disclosure has a very great potential in preparing MBL and SBL dual broad-spectrum inhibitors and drugs that overcome β-lactam antibiotic resistant bacteria. The present disclosure provides a new choice for the use of the antimicrobial drugs, and has a good application prospects.

Obviously, according to the above contents of the present disclosure, in accordance with common technical knowledge and customary means in the art, other various forms of modification, replacement or change may be made without departing from the above basic technical ideas of the present disclosure.

The above contents of the present disclosure are further described in detail below through specific embodiments in the form of examples. However, this should not be understood as the scope of the above subject matter of the present disclosure being limited to the following examples. All technologies realized based on the above contents of the present disclosure belong to the scope of the present disclosure.

### DETAILED DESCRIPTION

The raw materials and equipment used in the specific embodiments of the present disclosure are known products, and obtained by purchasing commercially available products.

### 1. Synthesis of key intermediate a5

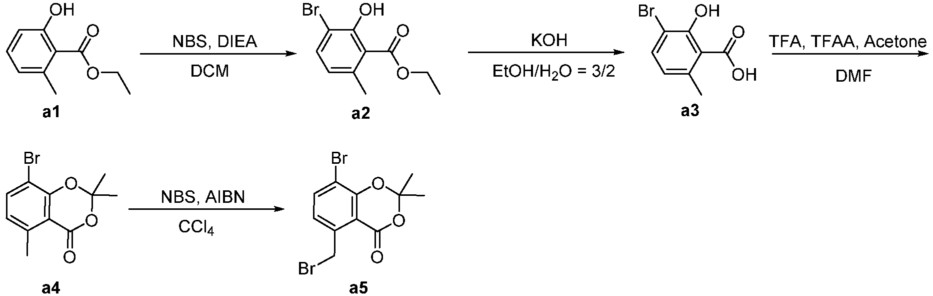

Full names of abbreviations and chemical formulae of the reagents are given as follows: DIEA: N,N-diisopropylethylamine, NBS: N-bromosuccinimide, TFA: Trifluoroacetic acid; TFAA: Trifluoroacetic anhydride, KOH: Potassium hydroxide, DCM: Dichloromethane, EtOH: Ethanol; Acetone: Acetone, DMF: N,N-Dimethylformamide, AIBN: Azodiisobutyronitrile, and CCl₄: Carbon tetrachloride.

The specific synthesis method is described below.

To a solution of raw material a1 (7.2 g, 40 mmol, 1 eq) and DIEA (600 µL, 4 mmol, 0.1 eq) in DCM (50 mL), a solution of NBS (7.12 g, 40 mmol, 1 eq) in DCM (340 mL) was added dropwise at a temperature of 0°C to 5°C maintained by using an ice bath. After the addition was completed, the ice bath was removed and the reaction solution was stirred overnight. Subsequently, the reaction solution was concentrated under reduced pressure and purified by column chromatography (PE/EA = 60:1-30:1, v/v) to obtain intermediate a2 (10.26 g, colorless oil), with a yield of 99%. ¹H NMR (400 MHz, *CDCl₃*) δ 12.05 (s, 1H), 7.53 (d, *J =* 8.1 Hz, 1H), 6.62 (d, *J =* 8.1 Hz, 1H), 4.46 (q, *J =* 7.1 Hz, 2H), 2.52 (s, 3H), 1.44 (t, *J =* 7.1 Hz, 3H). ESI-MS: m/z calcd for C₁₀H₁₁BrO₃ [M+H]⁺ 259.0, found 259.0 and 261.0.

The intermediate a2 (10.36 g, 40 mmol, 1 eq) was dissolved in a mixed solvent of EtOH/H₂O (3/2, v/v, 48 mL) in an eggplant-shaped flask. KOH (8 M, 21.28 g, 380 mmol, 9.5 eq) was added in batches. The reaction mixture was stirred at room temperature until the reaction solution was clear. The reaction solution was heated to 80°C and reacted under reflux for 4 h. After the reaction was completed as monitored by TLC, the reaction solution was cooled to room temperature, and adjusted to pH 3-4 with 2N hydrochloric acid solution. A large amount of white solid was precipitated. The mixture was filtered, and the filter cake was washed with water, collected and dried in a vacuum drying oven to obtain intermediate a3 (8.69 g, white solid), with a yield of 94%.¹H NMR (400 MHz, *DMSO-d₆*) *δ* 7.26 (d, *J =* 8.1 Hz, 1H), 6.30 (d, *J =* 8.0 Hz, 1H), 2.47 (s, 3H).

The intermediate a3 (6 g, 26 mmol, 1 eq) was dissolved in trifluoroacetic acid (36 mL) and DMF (26 mL) in a 250 mL two-necked flask under an ice bath. After replacement with N₂ (×3), acetone (19.5 mL) and TFAA (26 mL) were dropwise added slowly and simultaneously. After the addition was completed, the reaction mixture was placed in an oil bath (100°C) and reacted overnight. The reaction solution was cooled to room temperature, adjusted to pH 6-7 with saturated NaHCO₃, and extracted with EA (60 mL×3). The organic layers were combined, washed with saturated NaCl (60 mL×2), dried over anhydrous Na₂SO₄, concentrated under reduced pressure, and subjected to column chromatography (PE/EA = 80:1-10:1, v/v) to obtain intermediate a4 (3.17 g, orange-red solid), with a yield of 45%. 1H NMR (400 MHz, CDCl3) *δ* 7.61 (d, J = 8.2 Hz, 1H), 6.83 (d, J = 8.5 Hz, 1H), 2.64 (s, 3H), 1.75 (s, 6H).ESI-MS: m/z calcd for C₁₁H₁₁BrO₃ [M+H]+ 271.0, found 271.0 and 273.0.

The intermediate a4 (8.14 g, 30 mmol, 1 eq), NBS (8.01 g, 45 mmol, 1.5 eq) and AIBN (8.14 g, 30 mmol, 1 eq) were added to a reaction flask, dissolved in CCl₄ (30 mL), and refluxed at 80°C for 4 h. The reaction system was concentrated under reduced pressure to remove CCl₄, and purified by column chromatography (PE/EA = 300:1-170:1, v/v) to obtain the key intermediate a5 (8.28 g, light yellow solid), with a yield of 80%. ESI-MS: m/z calcd for C₁₁H₁₀BrO₃ [M+H]⁺ 348.9 found 348.9.

### Example 1 Synthesis of target compounds 1, 2 and 13-26

The synthesis route of target compounds 1, 2 and 13-26 are shown below:

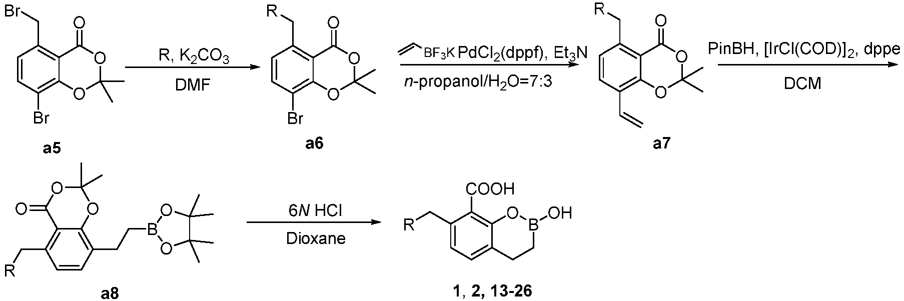

Full names of abbreviations and chemical formulae of the reagents are given as follows: K₂CO₃: potassium carbonate, DMF: N,N- dimethylformamide, pdCl₂ (dppf): [1,1-bis(diphenylphosphine)ferrocene] palladium dichloride, Et₃N: triethylamine, dppe: 1,2-bis (diphenylphosphine)ethane, pinBH: pinacolborane, [IrCl(COD)]₂: chloro(1,5-cyclooctadiene)iridium(I) dimer, DCM: dichloromethane, HCl: hydrochloric acid, and Dioxane: 1,4- dioxane.

### Synthesis and characterization of target compound 1:

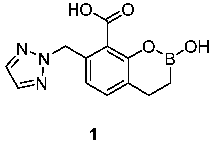

To a solution of 2H-1,2,3-triazole (4.3 mmol, 1.50 eq) in DMF (10 ml), K₂CO₃ (5.74 mmol, 2.0 eq) was added at 0°C under the protection of N₂, and a solution of a5 (2.87 mmol, 1.0 eq) in DMF (10 ml) was slowly added dropwise. After the addition was completed, the reaction system was left at room temperature to give a white suspension, and the white suspension was stirred overnight. After the reaction was completed as monitored by TLC, the reaction mixture was poured into water, and extracted with ethyl acetate (20 ml×3). The organic phase was combined, washed with saturated aqueous NaCl, dried over anhydrous Na₂SO₄, concentrated, and then purified by silica gel column chromatography to obtain intermediate 5-((2*H*-1,2,3-triazol-2-yl)methyl)-8-bromo-2,2-dimethyl-4*H*-benzo[*d*][1,3]dioxin-4-one (a6, 423 mg, white solid), with a yield of 44%. ESI-MS: m/z calcd for C₁₃H₁₃BrN₃O₃ [M+H]⁺ = 338.0, found 338.0 and 340.1.

The intermediate a6 (1.18 mmol, 1.0 eq), potassium trifluoroethylene borate (1.42 mmol, 1.2 eq), and Pd(dppf)₂Cl₂ (0.05 mmol, 0.04 eq) were dissolved in a 20 ml of solvent mixture of n-propanol and water (7:3, v/v). At room temperature, the mixture was replaced with N₂, and Et₃N (1.77 mmol, 1.5 eq) was added. The reaction mixture was heated to 100°C. The multi-phase reaction mixture turned from orange to light amber and slightly turbid when it reached 70°C. After the reaction was completed as monitored by TLC, the orange reaction mixture was cooled to 50°C, and added with water (20 mL) and ethyl acetate (20 mL). The two-phase reaction mixture was cooled to room temperature, filtered and rinsed with ethyl acetate (10 mL×2). The organic layers were washed with water (60 mL), concentrated and purified by column chromatography to obtain 5-((2*H*-1,2,3-triazol-2-yl)methyl)-2,2-dimethyl-8-vinyl-4H-benzo[*d*][1,3]dioxin-4-one (a7, 274 mg, colorless oily compound), with a yield of 81%. ESI-MS: m/z calcd for C₁₅H₁₆N₃O₃ [M+H]⁺ = 286.1, found 286.1.

A solution of [IrCl(cod)]₂ (0.02 mmol, 0.02 eq), dppe (0.04 mmol, 0.04 eq), pinacolborane (1.15 mmol, 1.2 eq), a7 (0.96 mmol, 1 eq) in dichloromethane (10 mL) was added to a two-necked flask under the protection of N₂, and stirred at room temperature for 18 h. After the reaction was completed as monitored by TLC, the reaction mixture was concentrated and purified by column chromatography to obtain 5-(2*H*-1,2,3-triazol-2-yl)methyl)-2,2-dimethyl-8-(2-(4,4,5,5-tetramethyl-1,3,2-dioxobenzaldehyd e-2-yl)ethyl)-4*H*benzo[*d*][1,3]dioxin-4-one (a8, 290 mg), with a yield of 73%. ESI-MS: m/z calcd for C₂₁H₂₉BN₃O₅ [M+H]⁺ = 414.2, found 414.2.

The intermediate a8 (0.70 mmol, 1eq) was dissolved in 10 ml of 1,4-dioxane solution, and 6*N* HCl (10 ml) was added. The reaction system was heated to 100°C and refluxed for 2 h. After the reaction was completed as monitored by TLC, the reaction system was concentrated under reduced pressure in vacuum and the residue was recrystallized to obtain the target compound 1 (83 mg), with a yield of 43%.¹H NMR (400 MHz, *CD₃OD*) *δ* 7.78 - 7.65 (m, 3H), 7.17 (d, *J =* 7.8 Hz, 1H), 5.84 (s, 2H), 2.72 (t, *J =* 7.9 Hz, 2H), 1.07 (t, *J =* 7.9 Hz, 2H). ESI-MS: m/z calcd for C₁₂H₁₃BN₃O₄ [M+H]⁺ = 274.1, found 274.1.

### Synthesis and characterization of target compound 2:

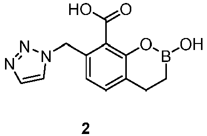

To a solution of 2H-1,2,3-triazole (4.3 mmol, 1.50 eq) in DMF (10 ml), K₂CO₃ (5.74 mmol, 2.0 eq) was added at 0°C under the protection of N₂, and a solution of a5 (2.87 mmol, 1.0 eq) in DMF (10 ml) was slowly added dropwise. After the addition was completed, the reaction system was left at room temperature to give a white suspension, and the white suspension was stirred overnight. After the reaction was completed as monitored by TLC, the reaction mixture was poured into water, and extracted with ethyl acetate (20 ml×3). The organic phase was combined, washed with saturated aqueous NaCl, dried over anhydrous Na₂SO₄, concentrated, and then purified by silica gel column chromatography to obtain 5-((1*H*-1,2,3-triazol-1-yl)methyl)-8-bromo-2,2-dimethyl-4*H*-benzo[*d*][1,3]dioxin-4-one (507 mg, white solid), with a yield of 52%. ESI-MS: m/z calcd for C₁₃H₁₃BrN₃O₃ [M+H]⁺ = 338.0, found 338.0 and 340.1. The remaining three steps of synthesis were the same as those in target compound 1, and the total yield of in the three steps was 19%. ¹H NMR (400 MHz, *CD₃OD*) *δ* 7.76 - 7.64 (m, 3H), 7.13 (d, *J =* 7.4 Hz, 1H), 5.85 (s, 2H), 2.70 (t, *J =* 7.8 Hz, 2H), 1.05 (t, *J* = 7.9 Hz, 2H). ESI-MS: m/z calcd for C₁₂H₁₃BN₃O₄ [M+H]⁺ = 274.1, found 274.0.

### Synthesis and characterization of target compound 13:

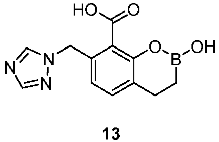

To a solution of 4*H*-1,2,4-triazole (2.25 mmol, 1.5 eq) in DMF (10 ml), K₂CO₃ (3.0 mmol, 2.0 eq) was added at 0°C under the protection of N₂, and a solution of a5 (1.5 mmol, 1.0 eq) in DMF (10 ml) was slowly added dropwise. After the addition was completed, the reaction system was left at room temperature to give a white suspension, and the white suspension was stirred overnight. After the reaction was completed as monitored by TLC, the reaction mixture was poured into water, and extracted with ethyl acetate (20 ml×3). The organic phase was combined, washed with saturated aqueous NaCl, dried over anhydrous Na₂SO₄, concentrated, and then purified by silica gel column chromatography to obtain 5-((1*H*-1,2,4-triazol-1-yl)methyl)-8-bromo-2,2-dimethyl-4*H*-benzo[*d*][1,3]dioxin-4-one (a6, 362 mg, white solid), with a yield of 71%. ESI-MS: m/z calcd for C₁₃H₁₃BrN₃O₃ [M+H]⁺ = 338.0, found 338.0 and 340.1. The remaining three steps of synthesis were the same as those in target compound 1, and the total yield of in the three steps was 11%.¹H NMR (400 MHz, *CD₃OD*) *δ* 7.72 (d, *J =* 1.4 Hz, 2H), 7.12 - 6.90 (m, 2H), 6.09 (s, 2H), 2.64 (t, *J =* 7.2 Hz, 2H), 0.65 (t, *J =* 7.2 Hz, 2H). ESI-MS: m/z calcd for C₁₂H₁₃BN₃O₄ [M+H]⁺ = 274.1, found 274.1.

### Synthesis and characterization of target compound 14:

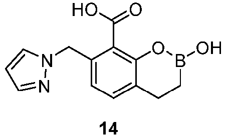

The specific synthesis was referred to the target compound 1, except that 2H-1,2,3-triazole was replaced by 1*H* pyrazole. The total yield of the four steps was 16%. ¹H NMR (400 MHz, *CD₃OD*) *δ* 7.65-7.35 (m, 2H), 7.26 - 7.15 (m, 2H), 6.91 (m, 1H), 6.09 (s, 2H), 2.63 (t, *J =* 7.6 Hz, 2H), 1.02 (t, *J =* 7.6 Hz, 2H). ESI-MS: m/z calcd for C₁₃H₁₃BN₂O₄ [M+H]⁺ = 273.1, found 273.1.

### Synthesis and characterization of target compound 15:

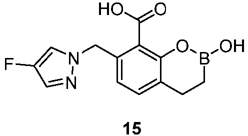

The specific synthesis was referred to the target compound 1, except that 2H-1,2,3-triazole was replaced by 4-fluoro-1*H*-pyrazole. The total yield of the four steps was 21%. ¹H NMR (400 MHz, *CD₃OD*) δ 7.58 (d, *J =* 4.5 Hz, 1H), 7.40 (d, *J =* 4.1 Hz, 1H), 7.18 (dd, *J =* 16.4, 7.6 Hz, 1H), 5.43 (s, 2H), 2.73 (t, *J =* 7.9 Hz, 2H), 1.08 (t, *J =* 7.9 Hz, 2H). ESI-MS:m/z calcd for C₁₃H₁₃BFN₂O₄ [M+H]⁺ = 291.1, found 291.2.

### Synthesis and characterization of target compound 16:

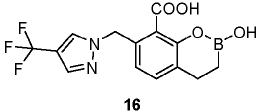

The specific synthesis was referred to the target compound 1, except that 2*H*-1,2,3-triazole was replaced by 4-(trifluoromethyl)-1*H*-pyrazole. The total yield of the four steps was 17%.¹H NMR (400 MHz, CD₃OD) *δ* 8.03 (s, 1H), 7.77 (s, 1H), 7.23 (d, *J =* 7.7 Hz, 1H), 6.53 (br s, 1H), 5.56 (s, 2H), 2.74 (t, *J* = 7.9 Hz, 2H), 1.08 (t, *J =* 7.9 Hz, 2H). ESI-MS: m/z calcd for C₁₄H₁₂BFN₂O₄ [M+H]⁺ 341.1, found 341.2; [M+Na]⁺ 363.1, found 363.2.

### Synthesis and characterization of target compound 17:

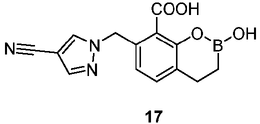

The specific synthesis was referred to the target compound 1, except that 2H-1,2,3-triazole was replaced by 4-cyanopyrazole. The total yield of the four steps was 19%.¹H NMR (400 MHz, *CD₃OD*) *δ* 8.00 (s, 1H), 7.75 (s, 1H), 7.22 (d, *J =* 7.8 Hz, 1H), 6.55 (m, 1H), 5.58 (s, 2H), 2.72 (t, *J* = 7.8 Hz, 2H), 1.06 (t, *J* = 7.8 Hz, 2H). ESI-MS: m/z calcd for C₁₄H₁₂BN₃O₄ [M+H]⁺ 298.1, found 298.1.

### Synthesis and characterization of target compound 18:

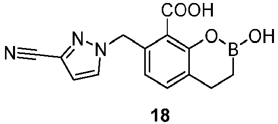

The specific synthesis was referred to the target compound 1, except that 2H-1,2,3-triazole was replaced by 3-cyanopyrazole. The total yield of the four steps was 18%. ¹H NMR (400 MHz, *CD₃OD*) δ 7.82-7.77 (m, 2H), 7.23 (d, *J =* 7.8 Hz, 1H), 6.52 (m, 1H), 5.58 (s, 2H), 2.74 (t, *J* = 7.8 Hz, 2H), 1.08 (t, *J* = 7.8 Hz, 2H). ESI-MS: m/z calcd for C₁₄H₁₂BN₃O₄ [M+H]⁺ 298.1, found 298.1.

### Synthesis and characterization of target compound 19:

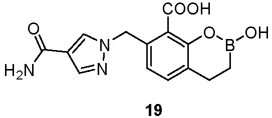

The specific synthesis was referred to the target compound 1, except that 2*H*-1,2,3-triazole was replaced by 1*H*-pyrazole-4-carboxamide. The total yield of the four steps was 15%. ¹H NMR (400 MHz, *CD₃OD*) *δ* 8.83 (s, 1H), 7.82 (s, 1H), 7.79 (s br, 2H), 7.26 (d, *J =* 7.8 Hz, 1H), 6.56 (s br, 1H), 5.56 (s, 2H), 2.74 (t, *J* = 7.9 Hz, 2H), 1.08 (t, *J* = 7.9 Hz, 2H). ESI-MS: m/z calcd for C₁₄H₁₄BN₃O₅ [M+H]⁺ 316.1, found 316.1.

### Synthesis and characterization of target compound 20:

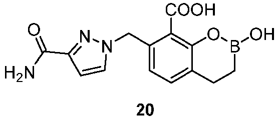

The specific synthesis was referred to the target compound 1, except that 2*H*-1,2,3-triazole was replaced by 1*H*-pyrazole-3-carboxamide. The total yield of the four steps was 16%. ¹H NMR (400 MHz, *CD₃OD*) *δ* 8.22 (s, 2H), 7.83 (m, 1H), 7.25 (d, *J =* 7.8 Hz, 1H), 6.96-6.88 (m, 2H), 5.56 (s, 2H), 2.76 (t, *J =* 7.8 Hz, 2H), 1.09 (t, *J =* 7.8 Hz, 2H). ESI-MS: m/z calcd for C₁₄H₁₄BN₃O₅ [M+H]⁺ 316.1, found 316.1.

### Synthesis and characterization of target compound 21:

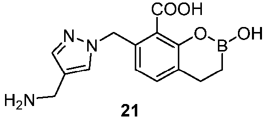

The specific synthesis was referred to the target compound 1, except that 2H-1,2,3-triazole was replaced by 4-(Boc-aminomethyl)pyrazole. The total yield of the four steps was 16%. ¹H NMR (400 MHz, *CD₃OD*) *δ* 7.77 (d, *J =* 9.9 Hz, 2H), 7.65 (d, *J =* 7.4 Hz, 1H), 7.19 (d, *J =* 7.9 Hz, 1H), 5.53 (s, 2H), 4.03 (s, 2H), 2.72 (t, *J =* 7.9 Hz, 2H), 1.06 (t, *J =* 7.9 Hz, 2H). ESI-MS: m/z calcd for C₁₄H₁₆BN₃O₄ [M+H]⁺ 302.1, found 302.1.

### Synthesis and characterization of target compound 22:

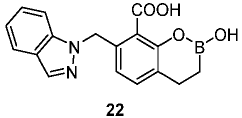

The specific synthesis was referred to the target compound 1, except that 2*H*-1,2,3-triazole was replaced by 1*H*-indazole. The total yield of the four steps was 17%. ¹H NMR (400 MHz, CD₃OD) *δ* 8.08 (d, *J =* 1.0 Hz, 1H), 7.78 (d, *J =* 8.2 Hz, 1H), 7.43 - 7.45 (m, 1H), 7.35 -7.39 (m, 1H), 7.15 - 7.19 (m, 1H), 7.05 (d, *J =* 7.9 Hz, 1H), 5.81 (s, 2H), 2.69 (t, *J=* 7.9 Hz, 2H), 1.05 (t, *J =* 7.9 Hz, 2H). ESI-MS: m/z calcd for C₁₇H₁₅BN₂O₄ [M+H]⁺ 323.1, found 323.4; [M+Na]⁺ 345.1, found 345.1.

### Synthesis and characterization of target compound 23:

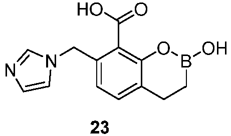

The specific synthesis was referred to the target compound 1, except that 2*H*-1,2,3-triazole was replaced by imidazole. The total yield of the four steps was 18%. ¹H NMR (400 MHz, *CD₃OD*) *δ* 7.82 (s, 1H), 7.25 (d, *J =* 7.8 Hz, 1H), 7.08-6.92 (m, 2H), 6.88 (m, 1H), 5.57 (s, 2H), 2.78 (t, *J* = 7.8 Hz, 2H), 1.08 (t, *J* = 7.8 Hz, 2H). ESI-MS: m/z calcd for C₁₃H₁₃BN₂O₄ [M+H]⁺ 273.1, found 273.1.

### Synthesis and characterization of target compound 24:

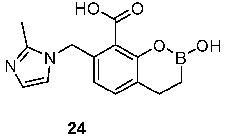

The specific synthesis was referred to the target compound 1, except that 2*H*-1,2,3-triazole was replaced by 2-methyl-1*H*-imidazole. The total yield of the four steps was 14%. ¹H NMR (400 MHz, *CD₃OD*) *δ* 6.99 - 6.77 (m, 4H), 5.31 (s, 2H), 2.65 (t, *J =* 7.0 Hz, 2H), 2.29 (m, 5H). ESI-MS: m/z calcd for C₁₄H₁₆BN₂O₄ [M+H]⁺ = 287.1, found 287.1.

### Synthesis and characterization of target compound 25:

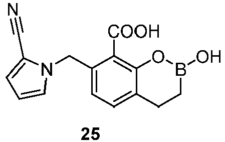

The specific synthesis was referred to the target compound 1, except that 2*H*-1,2,3-triazole was replaced by 1*H*-pyrrole-2-carbonitrile. The total yield of the four steps was 15%. ¹H NMR (400 MHz, *CD₃OD*) *δ* 7.90-7.86 (m, 1H), 7.25 (d, *J =* 7.8 Hz, 1H), 7.06-6.98 (m, 2H), 6.53(m, 1H), 5.56 (s, 2H), 2.78 (t, *J =* 7.8 Hz, 2H), 1.08 (t, *J =* 7.8 Hz, 2H). ESI-MS: m/z calcd for C₁₅H₁₃BN₂O₄ [M+H]⁺ = 297.1, found 297.1.

### Synthesis and characterization of target compound 26:

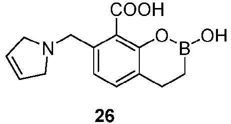

The specific synthesis was referred to the target compound 1, except that 2*H*-1,2,3-triazole was replaced by 2,5-dihydro-1H-pyrrole. The total yield of the four steps was 17%. ¹H NMR (400 MHz, *CD₃OD*) *δ* 7.28 (d, *J* = 7.8 Hz, 1H), 7.06 (d, *J* = 7.8 Hz, 1H), 5.81-5.76 (m, 2H), 3.72 (s, 2H), 3.12-3.07 (m, 4H), 2.73 (t, *J =* 7.8 Hz, 2H), 1.05 (t, *J =* 7.8 Hz, 2H). ESI-MS: m/z calcd for C₁₄H₁₆BNO₄ [M+H]⁺ = 274.1, found 274.1.

### Example 2 Synthesis of target compound 3

### Synthesis and characterization of target compound 3:

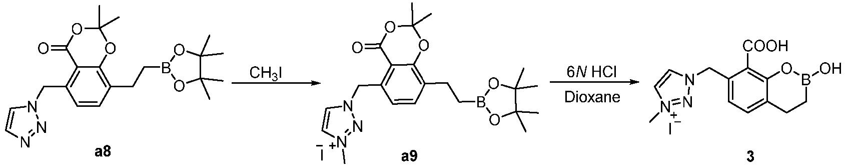

The intermediate a8 (0.44 mmol, 1.0 eq) and iodomethane ( CH₃I, 0.44 mmol, 1.0 eq) were added to a 25 mL capped tube and stirred at 80°C for 24 h. After the reaction was completed as monitored by TLC, the reaction mixture was concentrated to obtain the crude compound a9. ESI-MS: m/z calcd for C₂₂H₃₁BN₃O₅ [M]⁺ = 428.2, found 428.2.

The crude compound a9 was dissolved in 5 mL of 1,4-dioxane solution, and then 6N HCl (5 mL) was added. The mixture was heated to 100°C and refluxed for 2 h. After the reaction was completed as monitored by TLC, the reaction system was concentrated under reduced pressure in vacuum, and subjected to preparative liquid chromatography to obtain the target compound 3 (26 mg), with a yield of 21%. ¹H NMR (400 MHz, *CD₃OD*) *δ* 7.83 - 7.71 (m, 3H), 7.23 (d, *J =* 7.8 Hz, 1H), 5.88 (s, 2H), 4.12 (s, 3H), 2.73 (t, *J =* 7.8 Hz, 2H), 1.08 (t, *J =* 7.8 Hz, 2H). ESI-MS: m/z calcd for C₁₃H₁₅BN₃O₄ [M]⁺ = 288.1, found 288.2.

### Example 3 Synthesis of target compounds 4-12

The synthesis route of target compounds 4-12 was shown below:

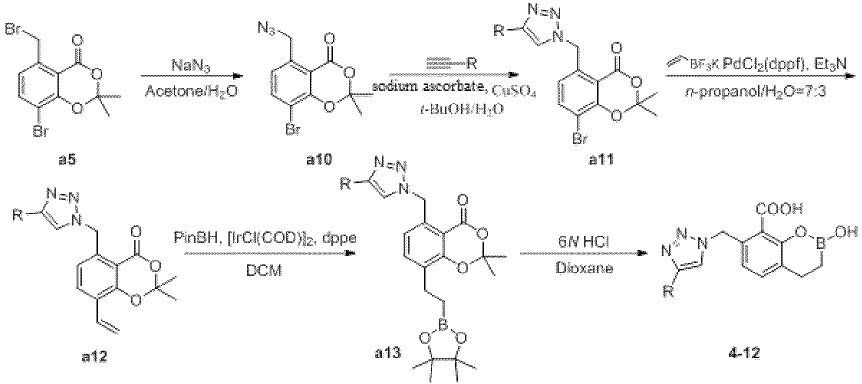

Full names of abbreviations and chemical formulae of some reagents are the same as described above, and the rest are as follows: NaN₃: sodium azide; t-BuOH: tert-butanol.

### Synthesis of intermediate a10

NaN₃ (260 mg, 4 mmol) was added to a mixed solution of intermediate a5 (696 mg, 2 mmol) in acetone (21 ml) and water (7 ml) at 0°C, and reacted at room temperature for 1 h. After the reaction was completed as monitored by TLC, the reaction system was diluted with water and extracted with ethyl acetate. The organic layers were combined, washed with saturated NaCl, and dried over anhydrous Na₂SO₄, and distillated under reduced pressure to obtain the intermediate a10 (light yellow solid, 620 mg, 1.98 mmol), with a yield of 99%. MS calcd for (C₁₉H₂₃BrN₄O₅): 310.0, 313.0; MS (ESI, positive) found: (M-N₂+H⁺): 287.0, 289.0.

### Synthesis and characterization of target compound 4:

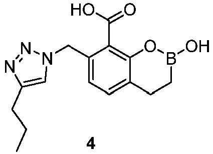

The intermediate a10 (542 mg, 1.74 mmol), 1-pentynyl (324 mg, 1.91 mmol), anhydrous CuSO₄ (27.7 mg, 0.174 mmol), and sodium ascorbate (172 mg, 0.87 mmol) were dissolved in a solvent mixture of tert-butanol (12 ml) and water (12 ml), and reacted overnight at room temperature. After the reaction was completed as monitored by TLC, the reaction system was diluted with water and extracted with ethyl acetate. The organic layers were combined, washed with saturated NaCl, dried over anhydrous Na₂SO₄, concentrated under reduced pressure and purified by silica gel column chromatography to obtain compound a11 (501 mg, 1.32 mmol), with a yield of 76%. MS calcd for (C₁₆H₁₈BrN₃O₃): 379.1, 381.1; MS (ESI, positive) found: (M+ H⁺): 380.1, 382.1

The intermediate all (400 mg, 1.06 mmol), potassium trifluoroethylene borate (171 mg, 1.27 mmol), and Pd(dppf)Cl₂ (31 mg, 0.042 mmol) were dissolved in a mixed solvent of n-propanol (12.5 ml) and water (5 ml). Under nitrogen atmosphere, Et₃N (0.24 ml, 1.59 mmol) was added and the mixture was reacted at 100°C for 5 h. After the reaction was completed as monitored by TLC, the reaction system was cooled down to room temperature, diluted with water and extracted with ethyl acetate. The organic phases were combined, washed with saturated NaCl, dried over anhydrous Na₂SO₄, dried under reduced pressure in vacuum to evaporate solvent, and purified by silica gel chromatography to obtain intermediate a12 (316 mg, 0.97 mmol), with a yield of 91%. MS calcd for (C₁₈H₂₁N₃O₃): 327.2; MS (ESI, positive) found: (M+ H⁺): 328.2.

The intermediate a12 (316 mg, 0.97 mmol), PinBH (0.18 ml, 1.16 mmol), dppe (23.2 mg, 0.066 mmol), and [IrCl(COD)]₂ (19.5 mg, 0.029 mmol) were dissolved in dry dichloromethane (5.2 ml) and reacted under nitrogen at room temperature for 16 h. After the reaction was completed as monitored by TLC, the reaction mixture was concentrated under reduced pressure and purified by silica gel column chromatography to obtain intermediate a13 (356 mg, 0.78 mmol), with a yield of 81%. MS calcd for (C₂₄H₃₄BN₃O₅): 455.3; MS (ESI, positive) found: (M+ H⁺): 456.3

6N HCl (4 ml) was added to the solution of intermediate a13 (149 mg, 0.33 mmol) in dioxane (4 ml), and the mixture was heated to 100°C, and refluxed for 2 h. After the reaction was completed as monitored by TLC, the reaction system was concentrated under reduced pressure in vacuum, and the residue was recrystallized to obtain the target compound 4 (45 mg, 0.14 mmol), with a yield of 43%. ¹H NMR (600 MHz, *CD3OD*) δ 7.83-6.87 (m, 2H), 6.42-5.73 (m, 3H), 2.61 (qm, 4H), 1.79 -1.22 (m, 3H), 0.93 (t, *J =* 9.1 Hz, 3H). MS calcd for (C₁₅H₁₈BN₃O₄): 315.1; MS (ESI, positive) found: (M+ H⁺): 316.1

### Synthesis and characterization of target compound 5:

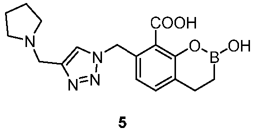

The specific synthesis was referred to the target compound 4, except that 1-pentynyl was replaced by 1-(prop-2-yn-1-yl)pyrrolidine. The total yield of the five steps was 22%. ¹H NMR (600 MHz, *CD₃OD*) δ 7.81 - 6.89 (m, 2H), 6.40 -6.33 (m, 1H), 5.84 (s, 2H), 2.72 (t, *J =* 7.8 Hz, 2H), 2.60-2.45 (m, 4H), 1.71- 1.49 (m, 4H), 0.98 (t, *J* = 7.8 Hz, 2H). MS calcd for (C₁₇H₂₁BN₄O₄): 356.2; MS (ESI, positive) found: (M+ H⁺): 356.2.

### Synthesis and characterization of target compound 6:

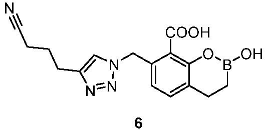

The specific synthesis was referred to the target compound 4, except that 1-pentynyl was replaced by hexyne nitrile. The total yield of the five steps was 20%. ¹H NMR (600 MHz, *CD3OD*) *δ* 7.76 (s, 1H), 7.28- 6.92 (m, 2H), 5.87 (s, 2H), 2.73 (t, *J =* 7.8 Hz, 2H), 2.52-2.46 (m, 2H), 1.99- 1.78 (m, 4H), 0.98 (t, *J =* 7.8 Hz, 2H). MS calcd for (C₁₆H₁₇BN₄O₄): 341.1; MS (ESI, positive) found: (M+ H⁺): 341.1.

### Synthesis and characterization of target compound 7:

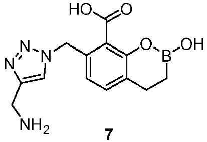

The specific synthesis was referred to the target compound 4, except that 1-pentynyl was replaced by N-Boc-aminopropyne. The total yield of the five steps was 11%. ¹H NMR (400 MHz, *CD₃OD*) *δ* 8.17-7.76 (m, 1H), 7.27 (m, 1H), 6.86 - 6.45 (m, 1H), 6.20-5.68 (m, 2H), 4.30-4.15 (m, 2H), 2.93-2.57(m ,2H), 1.27-0.91(m, 2H); MS (ESI, positive) found: (M+ H⁺): 303.1.

### Synthesis and characterization of target compound 8:

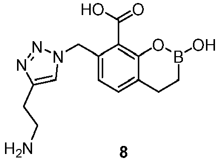

The specific synthesis was referred to the target compound 4, except that 1-pentynyl was replaced by *N*-Boc-3-butyne-1-amine. The total yield of the five steps was 9%. ¹H NMR (400 MHz, *CD₃OD*) *δ* 7.84 (s, 1H), 7.27 (d, *J =* 7.6 Hz, 1H), 6.87-6.67 (m, 1H), 5.76 (m, 2H), 3.28-3.22 (m, 2H), 3.10-3.03 (m, 2H), 2.92-2.57 (m, 2H), 1.28-0.90 (m, 2H). MS calcd for (C₁₄H₁₈N₄O₄⁺): 317.1; MS (ESI, positive) found: (M): 317.1.

### Synthesis and characterization of target compound 9:

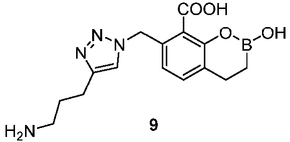

The specific synthesis was referred to the target compound 4, except that 1-pentynyl was replaced by *N*-Boc-4-pentyne-1-amine. The total yield of the five steps was 9%. ¹H NMR (400 MHz, *CD3OD*) *δ* 8.23-7.81 (m, 1H), 7.31 (m, 1H), 6.92-6.56 (m, 1H), 6.20-5.68 (m, 2H), 3.08-2.58 (m, 6H), 2.17-1.92 (m, 2H), 1.23-0.89 (m, 2H). MS: m/z calcd for C₁₅H₂₀BN₄O₄⁺ [M] 331.2, found 331.2.

### Synthesis and characterization of target compound 10:

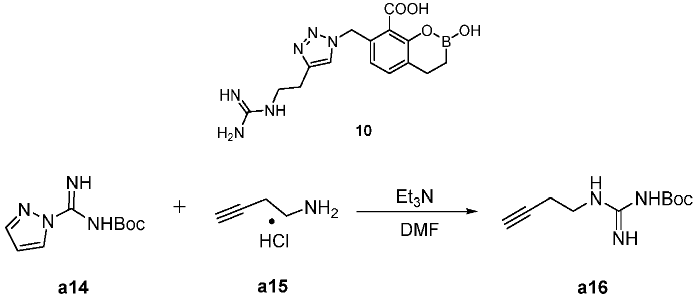

The compounds a14 (1.05 g, 5.0 mmol), and a15 (528 mg, 5.0 mmol) were dissolved in DMF (2.5 ml). Et₃N (1.4 ml, 10.0 mmol) was added, and the mixture was reacted overnight at room temperature. After the reaction was completed as monitored by TLC, the reaction mixture was poured into iced water and extracted with ethyl acetate. The organic layers were combined, washed with saturated NaCl, dried over anhydrous Na₂SO₄, concentrated under reduced pressure and purified by silica gel column chromatography to obtain alkyne intermediate a16 (433 mg, 2.05 mmol, 41%). MS calcd for (C₁₀H₁₇N₃O₂): 211.1, MS (ESI, positive) found: (M+ H⁺): 212.1.

The specific synthesis was referred to the target compound 4, except that 1-pentynyl was replaced by the synthesized intermediate a16. The total yield of the five steps was 8%. ¹H NMR (400 MHz, *CD3OD*) δ 7.89-7.71 (m, 1H), 7.41-7.14 (m, 1H), 6.79-6.44 (m, 1H), 6.10-5.67 (m, 2H), 3.28-3.20 (m, 2H), 3.12-3.02 (m, 2H), 2.91-2.53 (m, 2H), 1.24-0.91 (m, 2H). MS calcd for (C₁₅H₁₉BN₆O₄⁺): 359.2; MS (ESI, positive) found: (M+ H⁺): 359.2.

### Synthesis and characterization of target compound 11:

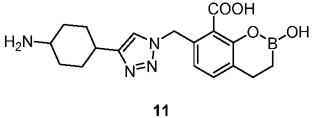

The specific synthesis was referred to the target compound 4, except that 1-pentynyl was replaced by 1-(Boc-amino)-4-ethynylcyclohexane. The total yield of the five steps was 19%. ¹H NMR (400 MHz, *CD3OD*) *δ* 8.21 - 8.00 (m, 1H), 7.40 - 7.20 (m, 1H), 6.91 - 6.54 (m, 1H), 6.18 - 5.76 (m, 2H), 3.26 - 3.10 (m, 1H), 2.99 - 2.59 (m, 3H), 2.20 (m, 4H), 1.69 - 1.51 (m, 4H), 1.24-0.91 (m, 2H). MS calcd for (C₁₈H₂₄BN₄O₄⁺): 331.2; MS (ESI, positive) found: (M+ H⁺): 331.2.

### Synthesis and characterization of target compound 12:

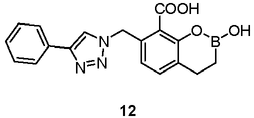

The specific synthesis was referred to the target compound 4, except that 1-pentynyl was replaced by phenylacetylene. The total yield of the five steps was 21%. ¹H NMR (400 MHz, *CD3OD*) δ 7.91 - 7.79 (m, 3H), 7.62 - 7.50 (m, 3H), 7.28 (d, *J =* 7.8 Hz, 1H), 6.87 - 6.79 (m, 1H), 5.88 (s, 2H), 2.78 (t, *J=* 7.8 Hz, 2H), 1.08 (t, *J=* 7.8 Hz, 2H). MS calcd for (C₁₈H₁₆BN₃O₄⁺): 350.1; MS (ESI, positive) found: (M+ H⁺): 350.1.

### Example 4 Synthesis of target compounds 27-28

### The synthesis route of target compound 27 was shown below:

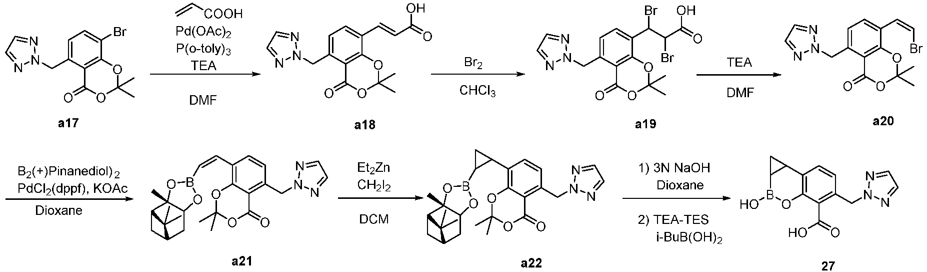

Full names of abbreviations and chemical formulae of the reagents are given as follows: Pd(Oac)₂: palladium acetate, P(o-toly)₃: tris(o-tolyl)phosphine, CHCl₃: trichloromethane, TEA: triethylamine, DMF: N,N-dimethylformamide, B₂((+)pinanediol)₂: bis[(+)-pinanediol], TFA: trifluoroacetic acid, TES: triethylsilane, pdCl₂(dppf): [1,1-bis(diphenylphosphine)ferrocene]palladium dichloride, KOAc: potassium acetate, Dioxane: 1,4-dioxane, Et₂Zn: diethyl zinc, CH₂I₂: diiodomethane, DCM: dichloromethane, NaOH: sodium hydroxide, TFA: trifluoroacetic acid, TES: triethylsilane, and i-BuB(OH)₂: isobutyl boric acid.

At room temperature, intermediate a17 (1.35 g, 4 mmol, 1 eq), acrylic acid (864 mg, 12 mmol, 3 eq), Pd(OAc)₂ (179 mg, 0.8 mmol, 0.2 eq), P(o-toly)₃ (486 mg, 1.6 mmol, 0.4 eq), and Et₃N (1.67 mL, 12 mmol, 3 eq) were dissolved in DMF (150 mL). The mixture was replaced with N₂ (×3). The mixture was placed in an oil bath of 100°C and reacted for 16 h. The reacted solution was cooled to room temperature, and then adjusted to pH 6-7 with 2N hydrochloric acid solution, and extracted with EA (40 mL×3). The organic layers were combined, washed with saturated NaCl (50 mL×2), dried with anhydrous Na₂SO₄, concentrated under reduced pressure, and subjected to column chromatography (DCM/MeOH=200:1-50:1, v/v) to obtain 792 mg of a18 as a brown solid, with a yield of 60%. ESI-MS: m/z calcd for C₁₆H₁₅N₃O₅ [M-H]⁺ 328.1, found 328.1.

The intermediate a18 (658 mg, 2 mmol, 1 eq) was dissolved in CHCl₃ (3 mL), pre-cooled in an ice bath at 0°C for 5 min, and then Br₂ (118 µL, 2.3 mmol, 1.15 eq) was slowly added dropwise. After the addition was completed, the reaction system was stirred at room temperature for 2 h, and concentrated under reduced pressure to obtain a yellow solid, which was directly used in the next step.

The obtained solid after concentration under reduced pressure was dissolved in DMF (2 mL) and pre-cooled in an ice bath at 0°C for 5 min, and then Et₃N (556 µL, 4 mmol, 2 eq) was slowly added dropwise. After the addition was completed, the ice bath was removed, and the reaction system was stirred overnight. After the raw materials were reacted completely, the reaction solution was washed with HCl (0.2 N, 50 mL) and H₂O (30 mL), and extracted with EA (40 mL×3). The organic layers were combined, washed with saturated NaCl (40 mL×2), dried over anhydrous Na₂SO₄, concentrated under reduced pressure, and subjected to column chromatography (PE/EA=10:1-4:1, v/v) to obtain intermediate a20 (473 mg, light yellow solid). ¹H NMR (400 MHz, *CDCl₃*) *δ* 8.05 (d, *J =* 8.2 Hz, 1H), 7.73 (s, 2H), 7.12 (d, *J =* 8.2 Hz, 1H), 6.56 (d, *J =* 8.2 Hz, 1H), 6.20 (s, 2H), 6.15 (d, *J =* 8.4 Hz, 1H), 1.76 (s, 6H)

The intermediates a20 (546 mg, 1.5 mmol, 1.0 eq), bis(1S,2S,3R,5S)(+)-pinanediol diboron ester (823 mg, 2.3 mmol, 1.5 eq), PdCl₂(dppf) (110 mg, 0.15 mmol, 0.1 eq) and KOAc (294 mg, 3 mmol, 2 eq) were dissolved in 1,4-dioxane (5 mL) at room temperature. The mixture was replaced with N₂ (×3), and then heated to 60°C and reacted for 2 h. The reaction system was filtered with diatomaceous earth to remove insoluble substance. The filter cake was washed with EA. The filtrates were combined and extracted with EA (30 mL×2). The organic layers were combined, washed with saturated NaCl (30 mL×2), dried over anhydrous Na₂SO₄, concentrated under reduced pressure, and subjected to column chromatography (PE/EA=10:1-4:1, v/v) to obtain intermediate a21 (312 mg, colorless oil), with a yield of 45%. ESI-MS: m/z calcd for C₂₅H₃₀BN₃O₅ [M+H]⁺ 464.2, found 464.2.

Extra-dry DCM (10 ml) and Et₂Zn (8 mL, 1M in hexanes, 8 mmol, 8.0 eq) were added to a 50 mL two-necked flask, which had been replaced with N₂ (×3). The mixture was transferred to cold hydrazine at -78°C , stirred for 20 min, and slowly added with CH₂I₂ (967 µL, 12 mmol, 12 eq) dropwise. White solid precipitation can be observed after half of the CH₂I₂ was added. After the addition was completed, the reaction system was continuously stirred for 30 min. Subsequently, the intermediate a21 (463 mg, 1 mmol, 1 eq) was dissolved in extra-dry DCM (2 mL), and the resulting solution was slowly added dropwise to the above reaction solution. The resulting reaction system was heated to room temperature, and stirred overnight. After the reaction was completed as monitored by TLC, the reaction solution was quenched by adding saturated NH₄Cl solution (40 mL), and extracted with EA (40 mL×3). The organic layers were combined and washed with saturated NaCl (40 mL×2), dried over anhydrous Na₂SO₄, concentrated under reduced pressure, and subjected to column chromatography (PE/EA (8:1-4:1, v/v) to obtain the intermediate a22 (248 mg, yellow oil), with a yield of 52%. ESI-MS: m/z calcd for C₂₆H₃₂BN₃O₅ [M+H]⁺ 478.3, found 478.3; [M+Na]⁺ 500.2, found 500.3.

The intermediate a22 (248 mg, 0.52 mmol, 1 eq) was dissolved in 1,4-dioxane (3 mL), and a 3N NaOH (3 mL) solution was added. The mixture was stirred at room temperature for 2 h. Subsequently, the reaction solution was transferred to an ice bath, and TES (300 mg), TFA (5 mL) and i-BuB (OH)₂ (150 mg) were added sequentially. The resulting light yellow reaction solution was continued to be stirred at room temperature for 2 h. After the reaction was completed as monitored by TLC, the reaction solution was evaporated to dryness using a rotary evaporator, and subjected to column chromatography (DCM/MeOH, 50:1-10:1, v/v) to obtain the target compound 27 (120 mg, white solid), with a yield of 28%. ¹H NMR (400 MHz, CD₃OD) δ 7.57 (s, 2H), 6.88 (d, *J =* 7.7 Hz, 1H), 5.95 (d, *J =* 7.8 Hz, 1H), 5.72 (d, *J =* 15.7 Hz, 1H), 5.62 (d, *J =* 15.3 Hz, 1H), 1.67 (td, *J =* 8.4, 3.7 Hz, 1H), 0.70 - 0.75 (m, 1H), 0.31 - 0.34 (m, 1H), 0.18 - 0.25 (m, 1H). ESI-MS: m/z calcd for C₁₃H₁₂BN₃O₄ [M+H]⁺ 286.1, found 286.1; [M+Na]⁺ 308.1, found 308.1.

### Synthesis and characterization of target compound 28:

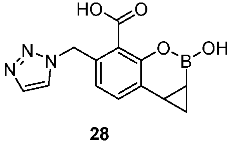

According to the synthesis process of compound 27 as described above, 5-(1*H*-1,2,3-triazol-1-yl)methyl)-8-bromo-2,2-dimethyl-4H-benzo[d][1,3]dioxin-4-one was used as raw material to finally produce compound 28 (82 mg, white solid). ¹H NMR (400 MHz, *CD3OD*) δ 7.97 (br s, 1H), 7.63 (br s, 1H), 6.96 (d, *J =* 7.7 Hz, 1H), 6.37 (d, *J =* 7.7 Hz, 1H), 5.69 (d, *J =* 14.6 Hz, 1H), 5.52 (d, *J =* 14.5 Hz, 1H), 1.72 - 1.77 (m, 1H), 0.78 (ddd, *J =* 10.3, 8.0, 2.6 Hz, 1H), 0.38 - 0.41 (m, 1H), 0.27 - 0.33 (m, 1H). ESI-MS: m/z calcd for C₁₃H₁₂BN₃O₄ [M+H]⁺ 286.1, found 286.1; [M+Na]⁺ 308.1, found 308.1.

### Example 5 Synthesis of target compound 29

### Synthesis and characterization of target compound 29:

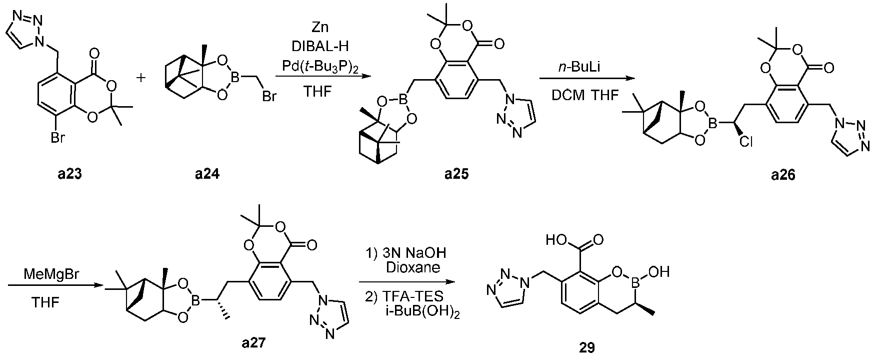

Full names of abbreviations and chemical formulae of the reagents are given as follows: Zn: zinc powder, DIBAL-H: diisobutyl aluminum hydride, Pd(t-Bu3P)₂: bis (tri-tert-butylphosphine)palladium, THF: tetrahydrofuran, n-BuLi: n-butyl lithium, DCM: dichloromethane, MeMgBr: methyl magnesium bromide, NaOH: sodium hydroxide, Dioxane: 1,4-dioxane, TFA: trifluoroacetic acid; TES: triethylsilane, and *i*-BuB(OH)₂: isobutyl boric acid.

Zinc powder (Zn, 2.4 g, 75 mmol) and a24 (50 mg, 0.37 mmol) were added to extra-dry THF (15 mL) and then DIBAL-H (0.5 mL, 0.75 mmol, 1.5 M dissolved in toluene) was added at room temperature, and the mixture was stirred for 5 min. The remaining compound a24 (4.05 g, 15 mmol) was dissolved in extra-dry THF (15 ml), and the resulting solution was slowly added dropwise to the reaction solution. After the addition was completed, the reaction solution was heated to 50°C, stirred for 1 h and cooled to room temperature. The supernatant was added to the reaction solution of intermediate a23 (3.87 g, 11.6 mmol) and Pd(t-Bu₃P )₂ (176 mg, 0.344 mmol) in extra-dry THF (50 mL) under the protection of N₂. The resulting reaction solution was stirred at room temperature for 1 h, concentrated, and subjected to column chromatography (PE/EA, 8:1-4:1, v/v) to obtain a25 as a yellow oily substance (2.35 g), with a yield of 45%. ESI-MS: m/z calcd for C₂₄H₃₀BN₃O₅ [M+H]⁺ 353.2, found 352.2.

To a 50 mL two-necked flask, a25 (2.34 g, 5.18 mmol) dissolved in extra-dry THF (20 mL) and chloroiodomethane (0.49 mL, 6.73 mmol) were added under the protection of N₂, and pre-cooled at -100°C (ethanol/liquid nitrogen system) for 20 min. A solution of n-butyllithium in n-hexane (2.7 mL, 6.73 mmol, 2.5 M) was slowly added dropwise along the flask wall. The entire addition process lasted for 30 minutes. During this process, it could be seen that the reaction solution gradually changed to yellow and then to light yellow. After the addition was completed, the reaction solution was continued to be stirred at -100°C for 30 min. A solution of anhydrous zinc chloride in tetrahydrofuran (5.2 mL, 5.2 mmol, 1 M) was added dropwise along the flask wall. The entire addition process lasted for 30 minutes. After the addition was completed, the reaction was continued to be carried out at -100°C for 30 min, and then the reaction solution was slowly heated to room temperature and reacted overnight. After the reaction was completed as monitored by TLC, the reaction was quenched by adding saturated NH₄Cl solution (40 mL), and extracted with EA (40 mL×3). The organic layers were combined, washed with saturated NaCl (40 mL×2), dried over anhydrous Na₂SO₄, concentrated under reduced pressure, and subjected to column chromatography (PE/EA, 8:1-4:1, v/v) to obtain a26 as a yellow oily substance (1.08 g), with w yield of 41%. ESI-MS: m/z calcd for C₂₅H₃₁BClN₃O₅ [M+H]⁺ 500.1, found 500.1.

Methyl magnesium bromide (1.03 mL, 3.1 mmol, 3 M in THF) was slowly added dropwise to a solution of compound a26 (1.03 g, 2.06 mmol) in extra-dry THF (10 mL) at -78°C, and the reaction mixture was slowly heated to room temperature and stirred for 18 h. After the reaction was completed as monitored by TLC, the reaction was quenched by adding a saturated NH₄Cl solution (40 mL). The reaction solution was extracted with EA (40 mL×3). The organic layers were combined, washed with saturated NaCl (40 mL×2), dried over anhydrous Na₂SO₄, concentrated under reduced pressure, and subjected to column chromatography to obtain a27 as a yellow oily substance (454 mg), with a yield of 46%. ESI-MS: m/z calcd for C₂₆H₃₄BClN₃O₅ [M+H]⁺ 480.2, found 480.2.

The intermediate a27 (249 mg, 0.52 mmol) was dissolved in 1,4-dioxane (3 mL), and a 3N NaOH (3 mL) solution was added, and the mixture was stirred at room temperature for 2 h. Subsequently, the reaction solution was transferred to an ice bath, and TES (300 mg), TFA (5 mL) and *i*-BuB (OH)₂ (150 mg) were added sequentially. The resulting light yellow reaction solution was continued to be stirred at room temperature for 2 h. After the reaction was completed as monitored by TLC, the reaction solution was evaporated to dryness using a rotary evaporator, and subjected to column chromatography (DCM/MeOH, 50:1-10:1, v/v) to obtain the target compound 29 as a white solid (49 mg), with a yield of 33%. ¹H NMR (400 MHz, CD₃OD) *δ* 7.96 (br s, 1H), 7.68 (s, 1H), 6.96 (d, *J =* 7.8 Hz, 1H), 6.37 (d, *J =* 7.8 Hz, 1H), 5.67(d, *J =* 14.6 Hz, 1H), 5.56 (d, *J =* 14.5 Hz, 1H), 2.53-2.45 (m, 2H), 2.01-1.98 (m, 1H), 0.98 (d, *J =* 7.2 Hz, 2H). ESI-MS: m/z calcd for C₁₃H₁₄BN₃O₄ [M+H]⁺ 288.1, found 288.1.

### Example 6 Synthesis of target compound 30

### Synthesis and characterization of target compound 30:

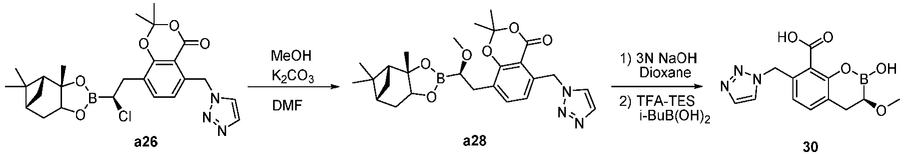

Full names of abbreviations and chemical formulae of the reagents are given as follows: MeOH: methanol, K₂CO₃: potassium carbonate, DMF: N,N-dimethylformamide, NaOH: sodium hydroxide, Dioxane: 1,4-dioxane, TFA: trifluoroacetic acid, TES: triethylsilane, and *i*-BuB(OH)₂: isobutyl boric acid.

At room temperature, MeOH (61 µL, 1.5 mmol) and K₂CO₃ (552 mg, 4 mmol) were sequentially added to a solution of intermediate a26 (500 mg, 1.0 mmol) in DMF (6 mL), and the reaction solution was heated to 50°C and stirred for 1 h. After the reaction was completed as monitored by TLC, the reaction was quenched by using a saturated NH₄Cl solution (40 mL). The reaction solution was extracted with EA (40 mL×3). The organic layers were combined, washed with saturated NaCl (40 mL×2), dried over anhydrous Na₂SO₄, concentrated under reduced pressure, and subjected to column chromatography (PE/EA, 8:1 - 4:1) to obtain the compound a28 (345 mg), with a yield of 70%. ESI-MS: m/z calcd for C₂₆H₃₄BN₃O₆ [M+H]⁺ 496.2, found 496.2.

The compound a28 was used to synthesize the target compound 30 (66 mg, white solid) with a yield of 36% by the synthesis method of compound 29 as described above. ESI-MS: m/z calcd for C₁₃H₁₄BN₃O₅ [M+H]⁺ 304.1, found 304.1.

### Example 7 Synthesis of target compounds 31-75

### Synthesis and characterization of target compound 31:

The specific synthesis was referred to the target compound 1, except that 2*H*-1,2,3-triazole was replaced by 4-nitro-1*H*-pyrazole. The total yield of the four steps was 12%. ¹H NMR (400 MHz, CD₃OD): *δ* 8.52 - 8.39 (m, 1H), 8.18 - 8.05 (m, 1H), 7.30 - 7.20 (m, 1H), 6.73 - 6.35 (m, 1H), 5.86 - 5.46 (m, 2H), 2.90 - 2.56 (m , 2H), 1.11-0.63 (m, 2H).

### Synthesis and characterization of target compound 32:

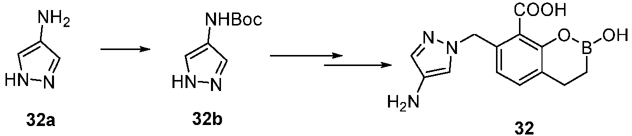

The compound 32a (10 mmol, 830 mg) was dissolved in tetrahydrofuran (THF, 30 mL), di-tert-butyl dicarbonate (11 mmol, 2.4 g) was added, and then saturated sodium bicarbonate solution (5.5 mL) was added to react at room temperature for 24 h. After the reaction was completed as monitored by TLC, the reaction solution was extracted with ethyl acetate, evaporated to remove solvent, triturated with hexane to obtain the intermediate 32b (1.7 g), with a yield of 93%.

Using intermediate 32b (1.5 mmol, 275 mg) as raw material, the compound 32 was synthesized by the synthesis method of target compound 1. ¹H NMR (400 MHz, CD₃OD): δ 7.88 - 7.83 (m, 1H), 7.67 - 7.53 (m, 1H), 7.25 - 7.16 (m, 1H), 6.62 - 6.30 (m, 1H), 5.86 - 5.46 (m, 2H), 3.15 - 2.35 (m, 2H), 1.30 - 0.48 (m, 2H).

### Synthesis and characterization of target compound 33:

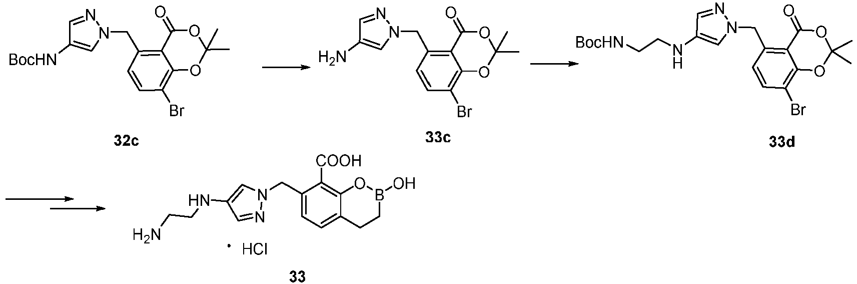

The intermediate 32c (1.2 mmol, 543 mg) was dissolved in dichloromethane (3 mL), and trifluoroacetic acid (3 mL) was added to the react at room temperature for 2 h. After the reaction was completed as monitored by TLC, the reaction solution was evaporated to remove excess solvent to obtain the crude intermediate 33c.

The intermediate 33c and N-Boc-bromoethylamine (1.5 mmol, 248 mg) were dissolved in dichloromethane (2 mL), and triethylamine (2.26 mmol, 0.32 ml) was added to react at 50°C for 2 h. After the reaction was completed as monitored by TLC, the reaction solution was cooled to room temperature, and added to iced water. The reaction solution was extracted with ethyl acetate, evaporated to remove solvent, and subjected to column chromatography to obtain the intermediate 33d (145 mg).

Using intermediate 33d (1.5 mmol, 275 mg) as raw material, the compound 33 was synthesized by the synthesis method of target compound 1. ¹H NMR (400 MHz, CD₃OD): δ 7.92 - 7.82 (m, 1H), 7.77 - 7.66 (m, 1H), 7.37 - 7.17 (m, 1H), 6.84 - 6.28 (m, 1H), 5.93 - 5.48 (m, 2H), 3.62 - 3.45 (m, 2H), 3.28 - 3.21 (m, 2H), 2.88 - 2.55 (m, 2H), 1.25 - 0.86 (m, 2H).

### Synthesis and characterization of target compound 34:

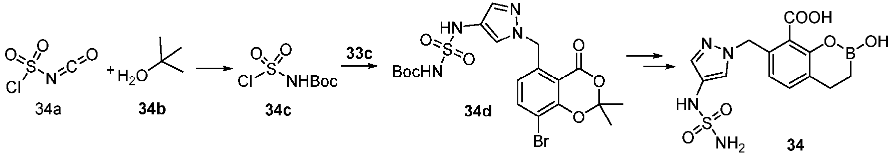

Tert-butanol (1.9 mmol, 0.18 ml) was dissolved in dry dichloromethane (3 mL). The solution was cooled to 0°C, and then chlorosulfonyl isocyanate (1.5 mmol, 0.15 ml) was slowly added. The mixture was continued to react at 0°C for 2 h, and then evaporated to remove solvent to obtain the crude intermediate 34c.

The intermediate 34c and intermediate 33c (1.13 mmol, 528 mg) was dissolved in pyridine (7 mL), and triethylamine (2.26 mmol, 0.32 mL) was added to react overnight at room temperature. After the reaction was completed as monitored by TLC, the reaction solution was evaporated to remove solvent. The residual solids were re-dissolved in dichloromethane, washed with 1N HCl, and then washed with saturated sodium chloride, dried over anhydrous sodium sulfate, evaporated to remove solvent, and then subjected to column chromatography to obtain the intermediate 34d (300 mg), with a yield of 50%.

Using intermediate 34d (0.56 mmol, 300 mg) as raw material, the compound 34 was synthesized by the synthesis method of target compound 1. ¹H NMR (400 MHz, CD₃OD): δ 8.00 - 7.77 (m, 1H), 7.68 - 7.62 (m, 1H), 7.37 - 7.16 (m, 1H), 6.58 - 6.36 (m, 1H), 6.15 - 5.48 (m, 2H), 2.92 - 2.51 (m, 2H), 1.16 - 0.59 (m, 2H).

### Synthesis and characterization of target compound 35:

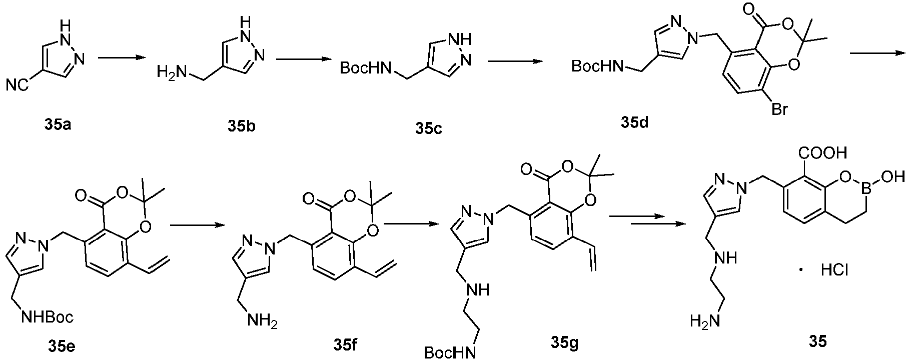

4-Cyanopyrazole (10 mmol, 930 mg) was dissolved in dry tetrahydrofuran (75 mL), and then lithium aluminum hydride (30 mmol, 1.14 g) was added, to react at 75°C under argon atmosphere overnight. After the reaction was completed as monitored by TLC, the reaction solution was cooled to room temperature. The reaction was quenched by adding saturated sodium sulfate. Anhydrous sodium sulfate was added. The reaction solution was then filtered, and evaporated to remove excess solvent to obtain the crude intermediate 35c.

Using intermediate 35c as raw material, the intermediate 35d was prepared by the synthesis method similar to that of intermediate a6, and then 35e was synthesized by the synthesis method of intermediate a7.

The compound 35e (3 mmol, 1.24 mg) was dissolved in DCM (5 mL), and then trifluoroacetic acid (5 mL) was added to react at room temperature for 2 h. After the reaction was completed as monitored by TLC, the reaction solution was evaporated to remove solvent to obtain the crude intermediate 35f.

Using intermediate 35f as raw material, the intermediate 35g (200 mg) was synthesized by the synthesis method similar to that of the intermediate 33d. Using intermediate 35g as raw material, the compound 35 was synthesized by the synthesis method of target compound 1. ESI-MS: [M+H⁺]: 345.2.

### Synthesis and characterization of target compound 36:

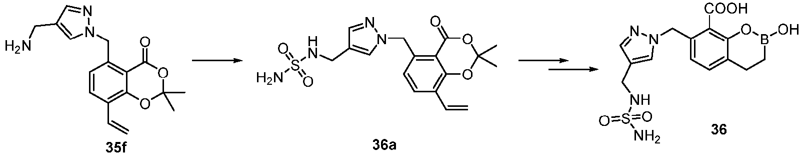

Using intermediate 35f as raw material, the intermediate 36a was synthesized by the synthesis method similar to that of the intermediate 34d. Using intermediate 36a (1.29 mmol, 505mg) as raw material, and using intermediate 36a (1 mmol, 517 mg) as raw material, the target compound 36 (300 mg) was synthesized by the synthesis method similar to that of compound 1. ¹H NMR (400 MHz, CD₃OD): δ 8.18 - 7.62 (m, 2H), 7.45 - 7.11 (m, 1H), 6.89 - 6.20 (m, 1H), 6.01 - 5.44 (m, 2H), 4.22 - 3.96 (m, 2H), 2.93 - 2.49 (m, 2H), 1.28 - 0.61 (m, 2H).

### Synthesis and characterization of target compound 37:

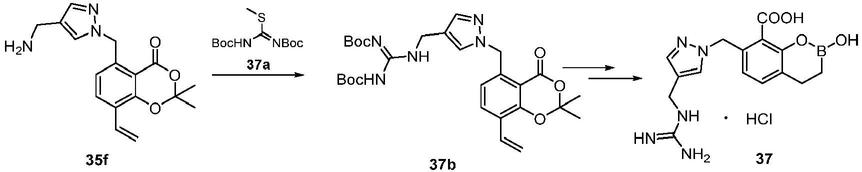

The intermediate 35f and compound 37a (1.95 mmol, 560 mg) were dissolved in tetrahydrofuran (8 mL), and triethylamine (3.9 mmol, 0.57 mL) was added. The mixture was heated to 50°C and reacted overnight. After the reaction was completed as monitored by TLC, the reaction solution was cooled to room temperature, extracted with ethyl acetate and water, washed with saturated sodium chloride, dried over anhydrous sodium sulfate, evaporated to remove solvent, and subjected to column chromatography to obtain the intermediate 37b (144 mg).

Using intermediate 37b (0.26 mmol, 144 mg) as raw material, the intermediate 8d (80 mg, 45% yield) was synthesized by the synthesis method similar to that of the intermediate 1d.

Using intermediate 8d (0.12mmol, 80mg) as raw material, the target compound 37 was synthesized by the synthesis method similar to that of the compound 1. ESI-MS: [M+H⁺]: 344.2.

### Synthesis and characterization of target compound 38:

Using intermediate 33c as raw material, the target compound 38 (50 mg) was synthesized by the synthesis method similar to that of the compound 38. ¹H NMR (400 MHz, CD₃OD): *δ* 8.02 - 7.53 (m, 2H), 7.39 - 7.09 (m, 1H), 6.69 - 6.11 (m, 1H), 5.91 - 5.38 (m, 2H), 3.37 - 3.23 (m, 2H), 2.82 - 2.46 (m, 2H), 1.15 - 0.57 (m, 2H).

### Synthesis and characterization of target compound 39:

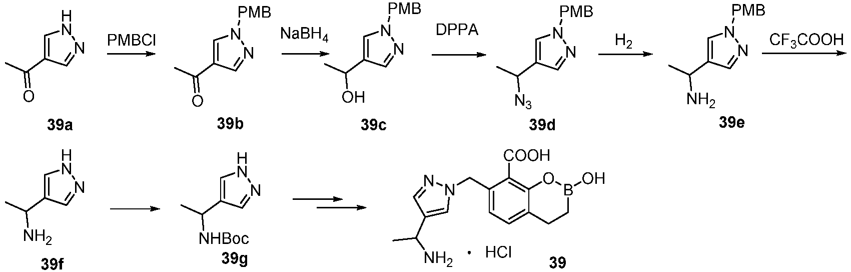

4-Acetamido-1H-pyrrole (10 mmol, 1.1 g) was dissolved in acetonitrile (60 mL), PMBCl (10 mmol, 1.36 mL) and potassium carbonate (20 mmol, 2.76 g) were added. The mixture was heated to 85°C and reacted overnight. After the reaction was completed as monitored by TLC, the reaction solution was cooled to room temperature, extracted with ethyl acetate and water, evaporated to remove solvent, and subjected to column chromatography to obtain the intermediate 39b (2.25 g, 98% yield).

The intermediate 39b (9.8 mmol, 2.25 g) was dissolved in dry methanol (45 mL), cooled to 0°C, and then sodium borohydride (19.6 mmol, 741 mg) was added. The mixture was continued to react at 0°C for 3 h. After the reaction was completed as monitored by TLC, the reaction was quenched by saturated ammonium chloride, extracted with ethyl acetate and water, and evaporated to remove solvent to obtain the intermediate 39c (2 g, 88% yield).

The intermediate 39c (8.61 mmol, 2 g) and DPPA (13 mmol, 2.8 mL) were then dissolved in dry tetrahydrofuran (22 mL), cooled to 0°C, and DBU (13 mmol, 1.97 mL) was added, to react overnight at room temperature under argon atmosphere. After the reaction was completed as monitored by TLC, the reaction solution was extracted with ethyl acetate and water, evaporated to remove solvent, and subjected to column chromatography to obtain the intermediate 39d (1.3 g, 59% yield).

The intermediate 39d (5.1 mmol, 1.3 g) was dissolved in methanol, and 10% Pd/C (300 mg) was added, to react overnight under hydrogen atmosphere. After the reaction was completed as monitored by TLC, the reaction solution was filtered, and evaporated to remove excess solvent to obtain the crude intermediate 39e.

The intermediate 39e was dissolved in trifluoroacetic acid (30 mL), and anisole (1 mL) was added. The mixture was heated to 95°C and reacted for 5h, and then cooled to room temperature, and evaporated to remove solvent to obtain the crude intermediate 39f.

Using intermediate 39f as raw material, the intermediate 39g (750 mg) was synthesized by the synthesis method similar to that of intermediate 35c. Using intermediate 39g (3.55 mmol, 750 mg) as raw material, the target compound 39 (100 mg) was synthesized by the synthesis method similar to that of compound 1. ¹H NMR (400 MHz, CD₃OD): δ 8.04 - 7.61 (m, 2H), 7.38 - 7.09 (m, 1H), 6.63 - 6.24 (m, 1H), 5.96 - 5.44 (m, 2H), 4.62 - 4.45 (m, 1H), 2.94 - 2.42 (m, 2H), 1.68 - 1.65 (m, 3H), 1.18 - 0.60 (m, 2H).

### Synthesis and characterization of target compound 40:

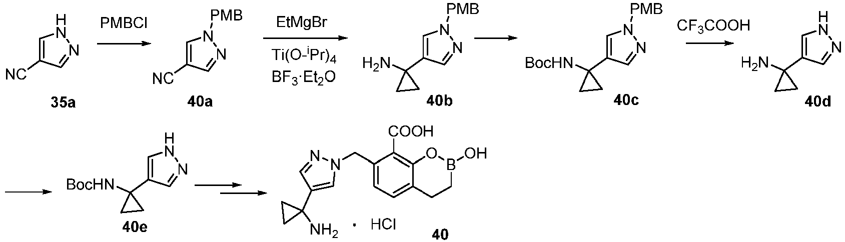

Using 4-cyanopyrazole as raw material, the intermediate 40a (2.1 g, 98% yield) was synthesized by the synthesis method similar to that of intermediate 39b. The intermediate 40a (9.8 mmol, 2.1 g) was dissolved in dry tetrahydrofuran (20 mL), and tetraisopropyl titanate (12 mmol, 7.2 mL) was added. The reaction system was cooled to -78°C, and then ethyl magnesium bromide (1.0 mmol in THF) (24.5 mmol, 24.5 mL) was added. The reaction system was stirred for 30 min, and then heated to room temperature, and continued to be stirred for 1 h, and then boron trifluoride ethyl ether (19.6 mmol, 2.5 mL) was added to react at room temperature for 16 h. After the reaction was completed as monitored by LC-MS, the reaction was quenched by adding saturated ammonium chloride, and the reaction solution was added with saturated sodium bicarbonate, extracted with dichloromethane and water, and evaporated to remove solvent to obtain the crude intermediate 40b.

40b was dissolved in dichloromethane (40 mL), and di-tert-butyl dicarbonate (10 mmol, 2.2 g) and triethylamine (15 mmol, 2.1 mL) were added, to react overnight at room temperature. After the reaction was completed as monitored by TLC, the reaction system was evaporated to remove excess solvent, and subjected to column chromatography to obtain the intermediate 40c (1.2 g).

Using intermediate 40c (3.5 mmol, 1.2 g) as raw material, the intermediate 40d (650 mg, 83% yield) was synthesized by the synthesis method similar to that of intermediate 39f. Using intermediate 40d (2.91 mmol, 650 mg) as raw material, the intermediate 40e (498 mg, 35% yield) was synthesized by the synthesis method of intermediate 40c.

Using intermediate 40e (1 mmol, 492 mg) as raw material, the compound 40 (100 mg) was synthesized by the synthesis method of target compound 1. ¹H NMR (400 MHz, CD₃OD): *δ* 7.90 - 7.72 (m, 1H), 7.72 - 7.49 (m, 1H), 7.34 - 7.14 (m, 1H), 6.63 - 6.14 (m, 1H), 5.87-5.45 (m, 2H), 2.96 - 2.48 (m, 2H), 1.35 - 1.24 (m, 2H), 1.17 (t, *J =* 7.0 Hz, 2H), 1.11 - 0.65 (m, 2H).

### Synthesis and characterization of target compound 41:

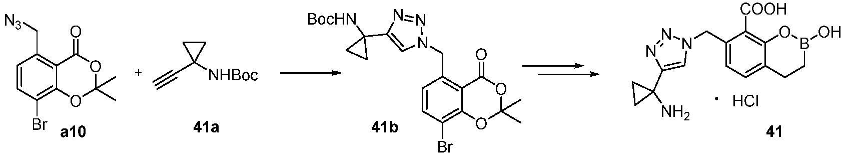

The intermediate a10 (2 mmol, 625 mg), compound 41a (2.2 mmol, 400 mg), anhydrous copper sulfate (0.2 mmol, 32 mg), and sodium ascorbate (1 mmol, 198 mg) were dissolved in tert-butanol (14 mL) and water (14 mL), to react overnight at room temperature. After the reaction was completed as monitored by TLC, the reaction system was extracted with ethyl acetate, evaporated to remove solvent and subjected to column chromatography to obtain the intermediate 41b (880 mg, 89% yield).

Using intermediate 41b (1.78 mmol, 880 mg) as raw material, the compound 41 (300 mg, 69% yield) was synthesized by the synthesis method of target compound 1. ¹H NMR (400 MHz, CD₃OD): δ 7.96 - 7.76 (m, 1H), 7.33-7.21 (m, 1H), 6.80 - 6.45 (m, 1H), 6.12 - 5.70 (m, 2H), 2.90 - 2.51 (m, 2H), 1.49 - 1.27 (m, 4H), 1.24 - 0.86 (m, 2H).

### Synthesis and characterization of target compound 42:

Using (1H-pyrazol-3-yl)methylamine as raw material, the target compound 42 (300 mg) was synthesized by the synthesis method of the target compound 1, and the yield of the five steps was 15%. ¹H NMR (400 MHz, CD₃OD): δ 7.78 - 7.58 (m, 1H), 7.30 - 7.06 (m, 1H), 6.63 - 6.15 (m, 2H), 5.84-5.43 (m, 2H), 4.12 (s, 2H), 3.08 - 2.43 (m, 2H), 1.42 - 0.60 (m, 2H).

### Synthesis and characterization of target compound 43:

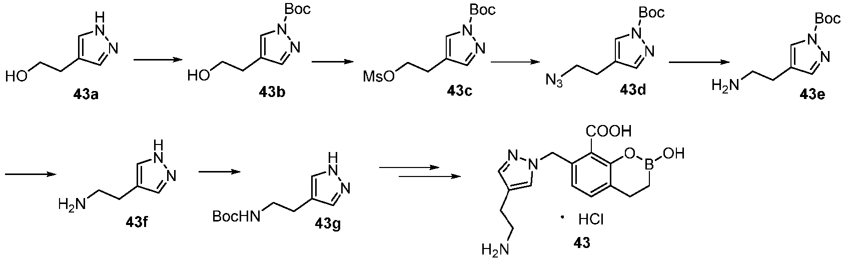

The compound 43a (10 mmol, 1.12 g) was dissolved in dichloromethane (30 mL), and di-tert-butyl dicarbonate (11 mmol, 2.4 g), DMAP (1 mmol, 122 mg), and triethylamine (15 mmol, 3 mL) were added to react overnight at room temperature. After the reaction was completed as monitored by TLC, the reaction system was evaporated to remove solvent and subjected to column chromatography to obtain intermediate 43b (1.71 g, 81%).

The intermediate 43b (8.1 mmol, 1.71 g) was dissolved in dry dichloromethane (10 mL), and triethylamine (8.1 mmol, 1.3 mL) was added. The reaction system was cooled to 0°C, and methanesulfonyl chloride (8.9 mmol, 0.9 mL) was added to react at room temperature for 2 h. After the reaction was completed as monitored by TLC, the reaction system was evaporated to remove solvent to obtain the crude intermediate 43c.

The intermediate 43c was dissolved in N,N-dimethylformamide (16 mL), and sodium azide (24 mmol, 1.56 g) was added. The mixture was heated to 70°C to react for 4-5 h. After the reaction was completed as monitored by TLC, the reaction system was cooled to room temperature, extracted with ethyl acetate and water, evaporated to remove solvent and subjected to column chromatography to obtain the intermediate 43d (1.2 g)

Using compound 43d as raw material, the compound 43 (115 mg) was synthesized by the synthesis method of the target compound 39. ¹H NMR (400 MHz, CD₃OD): δ 8.15 - 7.52 (m, 2H), 7.34 - 7.06 (m, 1H), 6.85 - 6.14 (m, 1H), 5.90 - 5.38 (m, 2H), 3.15 - 2.98 (m, 2H), 2.88 - 2.47 (m, 4H), 1.27 - 0.46 (m, 2H).

### Synthesis and characterization of target compound 44:

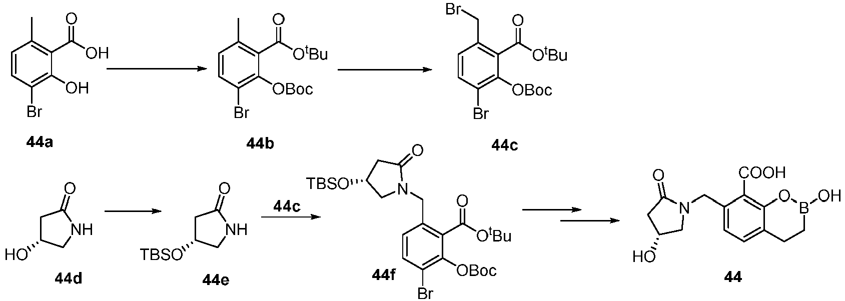

44a (42 mmol, 11 g) was dissolved in tetrahydrofuran (84 mL), and di-tert-butyl dicarbonate (168 mmol, 37 g), tert-butanol (84 mL), and DMAP (2.1 mmol, 257 mg) were added. The mixture was heated to 60°C for reaction overnight. After the reaction was completed as monitored by TLC, the reaction system was cooled to room temperature and subjected to column chromatography to obtain the intermediate 44b (7 g, 43% yield).

Using intermediate 44b (18 mmol, 7 g) as raw material, the intermediate 44c (4 g, 47% yield) was synthesized by the synthesis method of intermediate a5.

The compound 44d (20 mmol, 2 g) was dissolved in DMF (20 mL), and imidazole (40 mmol, 4.2 g), DMAP (2 mmol, 244 mg) and TBSCl (28 mmol, 4.2 g) were added, to react overnight at 40°C. After the reaction was completed as monitored by TLC, the reaction system was cooled to room temperature, extracted with ether and water, evaporated to remove solvent, triturated with n-pentane, and filtered to obtain the intermediate 44e (4 g, 93% yield).

The intermediate 44e (646 mg, 3 mmol) was dissolved in dry DMF (30 mL), and the solution was cooled to 0°C, and then sodium hydride (4.5 mmol, 108 mg) was added and stirred for 10 min, and then the intermediate 44c (4.5 mmol, 2 g) was added, to react overnight at room temperature under argon atmosphere. After the reaction was completed as monitored by TLC, the saturated ammonium chloride was added to quench the reaction, and the reaction system was extracted with ethyl acetate, evaporated to remove solvent, and subjected to column chromatography to obtain the intermediate 44f (377 mg, 21% yield).

Using intermediate 44f (0.63 mmol, 377 mg) as raw material, the compound 44 (20 mg) was synthesized by the synthesis method of the target compound 1. ¹H NMR (400 MHz, CD₃OD): δ 7.71 - 7.12 (m, 1H), 7.01-6.65(m, 1H), 4.81-4.23 (m, 2H), 3.77-3.51 (m, 1H), 3.27 - 3.07 (m, 1H), 2.95 - 2.20 (m, 4H), 1.31 - 0.49 (m, 2H).

### Synthesis and characterization of target compound 45:

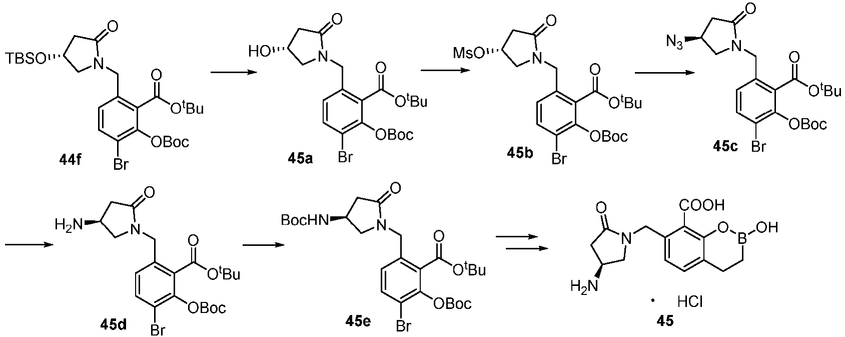

The intermediate 44f (6.66 mmol, 4 g) was dissolved in tetrahydrofuran (70 mL), and TBAF (20 mmol) was added to react at room temperature for 2 h. After the reaction was completed as monitored by TLC, the saturated ammonium chloride was added to quench the reaction, and the reaction system was extracted with ethyl acetate, and evaporated to remove solvent to obtain the intermediate 45a (1.7 g, 52% yield).

Using intermediate 45a (3.48 mmol, 1.7 g) as raw material, the intermediate 45c (610 mg) was synthesized by the synthesis method of intermediate 43d. The intermediate 45c (0.84 mmol, 610 mg) was dissolved in tetrahydrofuran (12 mL) and water (3 mL), and triphenylphosphine (1.26 mmol, 480 mg) was added, to react overnight at room temperature. After the reaction was completed as monitored by TLC, the reaction system was evaporated to remove solvent to obtain the crude intermediate 45d.

The intermediate 45d was dissolved in methanol (6 mL), and di-tert-butyl dicarbonate (0.9 mmol, 200 mg) and triethylamine (0.9 mmol, 0.3 mL) were added, to react overnight at room temperature. After the reaction was completed as monitored by TLC, the reaction system was evaporated to remove solvent, and added with DCM to redissolve the remaining residue. The resulting solution was washed with saturated ammonium chloride, evaporated to remove solvent, and subjected to column chromatography to obtain the intermediate 45e (635 mg).

Using intermediate 45e (1.08 mmol, 635 mg) as raw material, the target compound 45 (20 mg) was synthesized by the synthesis method of the target compound 1. ¹H NMR (400 MHz, CD₃OD): *δ* 7.42 - 7.07 (m, 1H), 7.07 - 6.58 (m, 1H), 5.27-5.04 (m, 1H), 4.76 - 4.26 (m, 1H), 4.15 - 3.63 (m, 2H), 3.51-3.30 (m, 1H), 3.09 - 2.38 (m, 4H), 1.41 - 0.36 (m, 2H).

### Synthesis and characterization of target compound 46:

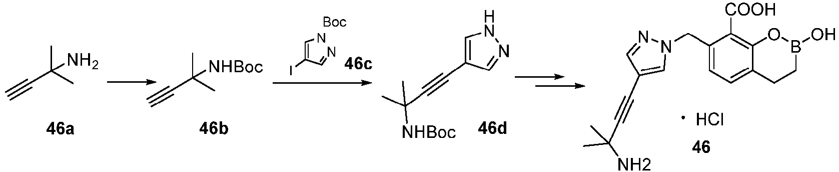

The compound 46a (10 mmol, 1.05 mL) was dissolved in tetrahydrofuran (20 mL) and water (47.5 mL), the saturated sodium bicarbonate (1.5 ml) was added, and then di-tert-butyl dicarbonate (10 mmol, 2.2 g) was added to react overnight at room temperature. After the reaction was completed as monitored by TLC, the reaction system was extracted with dichloromethane, and evaporated to remove solvent to obtain the intermediate 46b.

The intermediate 46b (5.4 mmol, 990 mg), compound 46c (2.7 mmol, 844 mg), cuprous iodide (0.11 mmol, 21 mg), and tetrakis(triphenylphosphine)palladium (0.22 mmol, 250 mg) were dissolved in DMF (6 mL), and triethylamine (4.3 mmol) was added. The mixture was heated to 100°C for 3 h of reaction under argon atmosphere. After the reaction was completed as monitored by TLC, the reaction system was cooled to room temperature, quenched by adding saturated ammonium chloride, extracted with ethyl acetate and water, evaporated to remove solvent, and subjected to column chromatography to obtain the intermediate 46d (748 mg, 55% yield).

Using intermediate 46d (1 mmol, 250 mg) as raw material, the target compound 46 (40 mg) was synthesized by the synthesis method of the target compound 1. ¹H NMR (400 MHz, CD₃OD): δ 8.46 - 7.53 (m, 2H), 7.31-7.15 (m, 1H), 6.71 - 6.05 (m, 1H), 5.97 - 5.44 (m, 2H), 2.94 - 2.45 (m, 2H), 1.42 (s, 6H), 1.11 - 0.64 (m, 2H).

### Synthesis and characterization of target compound 47:

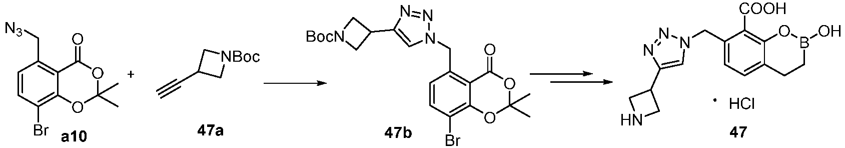

Using compound 47a (2.42 mmol, 439 mg) as raw material, the target compound 47 (100 mg) was synthesized by the synthesis method of the target compound 41. ¹H NMR (400 MHz, CD₃OD): δ 8.10 - 7.60 (m, 1H), 7.56 - 7.11 (m, 1H), 7.06 - 6.33 (m, 1H), 6.23 - 5.35 (m, 2H), 4.52 - 4.14 (m, 5H), 2.95 - 2.53 (m, 1H),1.99-1.76(m, 1H), 1.47 - 0.65 (m, 2H).

### Synthesis and characterization of target compound 48:

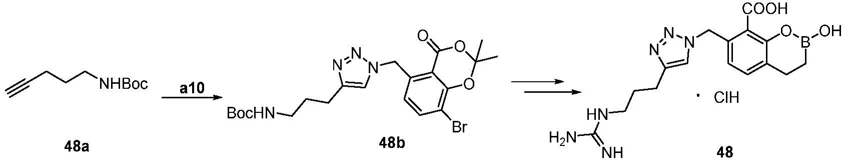

Using N-BOC-4-pentyne-1-amine (2.2 mmol, 420 mg) as raw material, the intermediate 48b (706 mg, 72% yield) was synthesized by the synthesis method of the intermediate 41b. Using intermediate 48b (1.43 mmol, 706 mg) as raw material, the compound 48 (60 mg) was synthesized by the synthesis method of target compound 37. ¹H NMR (400 MHz, CD₃OD): *δ* 8.06 - 7.67 (m, 1H), 7.43 - 7.07 (m, 1H), 6.87 - 6.41 (m, 1H), 6.10 - 5.58 (m, 2H), 3.18-3.09 (m, 2H), 2.83 - 2.46 (m, 4H), 2.06 - 1.71 (m, 2H), 1.22 - 0.56 (m, 2H).

### Synthesis and characterization of target compound 49:

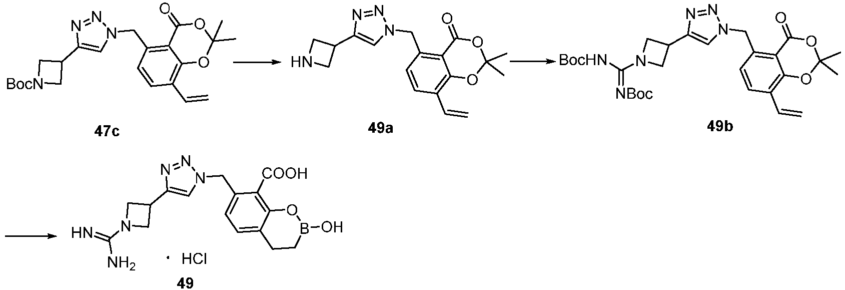

Using intermediate 47c (1.77 mmol, 781 mg) as raw material, the intermediate 49a was synthesized by the synthesis method of the intermediate 33c. Using intermediate 49a as raw material, the intermediate 49b was synthesized by the synthesis method of intermediate 37b. Using intermediate 49b (0.5 mmol, 289 mg) as raw material, the target compound 49 (30 mg) was synthesized by the synthesis method of target compound 1. ¹H NMR (400 MHz, CD₃OD): δ 7.98 - 7.69 (m, 1H), 7.36 - 7.10 (m, 1H), 6.77 - 6.43 (m, 1H), 6.13-5.67 (m, 2H), 4.50 (t, *J* = 8.2 Hz, 2H), 4.32 - 3.98 (m, 3H), 2.94 - 2.52 (m, 2H), 1.12 - 0.60 (m, 2H).

### Synthesis and characterization of target compound 50:

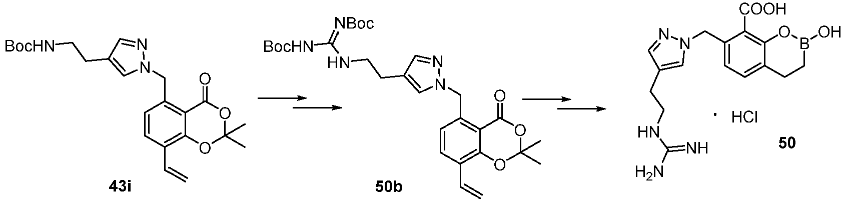

Using compound 43i (0.47 mmol, 203 mg) as raw material, the target compound 50 (50 mg) was synthesized by the synthesis method of target compound 48. ¹H NMR (400 MHz, CD₃OD): δ 8.01 - 7.54 (m, 1H), 7.40 - 7.08 (m, 1H), 6.70 - 6.11 (m, 1H), 5.90 - 5.41 (m, 2H), 3.36 - 3.23 (m, 2H), 2.85 - 2.45 (m, 2H), 1.16 - 0.57 (m, 2H).

### Synthesis and characterization of target compound 51:

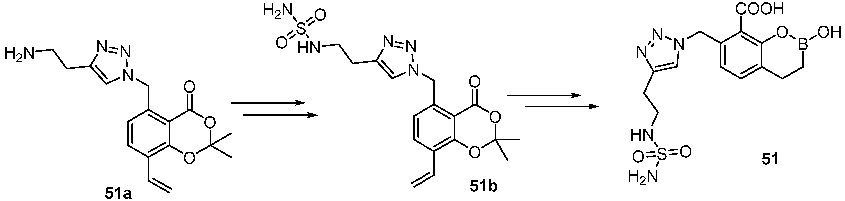

Using intermediate 51a as raw material, the target compound 51 was synthesized by the synthesis method of target compound 36. ESI-MS: [M+H⁺]:396.1.

### Synthesis and characterization of target compound 52:

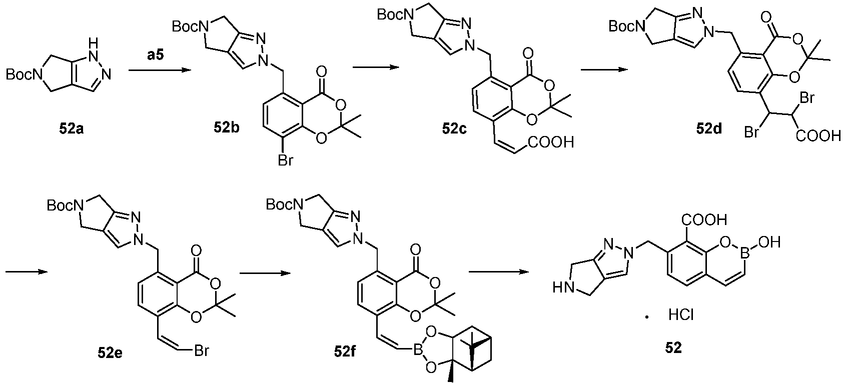

The compound 52a (5.7 mmol, 1.2 g) and intermediate a5 (5.7 mmol, 2 g) was dissolved in dry DMF (17 mL), and cesium carbonate (6.84 mmol, 2.23 g) was added, to react overnight at room temperature under argon atmosphere. After the reaction was completed as monitored by TLC, the reaction system was extracted with ethyl acetate and water, washed with saturated sodium chloride, dried over anhydrous sodium sulfate, evaporated to remove excess solvent and subjected to column chromatography to obtain the intermediate 52b (1.03 g, 48% yield).

The intermediate 52b (1.03 g, 2.72 mmol) was dissolved in dry DMF (14 mL), and acrylic acid (4.1 mmol, 0.29 ml), palladium acetate (0.14 mmol, 31 mg), P(o-toly)₃ (0.28 mmol, 83 mg), and triethylamine (8.1 mmol, 1.1 mL) were added sequentially. The mixture was heated to 100°C for 14 h of reaction under argon atmosphere. After the reaction was completed as monitored by TLC, the reaction system was cooled to room temperature, extracted with ethyl acetate and water, evaporated to remove solvent, and triturated with n-pentane to obtain the intermediate 52c (772 mg, 60% yield).

The intermediate 52c (1.64 mmol, 772 mg) was dissolved in chloroform (18 mL). The resulting solution was cooled to 0°C, and bromine (2 mmol, 110 µL) was slowly added dropwise, to continue to react at 0°C for 2-3 h. After the reaction was completed as monitored by TLC, the reaction system was evaporated to remove excess solvent to obtain the crude intermediate 52d.

The intermediate 52d was dissolved in DMF (6 mL). The resulting solution was cooled to 0°C, and triethylamine (1.12 mL) was added for reaction overnight at room temperature. After the reaction was completed as monitored by TLC, the reaction system was extracted with ethyl acetate and water , evaporated to remove solvent, and subjected to column chromatography to obtain the intermediate 52e (676 mg).

The intermediate 52e (1.34 mmol, 676 mg) was dissolved in 1,4-dioxane (7 mL), and bis[(+)-pinanediolato]diboron (2 mmol, 720 mg), Pd(dppf)Cl₂ (0.13 mmol, 98 mg), and potassium acetate (5.4 mmol, 526 mg) were sequentially added. The mixture was heated to 60°C under argon atmosphere for 2 h of reaction. After the reaction was completed as monitored by TLC, the reaction system was cooled to room temperature, extracted with ethyl acetate and water, evaporated to remove excess solvent, and subjected to column chromatography to obtain the intermediate 52f (300 mg, 37%). Using intermediate 52f (0.5 mmol, 300 mg) as raw material, the target compound 52 was synthesized by the synthesis method of the compound 1. ESI-MS:[M+H⁺]:312.1.

### Synthesis and characterization of target compounds 53 and 54:

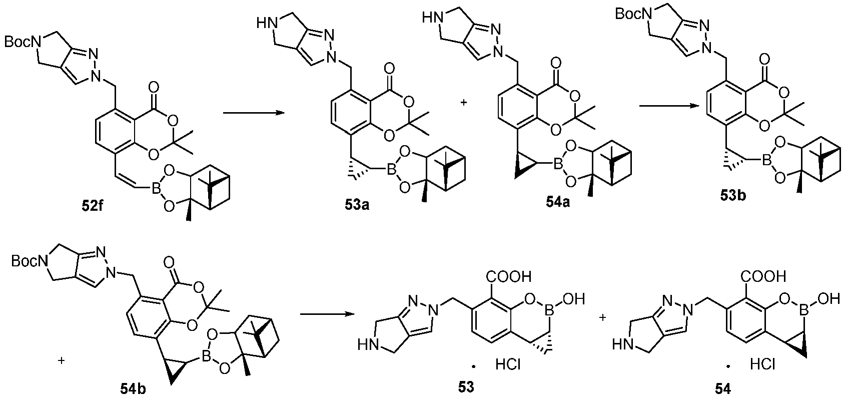

Diethyl zinc (4.4 mmol, 4.45 mL) was dissolved in dry DCM (3.5 mL). The resulting solution was cooled to -78°C, then diiodomethane (6.6 mmol, 0.59 ml) was added, the mixture was stirred for 30 min, and then a solution of 52f (334 mg, 0.55 mmol) in DCM (2.5 mL) was added. The resulting mixture was slowly heated to room temperature to continue to react for 24 h. After the reaction was completed as monitored by TLC, the reaction system was evaporated to remove solvent to obtain the crude intermediates 53a and 54a.

The intermediates 53a and 54a were dissolved in DCM (3 mL), and triethylamine (0.3 mL) and di-tert-butyl dicarbonate (138 mg) were added to react at room temperature for 1 h. After the reaction was completed as monitored by TLC, the reaction system was evaporated to remove excess solvent, and subjected to column chromatography to obtain the intermediates 53b (142 mg) and 54d (65 mg).

The target compound 53 was synthesized (30 mg) from the intermediate 53b (0.23 mmol, 142 mg) by the synthesis method of the target compound 1. ¹H NMR (400 MHz, CD₃OD): δ 7.74 - 7.38 (m, 1H), 6.99 - 6.41 (m, 1H), 6.41 - 6.16 (m, 1H), 6.07 - 5.36 (m, 1H), 4.49-4.29 (m, 4H), 2.23 - 1.54 (m, 2H), 1.15 - 0.76 (m, 1H), 0.75 - 0.47 (m, 1H).

The target compound 54 (10 mg) was synthesized from the intermediate 54b (0.1 mmol, 65 mg) by the synthesis method of the target compound 1. ESI-MS: [M+H⁺]:326.1.

### Synthesis and characterization of target compound 55:

The target compound 55 was synthesized according to the synthesis method of target compound 1, except that 2*H*-1,2,3-triazole was replaced by 7*H*-pyrazolopyrimidine, and total yield of the four steps was 9%. ¹H NMR (400 MHz, Methanol-d₄) δ 9.02 (d, *J* = 11.0 Hz, 1H), 8.81 (d, *J* = 25.7 Hz, 1H), 7.54 (d, *J =* 29.2 Hz, 1H), 7.11 (d, *J =* 23.0 Hz, 1H), 6.76 (d, 11.0 Hz , 1H), 6.34 - 5.80 (m, 3H), 2.86 - 2.42 (m, 2H), 2.11 (d, *J =* 65.1 Hz, 1H), 1.72 (m, 1H). ESI-MS: m/z calcd for C₁₆H₁₄BN₃O₄ [M+H]⁺ 324.1, found 324.1.

### Synthesis and characterization of target compound 56:

Using intermediate 52b as raw material, the compound 56 (30 mg) was synthesized by the synthesis method of the target compound 1. ¹H NMR ¹H NMR (400 MHz, D₂O) δ 7.48 (d, *J =* 15.0 Hz, 1H), 7.15 (d, *J=* 7.3 Hz, 1H), 6.66 (d, *J* = 7.9 Hz, 1H), 5.45 (s, 2H), 4.42 (d, *J=* 15.8 Hz, 4H), 2.73 (t, *J* = 8.0 Hz, 2H), 1.06 (t, *J* = 7.8 Hz, 2H). ESI-MS: m/z calcd for C₁₅H₁₇BN₃O₄ [M+H]⁺ 314.1, found 314.1.

### Synthesis and characterization of target compound 57:

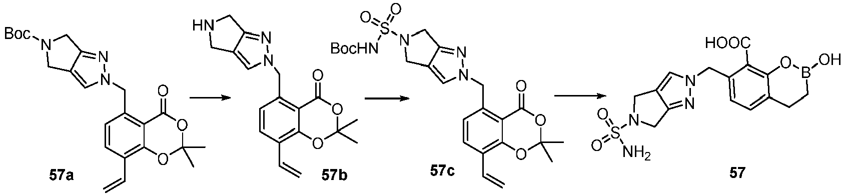

Using compound 57a as raw material, the compound 57 (40 mg) was synthesized by the synthesis method of the target compound 36. ¹H NMR¹H NMR (400 MHz, MeOD) δ 7.44 (s, 1H), 7.34 - 7.15 (m, 2H), 5.61 (d, *J =* 66.5 Hz, 3H), 4.40 (s, 4H), 2.75 (t, *J =* 8.0 Hz, 2H), 1.09 (t, *J =* 7.8 Hz, 2H). ESI-MS: m/z calcd for C₁₅H₁₈BN₄O₆S [M+H]⁺ 393.1, found 393.1.

### Synthesis and characterization of target compound 58:

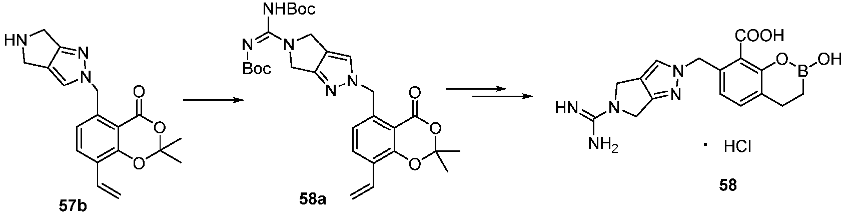

Using intermediate 57b as raw material, the compound 58 (78 mg) was synthesized by the synthesis method of the target compound 37. ¹H NMR (400 MHz, MeOD) δ 7.61 - 7.48 (m, 1H), 7.17 (m, 1H), 6.31 (m, 1H), 5.87 - 5.49 (m, 2H), 4.66 - 4.28 (m, 4H), 2.91 - 2.52 (m, 2H), 1.12 - 0.66 (m, 2H). ESI-MS: m/z calcd for C₁₆H₁₉BN₅O₄ [M+H]⁺ 356.1, found 356.1.

### Synthesis and characterization of target compound 59:

Using tert-butyl (4-ethynylphenyl)carbamate as raw material, the compound 59 was synthesized by the synthesis method of the target compound 4. ¹H NMR (400 MHz, MeOD) δ 8.33 - 8.16 (m, 1H), 8.04 - 7.88 (m, 2H), 7.44 (q, *J =* 8.6 Hz, 2H), 7.24 (dd, *J =* 27.3, 7.7 Hz, 1H), 6.81 - 6.43 (m, 1H), 6.17 - 5.77 (m, 2H), 2.87 - 2.53 (m, 2H), 1.12 - 0.68 (m, 2H). ESI-MS: m/z calcd for C₁₈H₁₈BN₄O₄ [M+H]⁺ 365.1, found 365.2.

### Synthesis and characterization of target compound 60:

Using tert-butyl (4-ethynylpyridyl)carbamate as raw material, the compound 60 was synthesized by the synthesis method of the target compound 4. ¹H NMR (400 MHz, MeOD) δ 8.45 - 8.00 (m, 3H), 7.43 - 6.93 (m, 2H), 6.84 - 6.52 (m, 1H), 6.15 - 5.75 (m, 2H), 2.90 - 2.52 (m, 2H), 1.23 - 0.72 (m, 2H). ESI-MS: m/z calcd for C₁₇H₁₇BN₅O₄ [M+H]⁺ 366.1, found 366.1.

### Synthesis and characterization of target compound 61:

Using tert-butyl (5-ethynyl-2-pyrimidinyl)carbamate as raw material, the compound 61 was synthesized by the synthesis method of the target compound 4. ¹H NMR (400 MHz, MeOD) δ 9.06 - 8.89 (m, 2H), 8.36 - 8.11 (m, 1H), 7.36 - 7.14 (m, 1H), 6.80 - 6.54 (m, 1H), 6.15 - 5.75 (m, 2H), 2.91 - 2.54 (m, 2H), 1.21 - 0.89 (m, 2H). ESI-MS: m/z calcd for C₁₆H₁₆BN₆O₄ [M+H]⁺ 367.1, found 367.1.

### Synthesis and characterization of target compound 62:

Using 4-ethynylbenzenesulfonamide as raw material, the compound 62 was synthesized by the synthesis method of the target compound 4. ¹H NMR (400 MHz, MeOD) δ 8.42 - 8.28 (m, 1H), 8.06 - 7.20 (m, 5H), 6.77 - 6.48 (m, 1H), 6.14 - 5.78 (m, 2H), 2.88 - 2.58 (m, 2H), 1.37 - 1.04 (m, 2H). ESI-MS: m/z calcd for C₁₈H₁₈BN₄O₆ [M+H]⁺ 429.1, found 429.1.

### Synthesis and characterization of target compound 63:

Using tert-butyl (4-ethynylbenzyl)carbamate as raw material, the compound 63 was synthesized by the synthesis method of the target compound 4. ¹H NMR (400 MHz, MeOD) δ 8.47 - 8.29 (m, 1H), 7.87 (d, *J* = 8.4 Hz, 2H), 7.53 (dd, *J* = 8.5, 2.3 Hz, 2H), 7.31 - 7.20 (m, 1H), 6.84 - 6.51 (m, 1H), 6.13 - 5.78 (m, 2H), 4.14 (s, 2H), 2.89 - 2.57 (m, 2H), 1.10 - 0.70 (m, 2H). ESI-MS: m/z calcd for C₁₈H₁₈BN₄O₆ [M+H]⁺ 379.1, found 379.2.

### Synthesis and characterization of target compound 64:

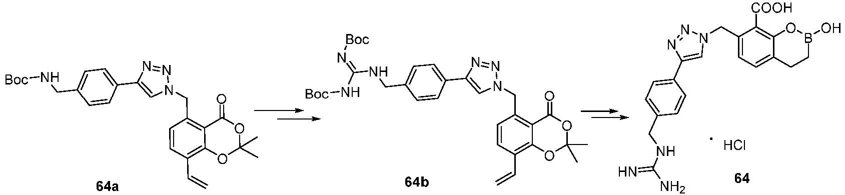

Using compound 64a as raw material, the compound 64 was synthesized by the synthesis method of the target compound 37. ¹H NMR (400 MHz, MeOD) δ 8.51 - 8.38 (m, 1H), 7.83 (m, 2H), 7.44 (m, 2H), 7.35 - 7.25 (m, 1H), 6.71 (m, 1H), 6.16 - 5.83 (m, 2H), 4.46 (s, 2H), 2.89 - 2.59 (m, 2H), 1.12 - 0.70 (m, 2H).

### Synthesis and characterization of target compound 65:

Using tert-butyl (4-ethynylphenylethyl)carbamate as raw material, the compound 65 was synthesized by the synthesis method of the target compound 4. ¹H NMR (400 MHz, MeOD) δ 8.53 - 8.35 (m, 1H), 7.84 - 7.74 (m, 2H), 7.46 - 7.25 (m, 3H), 6.73 (dd, *J =* 66.6, 7.6 Hz, 1H), 6.16 - 5.82 (m, 2H), 3.20 (t, *J =* 7.8 Hz, 2H), 3.08 - 2.95 (t, *J =* 7.8 Hz, 2H), 2.91 - 2.60 (m, 2H), 1.14 - 0.72 (m, 2H).

### Synthesis and characterization of target compound 66:

The compound 66 was synthesized by the synthesis method of the target compound 56. ¹H NMR (400 MHz, MeOD) δ 7.51 - 7.21 (m, 2H), 6.82 - 6.48 (m, 1H), 5.84 - 5.39 (m, 2H), 4.37 (m,, 2H), 4.12 (d, 2H), 2.89 - 2.58 (m, 2H), 1.16 - 0.66 (m, 2H). ESI-MS: m/z calcd for C₁₅H₁₇BN₃O₄ [M+H]⁺ 314.1, found 314.1.

### Synthesis and characterization of target compound 67:

The compound 67 was synthesized by the synthesis method of the target compou nd 58. ¹H NMR (400 MHz, MeOD) δ 7.52 - 7.18 (m, 2H), 6.69 - 6.24 (m, 1H), 5.88 - 5.42 (m, 2H), 4.63 - 4.31 (m, 4H), 2.88 - 2.57 (m, 2H), 1.12 - 0.67 (m, 2H).

### Synthesis and characterization of target compound 68:

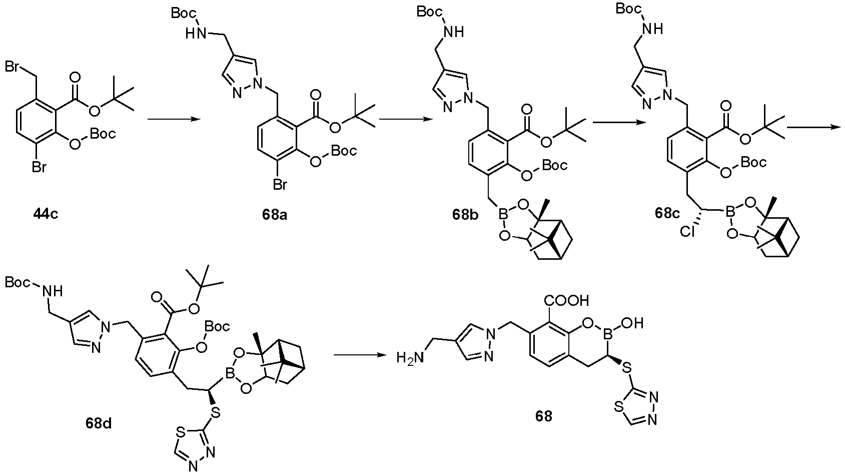

K₂CO₃ (2.25 mmol, 311 mg), 4-(Boc-aminomethyl)pyrazole (2.25 mmol, 443 mg), KI (0.02 mmol, 5 mg), and 44c (1.5 mmol, 700 mg) were dissolved in a DMF solution (10 mL) under an atmosphere of N₂, and stirred at 80°C overnight. After the reaction was completed as monitored by TLC, the reaction mixture was poured into water, and extracted with ethyl acetate (20 mL×3). The organic phases were combined, washed with saturated aqueous NaCl, dried over anhydrous Na₂SO₄, concentrated and purified by silica gel column chromatography to obtain the compound 68a (670 mg, 77% yield).

DIBAL-H (0.33 mmol, 220 µL) was added dropwise to a solution of zinc powder (6.6 mmol, 429 mg) and boron ester compounds (0.3 mmol, 75 mg) in anhydrous tetrahydrofuran (5 mL) at room temperature, and the mixture was stirred for 5 min, and the solution of boron ester compounds (7.2 mmol, 720 mg) in anhydrous tetrahydrofuran (10 mL) was added dropwise. The reaction mixture was heated to 50°C and stirred for 1.5 h. The supernatant was mixed with a solution of compound 68a (1.1 mmol, 620 mg) and Pd(t-Bu₃P)₂ (0.03 mmol, 14 mg) in THF (8 mL) and reacted under N₂ for 1 h. The reaction solution was concentrated and then purified directly by silica gel chromatography to obtain the compound 68b (627 mg, 82% yield). ESI-MS: m/z calcd for C₃₇H₅₅BN₃O₉ [M+H]⁺696.4, found 696.4.

A solution of DCM (2 mmol, 170 mg) in THF (4 mL) was cooled to -100°C under argon atmosphere, and n-BuLi (1.3 mmol, 512 µL) was slowly added dropwise. The mixture was stirred at this temperature for 30 min, and then a solution of compound 68b (0.8 mmol, 550 mg) in THF (3 mL) was added dropwise to this mixture. The mixture was slowly heated to room temperature and stirred overnight and evaporated to dryness using a rotary evaporator to obtain the compound 68c. ESI-MS: m/z calcd for C₃₈H₅₆BClN₃O₉ [M+H]⁺744.4, found 744.4.

To a solution of compound 68c (0.26 mmol, 190 mg) and 2-mercapto-1,3,4-thiadiazole (0.31 mmol, 36 mg) in DCM (2 mL), triethylamine (0.52 mmol, 52 mg) was added at room temperature. After stirring for 2 h, the solution was diluted with DCM, washed with dilute hydrochloric acid and water and concentrated to obtain the compound 68d (50 mg, 23% yield).

The compound 68d (0.06 mmol, 50 mg) was dissolved in 3 mL of 1,4-dioxane solution, and 6N HCl (3 mL) was added. The resulting solution was heated to 100°C and refluxed for 2 h. After the reaction was completed as monitored by TLC, the reaction system was concentrated under reduced pressure in vacuum, and recrystallized with acetonitrile-methanol to obtain the target compound 68 (20 mg, 79% yield). ¹H NMR (400 MHz, MeOD) δ 9.25 (m, *J =* 3.7 Hz, 1H), 7.97 - 7.57 (m, 2H), 7.49 - 7.10 (m, 1H), 5.92 - 5.72 (m, 1H), 5.60 - 5.34 (m, 1H), 4.14 - 3.97 (m, 2H), 3.81 - 3.58 (m, 1H), 3.25 - 3.00 (m, 1H), 2.99 - 2.71 (m, 1H). ESI-MS: m/z calcd for C₁₆H₁₇BN₅O₄S₂ [M+H]⁺418.1, found 418.1.

### Synthesis and characterization of target compound 69:

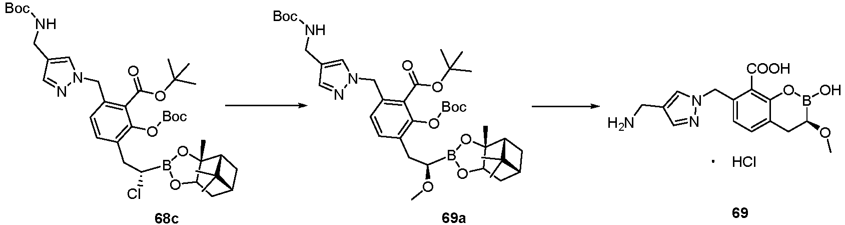

To a solution of compound 68c (0.27 mmol, 200 mg) in DMF (5 mL), MeOH (0.41 mmol, 13 mg) was added, and then K₂CO₃ (1.1 mmol, 149 mg) was added. The resulting mixture was stirred at 50°C for 1 h, then diluted with ethyl acetate and washed with saturated NH₄Cl and water. The organic layer was concentrated and dried and then purified by column chromatography to obtain pure compound 69a (70 mg, 79% yield). ESI-MS: m/z calcd for C₃₉H₅₉BN₃O₁₀ [M+H]⁺740.4, found 740.4.

The compound 69a (0.09 mmol , 70 mg) was dissolved in 3 mL of 1,4-dioxane solution, and 6N HCl (3 ml) was added. The resulting solution was heated to 100°C and refluxed for 2 h. After the reaction was completed as monitored by TLC, the reaction system was concentrated under reduced pressure in vacuum, and recrystallized with acetonitrile-methanol to obtain the target compound 69 (35 mg, 79% yield). ¹H NMR (400 MHz, MeOD) δ 7.83 - 7.56 (m, 2H), 7.32 - 7.10 (m, 1H), 6.56 - 6.25 (m, 1H), 5.81 - 5.41 (m, 2H), 4.05 (d, *J =* 17.5 Hz, 2H), 3.60 - 3.30 (m, 3H), 3.26 - 2.84 (m, 3H). ESI-MS: m/z calcd for C₁₅H₁₉BN₃O₅ [M+H]⁺332.1, found 332.2.

### Synthesis and characterization of target compound 70:

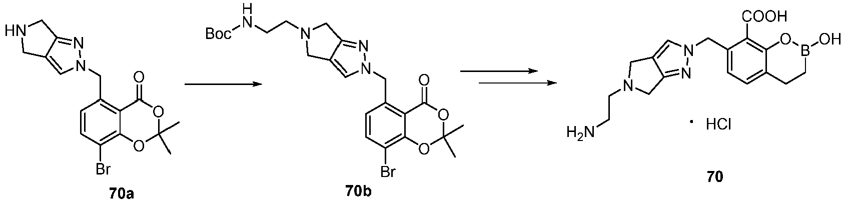

The compound 70a (1.73 mmol, 850 mg), N-Boc-bromoethylamine (426 mg, 1.9 mmol), and triethylamine (3.46 mmol, 350 mg) were dissolved in DMF (10 mL) at room temperature to react overnight at 50°C. After the reaction was completed as monitored by TLC, the reaction system was diluted with water and extracted with ethyl acetate. The organic layers were combined, washed with saturated NaCl, dried over anhydrous Na₂SO₄, concentrated under reduced pressure and purified by silica gel column chromatography to obtain the compound 70b (696 mg, 77% yield). Using compound 70b as raw material, the compound 70 (120 mg) was synthesized by the synthesis method of target compound 1. ¹H NMR (400 MHz, MeOD) δ 7.58 - 7.44 (m, 1H), 7.26 - 7.12 (m, 1H), 6.47 (m, 1H), 5.70 - 5.47 (m, 1H), 4.64 (s, 4H), 3.83 (m, *J =* 14.4, 6.8 Hz, 2H), 3.48 (m, 2H), 2.87 - 2.52 (m, 2H), 1.11 - 0.58 (m, 2H).

### Synthesis and characterization of target compound 71:

Using 3-cyanopyrrole raw material, the compound 71 was synthesized by the synthesis method of target compound 1, and total yield of the four steps was 12%. ¹H NMR (400 MHz, Methanol-*d*₄) δ 7.37 (m , 1H), 7.24 - 7.19 (m, 1H), 6.76 (m, 1H), 6.63 - 6.53 (m, 1H), 6.44 - 6.37 (m, 1H), 5.30 (s, 2H), 2.73 (t, *J =* 8.0 Hz, 2H), 1.08 (t, *J =* 7.9 Hz, 2H).

### Synthesis and characterization of target compound 72:

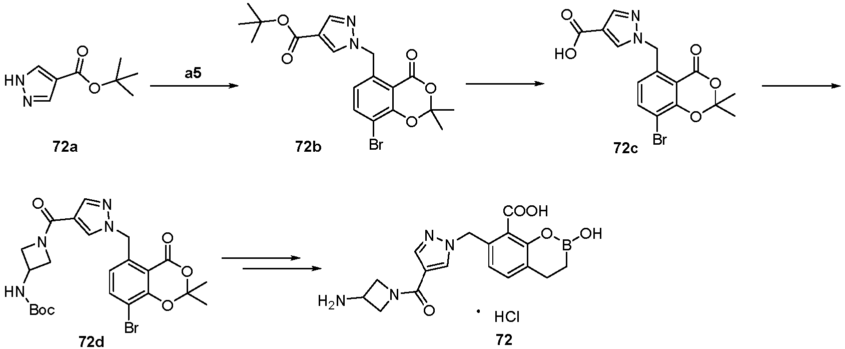

At room temperature, the compound 72a (2.86 mmol), a5 (2.86 mmol, 1.0 g), K₂CO₃ (593 mg, 4.29 mmol), and KI (0.03 mmol, 5 mg) were added to a two-necked flask, and DMF was added under the atmosphere of N₂, to react at 80°C overnight. After the reaction was completed as monitored by TLC, the reaction system was diluted with water and extracted with ethyl acetate. The organic layers were combined, washed with saturated NaCl, dried over anhydrous Na₂SO₄, concentrated under reduced pressure and purified by silica gel column chromatography to obtain the compound 72b (807 mg, 62% yield).

TFA (2.3 mL) was added dropwise to a solution of compound 72b (1.85 mmol, 807 mg) in DCM at room temperature, the mixture was stirred at room temperature for 3 h. After the reaction was completed as monitored by TLC, the reaction system was concentrated under reduced pressure, diluted with water and extracted with ethyl acetate. The organic layers were combined, washed with saturated NaCl, dried over anhydrous Na₂SO₄ and concentrated under reduced pressure to obtain the compound 72c (662 mg, 95%).

At room temperature, the compound 72c (1.75 mmol, 662 mg), and HBTU (2.1 mmol, 796 mg) was dissolved in DMF (7 mL), and EtN(Pr-i)₂ (5.25 mmol, 679 mg) was added and stirred at room temperature for 15 min, and 3-N-tert-butyloxycarbonylamino-cyclobutyl amine (2.1 mmol, 362 mg) was added to react at room temperature for 3 h. After the reaction was completed as monitored by TLC, the reaction system was diluted with water and extracted with ethyl acetate. The organic layers were combined, washed with saturated NaCl, dried over anhydrous Na₂SO₄, concentrated under reduced pressure and purified by silica gel column chromatography to obtain the compound 72d (639 mg, 69% yield).

Using compound 72d (1.20 mmol, 639 mg) as raw material, the compound 72 was synthesized by the synthesis method of target compound 1. ¹H NMR (400 MHz, Methanol-d4) δ 8.13 - 7.93 (m, 1H), 7.91 - 7.76 (m, 1H), 7.15 (m, J1H), 6.57 - 6.12 (m, 1H), 5.80 - 5.36 (m, 2H), 4.33 - 4.22 (m, 2H), 3.98 - 3.50 (m, 3H), 2.74 (m, 2H), 0.96 (m, 2H).

### Synthesis and characterization of target compound 73:

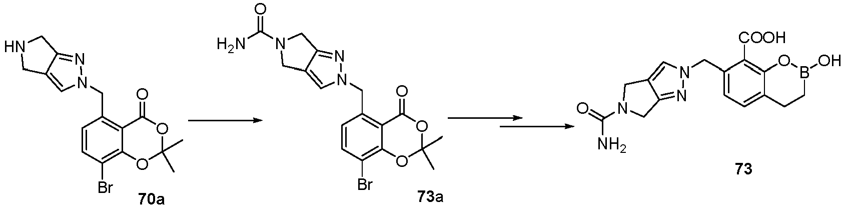

The compound 70a (2.7 mmol, 1000 mg), KOCN (8.1 mmol, 656 mg), and AcOH (5.4 mmol, 310 mg) were dissolved in dichloromethane (20 mL) and stirred at room temperature overnight. The reaction system was evaporated to dryness using a rotary evaporator and purified through column to obtain the target compound 73a (1.1 g, 96% yield).

Using compound 73a as raw material, the compound 73 was synthesized by the synthesis method of target compound 1. ¹H NMR (400 MHz, MeOD) δ 7.59 - 7.44 (m, 1H), 7.26 - 7.11 (m, 1H), 6.49 - 6.15 (m, 1H), 5.82 - 5.45 (m, 2H), 4.52 (m, 4H), 2.64 (m, 2H), 1.09 (t, 2H). ESI-MS: m/z calcd for C₁₆H₁₈BN₄O₅ [M+H]⁺ 357.1, found 357.1.

### Synthesis and characterization of target compound 74:

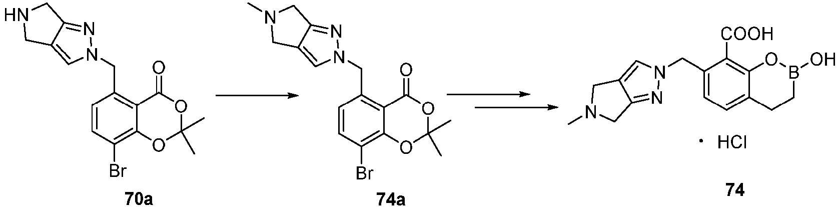

The compound 70a (3 mmol, 1100 mg), aqueous formaldehyde solution (21 mmol, 1.7 g), and AcOH (30 mmol, 1.8 ml) were dissolved in acetonitrile (20 mL) and stirred at room temperature for 30 min before NaBH₃CN (6 mmol, 377 mg) was added. The reaction system was evaporated to dryness using a rotary evaporator and purified through column to obtain the target compound 74a (490 mg, 42% yield). Using compound 73a as raw material, the compound 74 was synthesized by the synthesis method of target compound 1. ESI-MS: m/z calcd for C₁₆H₁₉BN₃O₄ [M+H]⁺ 328.1, found 328.1.

### Synthesis and characterization of target compound 75:

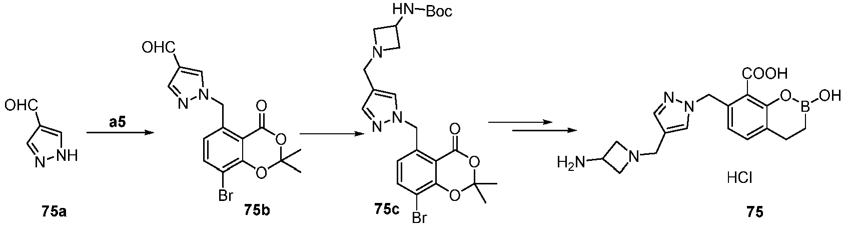

K₂CO₃ (4.32 mmol, 597 mg), aldehyde pyrazole (75a, 2.88 mmol, 276 mg), and a5 (2.88 mmol, 1 g) were dissolved in DMF (10 mL) solution at 0°C and under nitrogen atmosphere, and stirred at room temperature overnight. After the reaction was completed as monitored by TLC, the reaction mixture was poured into water, and extracted with ethyl acetate (20 mL×3). The organic phases were combined, washed with saturated aqueous NaCl, dried over anhydrous Na₂SO₄, concentrated and purified by silica gel column chromatography to obtain 75b as a white solid (903 mg, 86% yield).

The compound 75b (4.32 mmol, 597 mg), tert-butyl azetidine-3-carbamate (4.32 mmol, 597 mg), and 9 drops of acetic acid were dissolved in 24 mL of dichloromethane and refluxed for 1 h. NaBH₃CN (4.32 mmol, 597 mg), and 9 mL of ethanol were added, and the mixture was stirred at room temperature for 17 h. The reaction system was evaporated to dryness using a rotary evaporator and purified through column to obtain the compound 75c (800 mg, 70% yield). Using compound 75c as raw material, the compound 75 was synthesized by the synthesis method of target compound 1. ¹H NMR (400 MHz, MeOD) δ 7.86 (m, 1H), 7.77 - 7.67 (m, 1H), 7.30 - 7.19 (m, 1H), 6.83 (m, 1H), 6.29 - 6.18 (m, 1H), 5.84 - 5.28 (m, 3H), 4.47 - 3.82 (m, 6H), 3.55 - 3.27 (m, 2H). ESI-MS: m/z calcd for calcd for C₁₇H₂₂BN₄O₄ [M+H]⁺ 357.2, found 357.1.

The beneficial effects of the present disclosure are demonstrated below by specific experimental examples.

### Experimental Example 1 Inhibitory activity of compounds of the present disclosure on MBL and SBL

### 1. Experimental method

The principle of the activity assay is that MBL and SBL catalyze the reaction of fluorescent substrate to produce fluorescent groups. The activity of the enzymes can be reflected by testing the intensity of fluorescence. The activity assay was carried out in a black 96-well microplate. The total reaction system was 60 µL. The specific operation steps are described below.
(1) The solid compound to be tested was prepared into a 100 mM stock solution with DMSO. The stock solution was diluted with MBL activity assay buffer (20 mM Tris-HCl pH 7.5, 200 mM NaCl, 0.01% Triton X-100) or SBL activity assay buffer (50 mM Phosphate, pH 7.0 or 50 mM HEPES, pH 7.2, 200 mM NaCl, 1 µg/ml BSA) to a working solution (3.6 mM or 600 µM). The working solution was three-fold diluted using assay buffer to obtain 10 working solutions of varying concentrations.
(2) MBLs (including NDM-1, NDM-5, IMP-1, IMP-4, VIM-1 and VIM-2) and SBLs (KPC-2, TEM-1, SHV-12, CTX-M-14, AmpC, and OXA-48) to be tested, were prepared into enzyme solutions with appropriate concentrations using activity assay buffer.
(3) The fluorescent substrate FC-5 was prepared to a 30 µM substrate solution with assay buffer for later use.
(4) To a 96-well microplate, 10 µL of working solution of compound obtained in step (1), 30 µL of assay buffer, and 10 µL of enzyme solution were added to each well sequentially, and incubated for 10 minutes at room temperature.
(5) After the incubation was completed, 10 µL of fluorescent substrate solution was added to each well in a 96-well microplate, and the change in fluorescence value within 6 min was continuously measured using a microplate reader, wherein the excitation wavelength (λₑₓ) for fluorescence detection was 380 nm and the emission wavelength (λₑₘ) was 460 nm.
(6) Reaction wells without compounds were set as a control for each experiment, and all assays contained three sets of parallel experiments.

Based on the change in fluorescence intensity measured by the microplate reader, the residual activity of the enzyme in each well was calculated by the following calculation formula: residual activity (%)=(AF1)/(AF2)×100, where ΔF1 indicates the change in fluorescence value in the wells containing the tested compounds over a certain time period, and ΔF2 indicates the change in fluorescence value in the control wells without the compounds over the same time period. The processed data were fitted with Graphpad Prism 5 software to calculate the half inhibitory activity of the enzyme (i.e., IC₅₀ value).

### 2. Experimental results

The results of the inhibitory activity of the compounds of the present disclosure on MBL and SBL are shown in Tables 1 and 2.

**Table 1 Inhibitory activity of compounds of the present disclosure on MBL ^{α}**

| Number of compound | NDM-1 | NDM-5 | IMP-1 | IMP-4 | VIM-1 | VIM-2 |
|---|---|---|---|---|---|---|
| **1** | ++++ | ++++ | +++ | +++ | +++ | ++++ |
| **2** | ++++ | ++++ | +++ | +++ | +++ | ++++ |
| **3** | ++++ | ++++ | +++ | +++ | +++ | ++++ |
| **4** | ++++ | ++++ | ++++ | +++ | +++ | +++ |
| **5** | +++ | ++++ | +++ | +++ | +++ | ++++ |
| **6** | +++ | ++++ | +++ | +++ | +++ | ++++ |
| **7** | +++ | +++ | +++ | +++ | +++ | +++ |
| **8** | +++ | ++++ | +++ | +++ | +++ | ++++ |
| **9** | +++ | ++++ | +++ | +++ | +++ | ++++ |
| **10** | ++++ | ++++ | +++ | +++ | +++ | ++++ |
| **11** | ++++ | ++++ | +++ | +++ | +++ | ++++ |
| **12** | ++++ | ++++ | +++ | +++ | +++ | ++++ |
| **13** | ++++ | ++++ | +++ | +++ | +++ | ++++ |
| **14** | ++++ | ++++ | +++ | +++ | +++ | ++++ |
| **15** | ++++ | ++++ | +++ | +++ | +++ | ++++ |
| **16** | ++++ | ++++ | +++ | +++ | +++ | ++++ |
| **17** | ++++ | ++++ | +++ | +++ | +++ | ++++ |
| **18** | ++++ | ++++ | +++ | +++ | +++ | ++++ |
| **19** | ++++ | ++++ | +++ | +++ | ++++ | ++++ |
| **20** | ++++ | ++++ | +++ | +++ | ++++ | ++++ |
| **21** | ++++ | ++++ | +++ | +++ | +++ | ++++ |
| **22** | ++++ | ++++ | ++++ | ++++ | ++++ | ++++ |
| **23** | ++++ | ++++ | +++ | +++ | +++ | ++++ |
| **24** | +++ | +++ | +++ | +++ | +++ | ++++ |
| **25** | +++ | +++ | +++ | +++ | +++ | +++ |
| **26** | +++ | +++ | +++ | +++ | +++ | +++ |
| **27** | +++ | +++ | +++ | +++ | +++ | ++++ |
| **28** | +++ | +++ | ++++ | +++ | ++++ | ++++ |
| **29** | ++++ | ++++ | ++++ | +++ | ++++ | ++++ |
| **30** | ++++ | ++++ | ++++ | +++ | ++++ | ++++ |
| **31** | ++++ | ++++ | +++ | +++ | +++ | ++++ |
| **32** | ++++ | ++++ | +++ | +++ | +++ | ++++ |
| **33** | ++++ | ++++ | +++ | +++ | +++ | ++++ |
| **34** | ++++ | ++++ | +++ | +++ | +++ | ++++ |
| **35** | +++ | ++++ | +++ | +++ | +++ | ++++ |
| **36** | ++++ | ++++ | +++ | +++ | +++ | ++++ |
| **37** | ++++ | ++++ | +++ | +++ | +++ | ++++ |
| **38** | ++++ | ++++ | +++ | +++ | +++ | ++++ |
| **39** | +++ | +++ | +++ | +++ | +++ | +++ |
| **40** | +++ | ++++ | +++ | +++ | +++ | ++++ |
| **41** | +++ | +++ | +++ | +++ | +++ | ++++ |
| **42** | ++++ | ++++ | +++ | +++ | ++++ | ++++ |
| **43** | +++ | ++++ | +++ | +++ | +++ | ++++ |
| **44** | ++++ | ++++ | +++ | +++ | ++++ | ++++ |
| **45** | +++ | +++ | +++ | +++ | +++ | +++ |
| **46** | +++ | ++++ | +++ | +++ | +++ | ++++ |
| **47** | ++++ | +++ | +++ | +++ | +++ | +++ |
| **48** | +++ | ++++ | +++ | +++ | +++ | ++++ |
| **49** | ++++ | ++++ | ++++ | ++++ | +++ | ++++ |
| **50** | +++ | ++++ | +++ | +++ | +++ | +++ |
| **51** | ++++ | ++++ | +++ | +++ | +++ | ++++ |
| **52** | +++ | +++ | +++ | +++ | +++ | +++ |
| **53** | ++++ | ++++ | +++ | +++ | +++ | ++++ |
| **54** | ++++ | ++++ | +++ | +++ | +++ | ++++ |
| **55** | ++++ | ++++ | ++++ | ++++ | +++ | ++++ |
| **56** | ++++ | ++++ | +++ | +++ | ++++ | ++++ |
| **57** | ++++ | ++++ | +++ | +++ | ++++ | ++++ |
| **58** | ++++ | ++++ | +++ | +++ | ++++ | ++++ |
| **59** | ++++ | ++++ | ++++ | ++++ | ++++ | ++++ |
| **60** | ++++ | ++++ | ++++ | ++++ | ++++ | ++++ |
| **61** | ++++ | ++++ | ++++ | ++++ | ++++ | ++++ |
| **62** | ++++ | ++++ | ++++ | ++++ | ++++ | +++++ |
| **63** | ++++ | ++++ | ++++ | ++++ | ++++ | ++++ |
| **64** | ++++ | ++++ | ++++ | ++++ | ++++ | ++++ |
| **65** | ++++ | ++++ | ++++ | ++++ | ++++ | ++++ |
| **66** | ++++ | +++ | +++ | +++ | +++ | +++ |
| **67** | ++++ | +++ | +++ | +++ | +++ | ++++ |
| **68** | ++++ | +++ | +++ | +++ | +++ | ++++ |
| **69** | ++++ | +++ | +++ | +++ | +++ | ++++ |
| **70** | ++++ | ++++ | ++++ | ++++ | ++++ | ++++ |
| **71** | ++++ | ++++ | ++++ | +++ | ++++ | ++++ |
| **72** | ++++ | ++++ | ++++ | +++ | ++++ | ++++ |
| **73** | ++++ | ++++ | ++++ | +++ | ++++ | ++++ |
| **74** | ++++ | ++++ | ++++ | +++ | ++++ | ++++ |
| **75** | +++ | ++ | ++ | +++ | +++ | ++ |
| Tazobactam | ++ | ++ | ++ | ++ | ++ | ++ |
| Avibactam | ++ | ++ | ++ | ++ | ++ | ++ |
| Taniborbactam | ++++ | ++++ | +++ | +++ | ++++ | +++++ |

| | | | | | | |
|---|---|---|---|---|---|---|
| *^{a}* Note: +++++ indicates IC₅₀ < 10nM, ++++ indicates 10nM < IC₅₀ < 1 µM, +++ indicates 1 µM < IC₅₀ < 100 µM, and ++ indicates IC₅₀ > 100 µM. | | | | | | |

**Table 2 Inhibitory activity of compounds of the present disclosure on SBL^{α}**

| Number of compound | KPC-2 | TEM-1 | SHV-12 | CTX-M-14 | AmpC | OXA-48 |
|---|---|---|---|---|---|---|
| **1** | +++++ | ++++ | ++++ | ++++ | +++ | +++++ |
| **2** | +++++ | ++++ | ++++ | ++++ | +++ | ++++ |
| **3** | +++++ | ++++ | ++++ | ++++ | +++ | ++++ |
| **4** | +++++ | ++++ | ++++ | ++++ | +++ | ++++ |
| **5** | +++++ | ++++ | ++++ | ++++ | +++ | ++++ |
| **6** | +++++ | ++++ | ++++ | ++++ | +++ | ++++ |
| **7** | ++++ | ++++ | +++ | ++++ | +++ | ++++ |
| **8** | ++++ | ++++ | +++ | ++++ | +++ | ++++ |
| **9** | +++++ | ++++ | +++ | ++++ | ++ | +++++ |
| **10** | +++++ | ++++ | +++ | ++++ | ++ | +++++ |
| **11** | +++++ | ++++ | +++ | ++++ | ++ | +++++ |
| **12** | +++++ | ++++ | +++ | ++++ | ++ | +++++ |
| **13** | +++++ | ++++ | ++++ | +++++ | +++ | +++++ |
| **14** | +++++ | ++++ | ++++ | +++++ | +++ | +++++ |
| **15** | +++++ | ++++ | ++++ | ++++ | +++ | ++++ |
| **16** | +++++ | ++++ | ++++ | +++++ | +++ | +++++ |
| **17** | +++++ | +++++ | ++++ | ++++ | +++ | +++++ |
| **18** | +++++ | +++++ | ++++ | ++++ | +++ | +++++ |
| **19** | +++++ | +++++ | ++++ | ++++ | +++ | +++++ |
| **20** | +++++ | +++++ | ++++ | ++++ | +++ | +++++ |
| **21** | +++++ | ++++ | ++++ | ++++ | +++ | +++++ |
| **22** | +++++ | ++++ | ++++ | ++++ | +++ | +++++ |
| **23** | +++++ | ++++ | ++++ | ++++ | +++ | +++++ |
| **24** | +++++ | ++++ | ++++ | ++++ | +++ | +++++ |
| **25** | +++++ | ++++ | ++++ | ++++ | +++ | ++++ |
| **26** | +++++ | ++++ | ++++ | ++++ | +++ | ++++ |
| **27** | +++++ | ++++ | +++ | ++++ | +++ | +++++ |
| **28** | +++++ | ++++ | +++ | ++++ | +++ | ++++ |
| **29** | +++++ | ++++ | +++ | ++++ | +++ | ++++ |
| **30** | +++++ | ++++ | +++ | ++++ | +++ | ++++ |
| **31** | +++++ | ++++ | ++++ | +++++ | +++ | +++++ |
| **32** | +++++ | ++++ | ++++ | +++++ | +++ | +++++ |
| **33** | +++++ | ++++ | +++ | ++++ | +++ | +++++ |
| **34** | +++++ | +++++ | ++++ | +++++ | +++ | +++++ |
| **35** | ++++ | +++++ | ++++ | ++++ | +++ | +++++ |
| **36** | +++++ | +++++ | ++++ | ++++ | +++ | +++++ |
| **37** | +++++ | +++++ | +++ | +++++ | +++ | +++++ |
| **38** | ++++ | ++++ | +++ | ++++ | +++ | +++++ |
| **39** | +++++ | ++++ | +++ | ++++ | +++ | ++++ |
| **40** | +++++ | +++++ | ++++ | ++++ | +++ | ++++ |
| **41** | +++++ | ++++ | ++++ | ++++ | +++ | ++++ |
| **42** | +++++ | +++++ | ++++ | ++++ | +++ | ++++ |
| **43** | +++++ | ++++ | +++ | ++++ | +++ | ++++ |
| **44** | ++++ | ++++ | ++++ | ++++ | +++ | ++++ |
| **45** | ++++ | ++++ | +++ | ++++ | +++ | ++++ |
| **46** | +++++ | ++++ | +++ | +++++ | +++ | ++++ |
| **47** | ++++ | ++++ | +++ | ++++ | +++ | ++++ |
| **48** | +++++ | ++++ | ++++ | ++++ | +++ | ++++ |
| **49** | +++++ | ++++ | ++++ | ++++ | +++ | ++++ |
| **50** | +++++ | ++++ | ++++ | ++++ | +++ | ++++ |
| **51** | +++++ | ++++ | ++++ | ++++ | +++ | +++++ |
| **52** | +++ | +++ | +++ | +++ | +++ | +++ |
| **53** | +++++ | ++++ | ++++ | ++++ | +++ | +++++ |
| **54** | ++++ | +++ | ++++ | ++++ | +++ | ++++ |
| **55** | +++++ | +++++ | ++++ | +++++ | +++ | ++++ |
| **56** | +++++ | +++++ | ++++ | ++++ | +++ | +++++ |
| **57** | +++++ | +++++ | ++++ | +++++ | +++ | +++++ |
| **58** | +++++ | +++++ | ++++ | ++++ | +++ | +++++ |
| **59** | +++++ | +++++ | ++++ | +++++ | ++++ | +++++ |
| **60** | +++++ | +++++ | ++++ | ++++ | ++++ | +++++ |
| **61** | +++++ | ++++ | ++++ | ++++ | ++++ | +++++ |
| **62** | +++++ | ++++ | ++++ | +++++ | ++++ | +++++ |
| **63** | +++++ | ++++ | ++++ | ++++ | ++++ | +++++ |
| **64** | +++++ | ++++ | ++++ | ++++ | ++++ | +++++ |
| **65** | +++++ | ++++ | ++++ | ++++ | +++ | ++++ |
| **66** | +++++ | ++++ | +++ | ++++ | ++ | +++++ |
| **67** | +++++ | ++++ | +++ | ++++ | ++ | +++++ |
| **68** | +++ | +++ | +++ | +++ | +++ | +++ |
| **69** | +++ | +++ | +++ | +++ | +++ | +++ |
| **70** | +++++ | +++++ | +++++ | ++++ | ++++ | ++++ |
| **71** | +++++ | +++++ | +++++ | ++++ | +++ | ++++ |
| **72** | +++++ | +++++ | +++++ | ++++ | +++ | ++++ |
| **73** | +++++ | +++++ | +++++ | ++++ | ++++ | ++++ |
| **74** | +++++ | +++++ | +++++ | ++++ | ++++ | ++++ |
| **75** | ++++ | +++ | +++ | +++ | +++ | +++ |
| Tazobactam | +++ | ++++ | +++++ | +++++ | +++ | +++ |
| Avibactam | ++++ | +++++ | +++++ | +++++ | ++++ | ++++ |
| Taniborbactam | ++++ | ++++ | ++++ | ++++ | ++++ | ++++ |

| | | | | | | |
|---|---|---|---|---|---|---|
| *^{a}* Note: +++++ indicates IC₅₀ < 10nM, ++++ indicates 10nM < IC₅₀ < 1 µM, +++ indicates 1 µM < IC₅₀ < 100 µM, and ++ indicates IC₅₀> 100 µM. | | | | | | |

As can be seen from the above experimental results, the compounds of the present disclosure exhibited a good and broad-spectrum inhibitory activity on clinically important MBLs, including NDM-1, NDM-5, IMP-1, IMP-4, VIM-1 and VIM-2. Among them, compound 62 exhibited an inhibitory activity of less than 10 nM on VIM-2, compounds 1, 2, 3, 4, 10, 11, 12, 13, 14, 15, 16, 17, 18, 19, 20, 21, 22, 23, 29, 30, 31, 32, 33, 34, 36, 37, 38, 42, 44, 47, 49, 51, 53, 54, 55, 56, 57, 58, 59, 60, 61, 62, 63, 64, 65, 70, 71, 72, 73 and 74 exhibited an inhibitory activity at a nanomolar-level (less than 1 µM) on NDM-1 and NDM-5, compounds 4, 22, 28, 29, 30, 49, 55, 59, 60, 61, 62, 63, 64, 65 and 70 exhibited an inhibitory activity at a nanomolar-level (less than 1 µM) on IMP-1 and/or IMP-4, and compounds 1, 2, 3, 5, 6, 8, 9, 10, 11, 12, 13, 14, 15, 16, 17, 18, 19, 20, 21, 22, 23, 24, 27, 28, 29, 30, 42, 44, 56, 57, 58, 59, 60, 61, 62, 63, 64, 65, 70, 71, 72, 73 and 74 exhibited an inhibitory activity at a nanomolar-level (less than 1 µM) on VIM-1 and/or VIM-2. The compounds of the present disclosure exhibited better inhibitory activity on the tested MBLs compared to the existing drugs such as tazobactam and avibactam, several compounds of the present disclosure exhibited inhibitory activity on MBL comparable to taniborbactam, which is currently in clinical evaluation.

Similarly, the compounds of the present disclosure exhibit a good and broad-spectrum inhibitory activity on clinically important SBLs, including KPC-2, TEM-1, SHV-12, CTX-M-14, AmpC and OXA-48. All compounds of the present disclosure exhibited a better inhibitory activity at a nanomolar-level (less than 1 µM) on KPC-2, TEM-1, CTX-M-14 and OXA-48, compared to the control drugs tazobactam and taniborbactam. Some of these compounds (e.g., 17, 18, 19, 59, 60, 61, 62, 63, 64, 70, 73, and 74) exhibited a better or comparable inhibitory activity on these four enzymes compared to avibactam. Although compounds 1-69 and 75 exhibited an inhibitory activity on SHV-12 lower than that of avibactam and tazobactam, the inhibitory activity of most of them was comparable to that of taniborbactam. Compounds 70, 71, 72, 73, and 74 exhibited an inhibitory activity on SHV-12 that was comparable to avibactam and tazobactam and superior to taniborbactam. Although compounds 1-58 exhibited an inhibitory activity on AmpC lower than that of avibactam and taniborobactam, the inhibitory activity of most of them was comparable to that of tazobactam. Compounds 59, 60, 61, 62, 63, 64, 70, 73 and 74 exhibited an inhibitory activity on AmpC that was comparable to that of avibactam and taniborobactam, and superior to tazobactam.

In summary, the compounds of the present disclosure have good inhibitory effects on both MBL and SBL, and can be used to prepare inhibitors with dual efficacy against MBL and SBL.

### Experimental example 2 Antibacterial activity of compounds of the present disclosure in combination with meropenem

### 1. Experimental methods

(1) Resuscitation of Strains: The strains stored at a -80°C refrigerator was resuscitated, streaked onto a non-resistant LB plate with an inoculating loop, and cultured at 37°C for 18-20 h.
(2) Dilution of Drug Solution: The meropenem solid was weighed and dissolved in DMSO to prepare a stock solution of 12.8 mg/ml. The stock solution was diluted with CAMHB medium to a 512 µg/mL working solution. 100 µL of CAMHB was added to a clear 96-well plate, and 100 µL of meropenem working solution was added. The working solution was 2-fold diluted to obtain 9 concentrations, and control wells without meropenem were set up. 2 µL of compounds at a concentration of 1 mM per well was added.
(3) Dilution of Bacterial Solution: A single colony was picked from the plate with an inoculating loop and suspended in physiological saline. The bacterial solution was adjusted to an OD600 of 0.08-0.13 (equivalent to 1 × 108 CFU/mL), diluted 100-fold with CAMHB, and added to wells (100 µL/well). At this time, the concentration of meropenem was 128 µg/mL to 0.25 µg/mL, and the concentration of the enzyme inhibitor was 10 µM.
(4) Static Incubation and Result Analysis: The 96-well plate was placed in the incubator at 37°C and statically incubated for 16-20 h. The experimental results were observed on the next day. The minimum concentration of meropenem that completely or significantly inhibits bacterial growth is defined as the Minimum Inhibitory Concentration (MIC) of meropenem.

### 2. Experimental results

The antibacterial activity of the compounds of the present disclosure in combination with meropenem against clinically isolated strains is shown in Table 3.

**Table 3 Antibacterial activity of compounds of the present disclosure in combination with meropenem against clinically isolated strains ^{a}**

| Number of compound | MIC value of compounds in combination with meropenem MIC (µg/ml) | | | | | |
|---|---|---|---|---|---|---|
| | *KP140912* (bla_{KPC-2}) | *KP090344* (bla_{KPC-2}) | *KP140508* (bla_{KPC-2}) | *EC1541* (bla_{IMP-1}) | *KP 090571* (bla_{NDM-1}) | *KP 140509* (bla_{NDM-1}) |
| No compound | ++ | ++ | +++ | +++ | ++ | ++ |
| **1** | ++++ | ++++ | ++++ | +++ | ++++ | ++++ |
| **2** | ++ | ++ | ++++ | ++++ | ++++ | ++++ |
| **3** | ++++ | ++++ | ++++ | +++ | ++++ | ++++ |
| **4** | ++ | ++ | ++++ | ++++ | +++ | ++++ |
| **7** | ++++ | ++++ | ++++ | +++ | +++ | ++++ |
| **8** | ++++ | ++++ | ++++ | ++++ | ++++ | ++++ |
| **9** | ++++ | ++++ | ++++ | ++++ | ++++ | ++++ |
| **10** | ++++ | ++++ | ++++ | ++++ | ++++ | ++++ |
| **13** | ++++ | ++++ | ++++ | ++++ | ++++ | ++++ |
| **14** | ++++ | ++++ | ++++ | ++++ | ++++ | ++++ |
| **15** | ++ | ++ | ++++ | +++ | ++ | ++++ |
| **16** | ++++ | +++ | ++++ | ++++ | +++ | ++++ |
| **17** | ++++ | ++++ | ++++ | ++++ | ++++ | ++++ |
| **21** | ++++ | ++++ | ++++ | ++++ | ++++ | ++++ |
| **22** | ++++ | ++++ | ++++ | ++++ | ++++ | ++++ |
| **23** | ++++ | ++++ | ++++ | ++++ | ++++ | ++++ |
| **24** | ++++ | ++++ | ++++ | +++ | ++ | ++++ |
| **27** | ++ | ++ | ++++ | ++++ | +++ | ++++ |
| **28** | ++ | ++ | ++++ | ++++ | ++ | ++++ |
| **31** | ++++ | ++++ | ++++ | ++++ | +++ | ++++ |
| **32** | +++ | ++ | ++++ | ++++ | +++ | ++++ |
| **33** | ++++ | ++ | ++++ | ++++ | ++++ | ++++ |
| **34** | ++++ | +++ | ++++ | ++++ | ++++ | ++++ |
| **35** | ++++ | +++ | ++++ | ++++ | ++++ | ++++ |
| **36** | ++++ | ++++ | ++++ | ++++ | ++++ | ++++ |
| **37** | ++++ | ++++ | ++++ | ++++ | ++++ | ++++ |
| **38** | ++++ | ++++ | ++++ | ++++ | ++++ | ++++ |
| **39** | ++++ | ++++ | ++++ | +++ | +++ | ++++ |
| **40** | ++++ | ++++ | ++++ | ++++ | ++++ | ++++ |
| **41** | ++++ | ++++ | ++++ | +++ | +++ | +++ |
| **42** | ++++ | ++++ | ++++ | ++++ | ++++ | +++ |
| **43** | ++++ | ++++ | ++++ | ++++ | ++++ | +++ |
| **44** | ++ | ++ | ++ | ++++ | +++ | ++++ |
| **45** | ++++ | ++++ | ++++ | +++ | ++ | ++ |
| **46** | +++ | ++ | ++++ | ++++ | ++++ | ++++ |
| **47** | ++ | ++ | ++++ | ++++ | ++++ | ++++ |
| **48** | ++++ | ++++ | ++++ | ++++ | ++++ | ++++ |
| **49** | ++++ | ++++ | ++++ | ++++ | ++++ | ++++ |
| **50** | ++++ | ++++ | ++++ | ++++ | ++++ | ++++ |
| **51** | +++ | +++ | ++++ | ++++ | ++++ | ++++ |
| **52** | +++ | ++ | ++ | ++ | ++ | ++ |
| **53** | ++++ | +++ | +++ | +++ | +++ | +++ |
| **54** | ++++ | +++ | +++ | +++ | ++++ | ++++ |
| **55** | +++ | ++ | ++++ | ++++ | +++ | ++++ |
| **56** | ++++ | ++++ | ++++ | ++++ | ++++ | ++++ |
| **57** | ++++ | ++++ | ++++ | ++++ | ++++ | ++++ |
| **58** | ++++ | ++++ | ++++ | ++++ | ++++ | ++++ |
| **59** | +++ | ++ | ++++ | ++++ | +++ | ++++ |
| **60** | +++ | ++ | ++++ | ++++ | +++ | ++++ |
| **61** | +++ | ++ | ++++ | ++++ | +++ | ++++ |
| **62** | +++ | ++ | ++++ | ++++ | +++ | ++++ |
| **63** | ++++ | ++++ | ++++ | ++++ | ++++ | ++++ |
| **64** | ++++ | ++++ | ++++ | ++++ | ++++ | ++++ |
| **65** | +++ | ++++ | ++++ | ++++ | ++++ | +++ |
| **66** | ++++ | ++++ | ++++ | ++++ | ++ | +++ |
| **67** | ++++ | ++++ | ++++ | ++++ | ++ | +++ |
| **68** | ++ | ++ | ++++ | +++ | +++ | ++++ |
| **69** | ++ | ++ | ++++ | +++ | +++ | +++ |
| **70** | ++++ | ++++ | ++++ | ++++ | ++++ | ++++ |
| **71** | ++++ | +++ | ++++ | ++++ | +++ | ++++ |
| **72** | ++ | ++ | ++++ | ++++ | +++ | +++ |
| **73** | ++++ | +++ | +++ | +++ | +++ | +++ |
| **74** | ++++ | +++ | +++ | +++ | +++ | +++ |
| **75** | ++++ | +++ | +++ | ++ | +++ | ++ |
| Avibactam | ++++ | ++++ | ++++ | +++ | ++ | ++ |
| Taniborbactam | ++++ | ++++ | ++++ | ++++ | ++++ | ++++ |

| | | | | | | |
|---|---|---|---|---|---|---|
| *^{a}* Note: ++++ indicates MIC< 4µg/ml, +++ indicates 4µg/ml < MIC ≤ 16 µg/ml, and ++ indicates MIC >16 µg/ml. The compounds have a final concentration of 10µM. | | | | | | |

The experimental results above demonstrated that some of the compounds in the present disclosure, when used in combination with meropenem, exhibited a good antibacterial activity against clinically isolated drug-resistant strains such as *Klebsiella pneumoniae* expressing KPC-2 and NDM-1, and *Escherichia coli* expressing IMP-1. For example, compounds 1, 2, 7, 8, 9, 10, 13, 14, 16, 17, 21, 22, 23, 24, 31, 36, 37, 38, 39, 40, 41, 42, 43, 45, 48, 49, 50, 56, 57, 58, 63, 64, 66, 67 and 70, when combined with meropenem, exhibited MIC values of ≤ 4 µg/mL for the tested clinically isolated drug-resistant strains. These compounds exhibited antibacterial activity against *Klebsiella pneumoniae* expressing KPC-2 comparable to the control drugs avibactam and taniborbactam, while the inhibitory activity against *Escherichia coli* expressing IMP-1 and *Klebsiella pneumoniae* expressing NDM-1 was significantly superior to avibactam and comparable to taniborbactam.

In summary, the present disclosure provides a class of 7-((five-membered heterocyclic)methyl)-8-carboxylic acid-benzo cyclic borate derivatives. The derivative provided by the present disclosure has good broad-spectrum inhibitory activity on clinically common metallo-β-lactamase (MBLs) and serine-β-lactamase (SBLs), can be used as an inhibitor of MBL and/or SBL, and is used for preparing antibacterial drugs, especially drugs against drug-resistant bacteria. In addition, the derivative of the present disclosure combined with β-lactam antibiotics has good antibacterial activity on various β-lactam antibiotic-resistant bacteria. The derivative of the present disclosure has a very great potential in preparing MBL and SBL dual broad-spectrum inhibitors and drugs overcoming β-lactam antibiotic-resistant bacteria. The derivative of the present disclosure provides a new choice for the use of the antibacterial drugs, and has a good application prospect.

## Claims

1. A compound represented by formula I, or a salt, a conformational isomer or an optical isomer thereof, 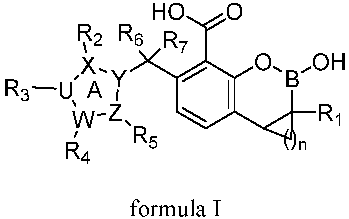 wherein,
n is an integer of 0 to 5,
R₁ is selected from the group consisting of hydrogen, substituted or unsubstituted C₁-C₈ alkyl, C₁-C₈ alkoxy, halogen, hydroxyl, amino, nitro, carboxyl, cyano and SR₉,
A ring is a five-membered unsaturated heterocyclic ring, wherein X, Y, Z, W and U are independently selected from the group consisting of C, CR₈ and N,
R₂, R₃, R₄ and R₅ are substituents on ring A, and R₂, R₃, R₄ and R₅ are independently selected from the group consisting of absence, hydrogen, halogen, hydroxyl, amino, nitro, carboxyl, cyano, amido, quaternary ammonium salt, substituted or unsubstituted C₁-C₈ alkyl, substituted or unsubstituted C₂-C₈ alkynyl, C₁-C₈ alkoxy, substituted or unsubstituted 3- to 8-membered cycloalkyl, substituted or unsubstituted 4- to 8-membered heterocycloalkyl, substituted or unsubstituted 5- to 8-membered aryl, substituted or unsubstituted 5- to 8-membered heteroaryl, and -COR₁₀,
alternatively, any two adjacent positions in R₂, R₃, R₄, and R₅ are connected to form substituted or unsubstituted 3- to 8-membered cycloalkyl, substituted or unsubstituted 4- to 8-membered heterocycloalkyl, substituted or unsubstituted 5- to 8-membered aryl, or substituted or unsubstituted 5- to 8-membered heteroaryl,
R₆ and R₇ are independently selected from the group consisting of hydrogen, substituted or unsubstituted C₁-C₈ alkyl, C₁-C₈ alkoxy, halogen, hydroxyl, amino, nitro, carboxyl, and cyano,
R₈ is selected from the group consisting of hydrogen, halogen, hydroxyl, amino, nitro, carboxyl, cyano, amido, quaternary ammonium salt, substituted or unsubstituted C₁-C₈ alkyl, C₁-C₈ alkoxy, substituted or unsubstituted 3- to 8-membered cycloalkyl, substituted or unsubstituted 4- to 8-membered heterocycloalkyl, substituted or unsubstituted 5- to 8-membered aryl, and substituted or unsubstituted 5- to 8-membered heteroaryl,
R₉ is a five-membered unsaturated heterocyclic group,
R₁₀ is selected from the group consisting of amino, 4- to 8-membered heterocycloalkyl and amino-substituted 4- to 8-membered heterocycloalkyl,
a substituent on the alkyl is selected from the group consisting of halogen, hydroxyl, amino, nitro, carboxyl, cyano, guanidino, quaternary ammonium salt, sulfonamido, and substituted or unsubstituted 4- to 8-membered heterocycloalkyl;
a substituent on the alkynyl, the cycloalkyl, the heterocycloalkyl, the aryl, and the heteroaryl is selected from the group consisting of C₁-C₈ alkyl, C₁-C₈ aliphatic amine group, C₁-C₈ alkoxy, halogen, hydroxyl, amino, nitro, carboxyl, cyano, guanidino, and sulfonamido,
a heteroatom on the heterocycloalkyl and the heteroaryl is selected from the group consisting of N, O and S, and a number of the heteroatom is selected from the group consisting of 1, 2, 3, 4 and 5, and
one or more hydrogen atoms on the amino can be further substituted with a substituent selected from the group consisting of C₁-C₈ alkyl, C₁-C₈ aliphatic amine group and sulfonamido.

2. The compound, or the salt, the conformational isomer or the optical isomer thereof according to claim 1, wherein
n is selected from the group consisting of 0, 1, 2, 3, 4 and 5,
R₁ is selected from the group consisting of hydrogen, C₁-C₄ alkyl, C₁-C₄ alkoxy, halogen, hydroxyl, amino, nitro, carboxyl, cyano and SR₉,
A ring is a five-membered unsaturated heterocyclic ring, wherein X is selected from the group consisting of C, CR₈ and N, Y is N, Z is selected from the group consisting of C, CR₈ and N, W is selected from the group consisting of C and N, and U is selected from the group consisting of C and N,
R₂, R₃, R₄ and R₅ are substituents on ring A, and R₂, R₃, R₄ and R₅ are independently selected from the group consisting of absence, hydrogen, halogen, hydroxyl, amino, nitro, carboxyl, cyano, amido, quaternary ammonium salt, substituted or unsubstituted C₁-C₄ alkyl, substituted or unsubstituted C₂-C₄ alkynyl, C₁-C₄ alkoxy, substituted or unsubstituted 3- to 6-membered cycloalkyl, substituted or unsubstituted 4- to 6-membered heterocycloalkyl, substituted or unsubstituted 5- to 6-membered aryl, substituted or unsubstituted 5- to 6-membered heteroaryl, and -COR₁₀,
alternatively, any two adjacent positions in R₂, R₃, R₄, and R₅ are connected to form substituted or unsubstituted 3- to 6-membered cycloalkyl, substituted or unsubstituted 4- to 6-membered heterocycloalkyl, substituted or unsubstituted 5- to 6-membered aryl, or substituted or unsubstituted 5- to 6-membered heteroaryl,
R₆ and R₇ are independently selected from the group consisting of hydrogen, substituted or unsubstituted C₁-C₄ alkyl, C₁-C₄ alkoxy, halogen, hydroxyl, amino, nitro, carboxyl, and cyano,
R₈ is selected from the group consisting of hydrogen, halogen, hydroxyl, amino, nitro, carboxyl, cyano, amido, quaternary ammonium salt, substituted or unsubstituted C₁-C₄ alkyl, C₁-C₄ alkoxy, substituted or unsubstituted 3- to 6-membered cycloalkyl, substituted or unsubstituted 4- to 6-membered heterocycloalkyl, substituted or unsubstituted 5- to 6-membered aryl, and substituted or unsubstituted 5- to 6-membered heteroaryl,
R₉ is a five-membered unsaturated heterocyclic group, wherein a heteroatom on the heterocyclic group is selected from the group consisting of N, O, S, and a combination thereof,
R₁₀ is selected from the group consisting of amino, 4- to 5-membered heterocycloalkyl and amino-substituted 4- to 5-membered heterocycloalkyl,
a substituent on the alkyl is selected from the group consisting of halogen, hydroxyl, amino, nitro, carboxyl, cyano, guanidino, quaternary ammonium salt, sulfonamido, and substituted or unsubstituted 4- to 6-membered heterocycloalkyl;
a substituent on the alkynyl, the cycloalkyl, the heterocycloalkyl, the aryl, and the heteroaryl is selected from the group consisting of C₁-C₄ alkyl, C₁-C₄ aliphatic amine group, C₁-C₄ alkoxy, halogen, hydroxyl, amino, nitro, carboxyl, cyano, guanidino, and sulfonamido,
a heteroatom on the heterocycloalkyl and the heteroaryl is selected from the group consisting of N, O and S, and a number of the heteroatom is 1 or 2, and
one or more hydrogen atoms of the amino can be further substituted with a substituent selected from the group consisting of C₁-C₄ alkyl, C₁-C₄ aliphatic amine group and sulfonamido.

3. The compound, or the salt, the conformational isomer or the optical isomer thereof according to claim 1, wherein A ring is selected from the group consisting of:

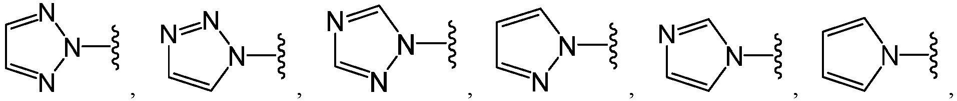

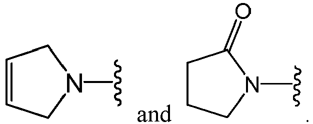

4. The compound, or the salt, the conformational isomer or the optical isomer thereof according to claim 1, wherein the compound has a structure represented by formula II: 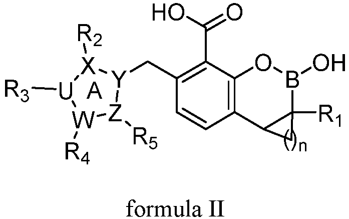 wherein n, A ring, X, Y, Z, W, U, R₁, R₂, R₃, R₄ and R₅ are each selected as defined in any one of claims 1 to 3.

5. The compound, or the salt, the conformational isomer or the optical isomer thereof according to claim 4, wherein the compound has a structure represented by formula III: 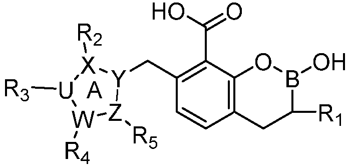 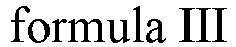
wherein A ring, X, Y, Z, W, U, R₁, R₂, R₃, R₄ and R₅ are each selected as defined in any one of claims 1 to 3;
alternatively, the compound has a structure represented by formula IV: 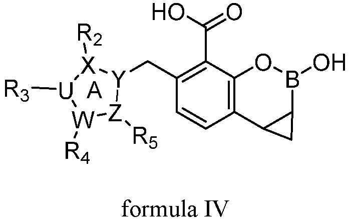
wherein A ring, X, Y, Z, W, U, R₂, R₃, R₄ and R₅ are each selected as defined in any one of claims 1 to 3.

6. The compound, or the salt, the conformational isomer or the optical isomer thereof according to claim 5, wherein the compound has a structure represented by formula V: 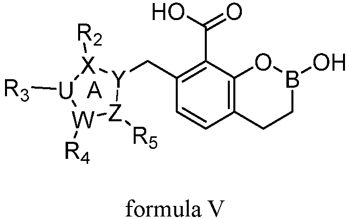 wherein A ring, X, Y, Z, W, U, R₂, R₃, R₄ and R₅ are each selected as defined in any one of claims 1 to 3.

7. The compound, or the salt, the conformational isomer or the optical isomer thereof according to claim 1, wherein the compound has a structure represented by formula VI-1: 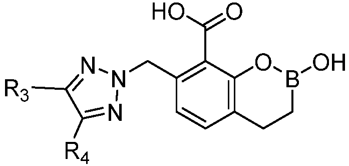 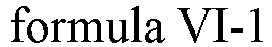
wherein, R₃ and R₄ are each selected as defined in any one of claims 1 to 3;
alternatively, the compound has a structure represented by formula VI-2: 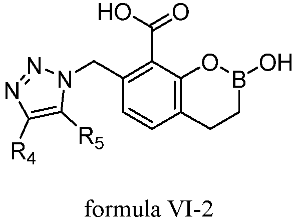
wherein, R₄ and R₅ are each selected as defined in any one of claims 1 to 3;
alternatively, the compound has a structure represented by formula VI-3: 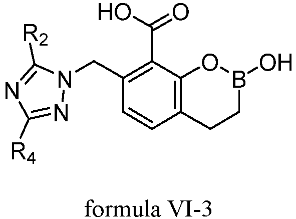
wherein, R₂ and R₄ are each selected as defined in any one of claims 1 to 3;
alternatively, the compound has a structure represented by formula VI-4: 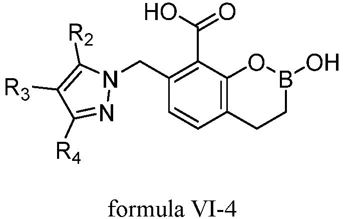
wherein, R₂, R₃ and R₄ are each selected as defined in any one of claims 1 to 3;
alternatively, the compound has a structure represented by formula VI-5: 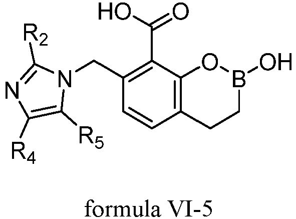
wherein, R₂, R₄ and R₅ are each selected as defined in any one of claims 1 to 3;
alternatively, the compound has a structure represented by formula VI-6: 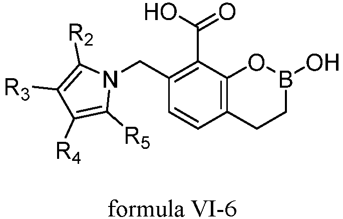
wherein, R₂, R₃, R₄ and R₅ are each selected as defined in any one of claims 1 to 3;
alternatively, the compound has a structure represented by formula VI-7: 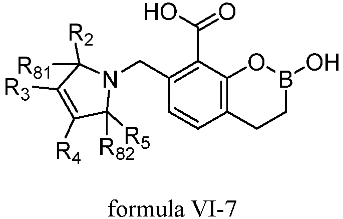
alternatively, the compound has a structure represented by formula VI-8: 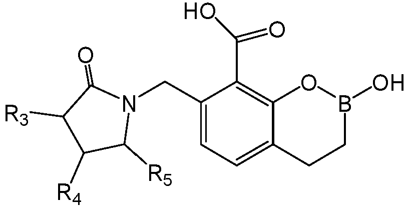 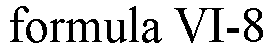
wherein, R₂, R₃, R₄ and R₅ are each selected as defined in any one of claims 1 to 3;
R₈₁ and R₈₂ are independently selected from the group consisting of hydrogen, halogen, hydroxyl, amino, nitro, carboxyl, cyano, amido, quaternary ammonium salt, substituted or unsubstituted C₁-C₄ alkyl, C₁-C₄ alkoxy, substituted or unsubstituted 3- to 6-membered cycloalkyl, substituted or unsubstituted 4- to 6-membered heterocycloalkyl, substituted or unsubstituted 5- to 6-membered aryl, and substituted or unsubstituted 5- to 6-membered heteroaryl;
a substituent on the alkyl is selected from the group consisting of halogen, hydroxyl, amino, nitro, carboxyl, cyano, guanidino, quaternary ammonium salt, and substituted or unsubstituted 4-to 6-membered heterocycloalkyl;
a substituent on the cycloalkyl, the heterocycloalkyl, the aryl, and the heteroaryl is selected from the group consisting of C₁-C₄ alkyl, C₁-C₄ alkoxy, halogen, hydroxyl, amino, nitro, carboxyl, and cyano; and
a heteroatom on the heterocycloalkyl and the heteroaryl is selected from the group consisting of N, O and S, and a number of the heteroatom is 1 or 2.

8. The compound, or the salt, the conformational isomer or the optical isomer thereof according to any one of claims 1 to 7, wherein the compound is selected from the group consisting of:

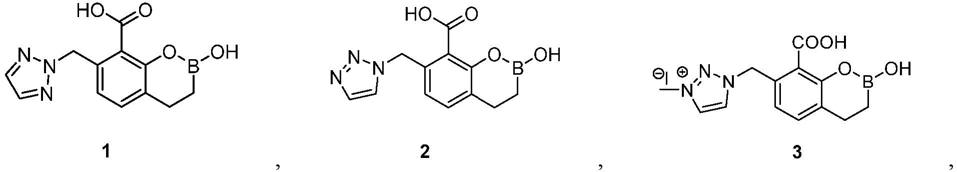

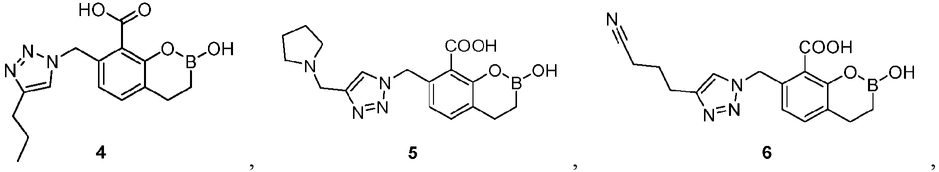

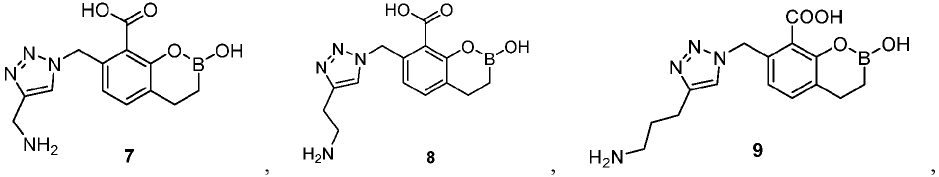

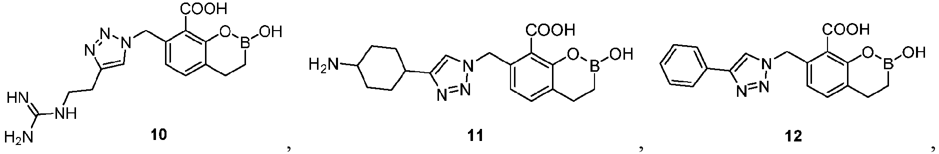

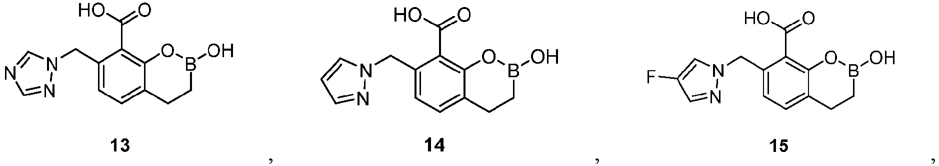

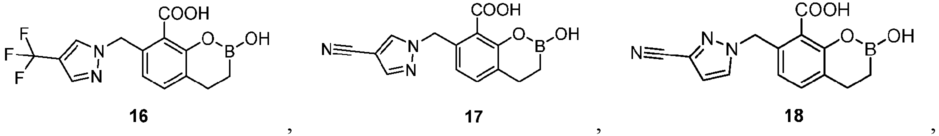

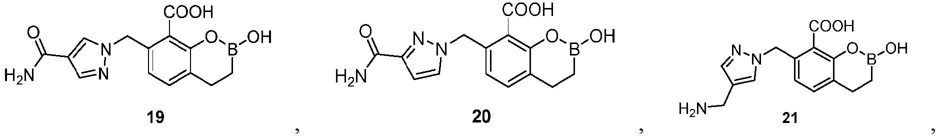

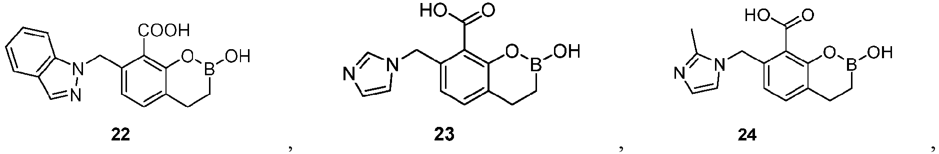

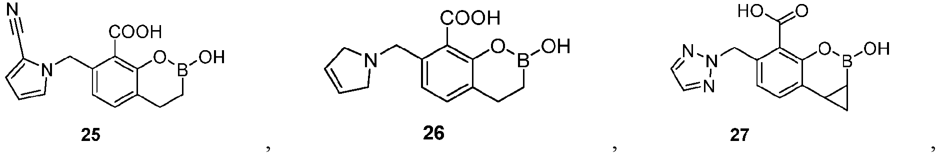

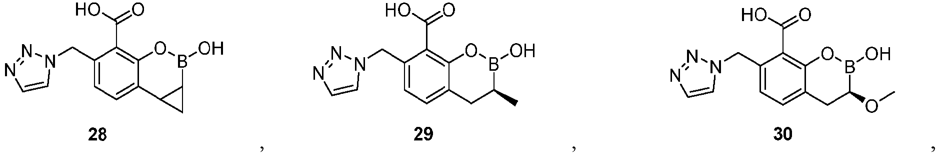

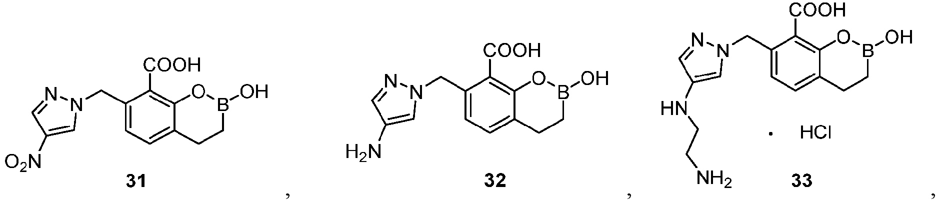

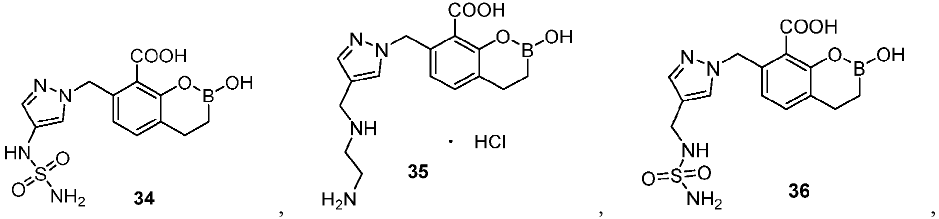

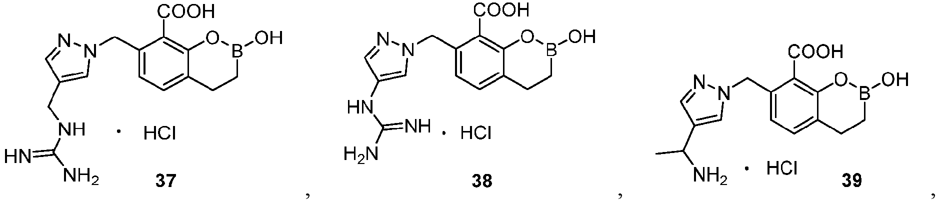

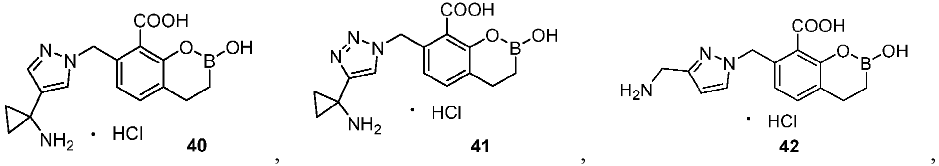

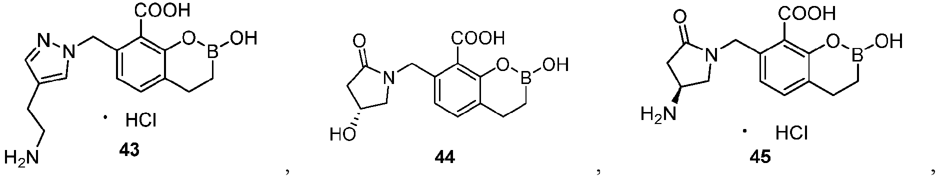

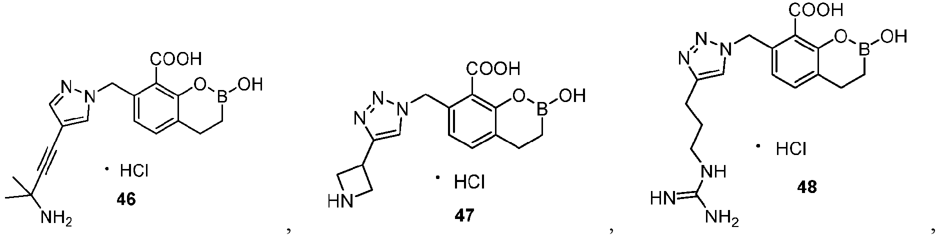

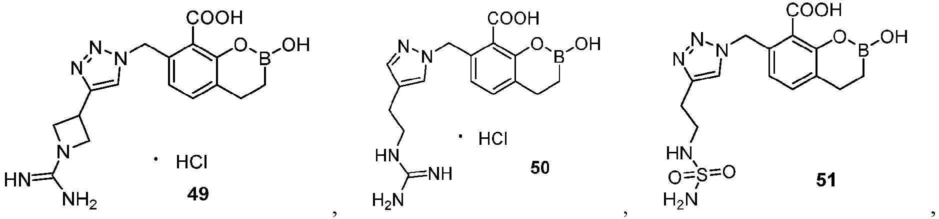

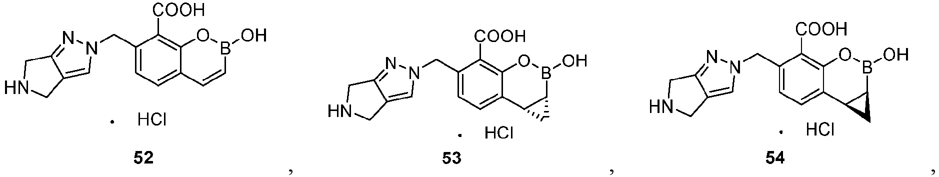

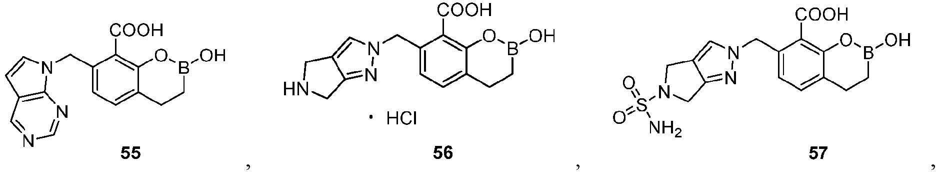

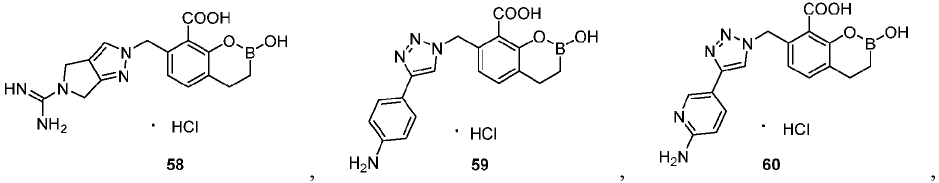

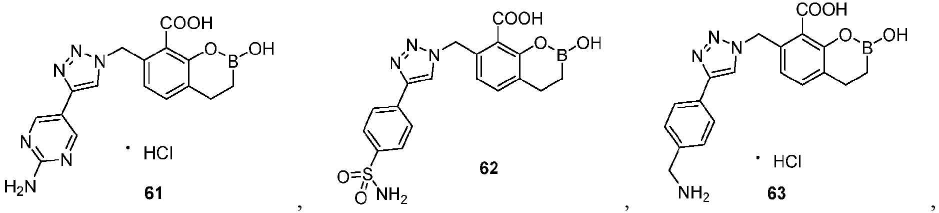

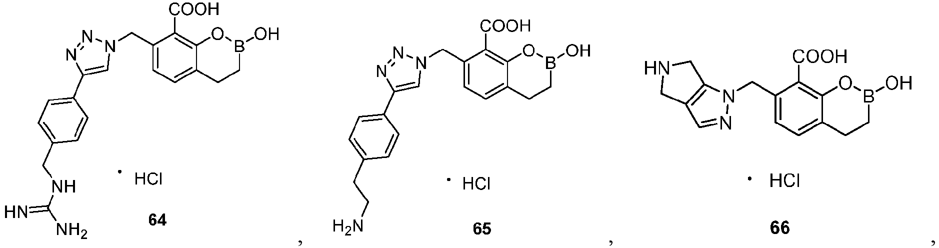

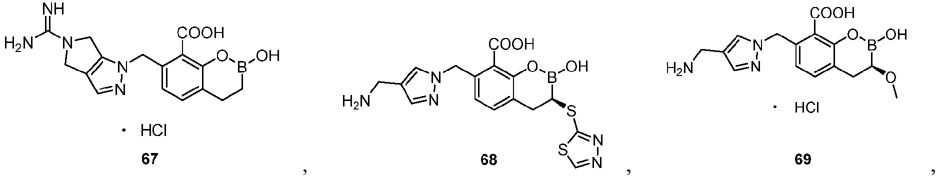

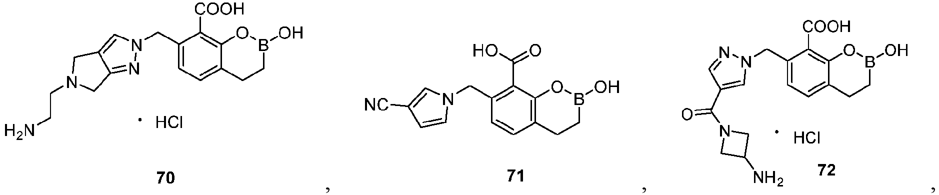

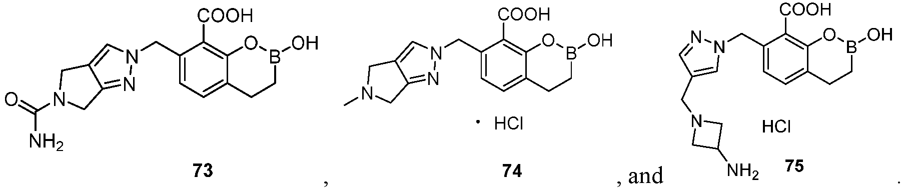

9. Use of the compound, or the salt, the conformational isomer or the optical isomer thereof according to any one of claims 1 to 8 in the manufacture of a metallo-β-lactamase and/or serine-β-lactamase inhibitor.

10. Use of the compound, or the salt, the conformational isomer or the optical isomer thereof according to any one of claims 1 to 8 in the manufacture of an antibacterial medicament,
preferably, the antibacterial medicament is a medicament against drug-resistant bacteria, and
more preferably, the medicament against drug-resistant bacteria is a medicament against β-lactam antibiotic-resistant bacteria.

11. A medicament, which is a formulation obtained by using the compound, or the salt, the conformational isomer or the optical isomer thereof according to any one of claims 1 to 8 as an active ingredient, and a pharmaceutically acceptable excipient or an auxiliary ingredient.

12. A pharmaceutical composition comprising the compound, or the salt, the conformational isomer or the optical isomer thereof according to any one of claims 1 to 8, and an antibiotic,
preferably, the antibiotic is a β-lactam antibiotic, and
more preferably, the antibiotic is selected from the group consisting of meropenem, imipenem, cefepime, and a combination thereof.

13. Use of the compound, or the salt, the conformational isomer or the optical isomer thereof according to any one of claims 1 to 8 in combination with an antibiotic in the manufacture of an antibacterial medicament,
preferably, the antibacterial medicament is a medicament against drug-resistant bacteria, and/or the antibiotic is a β-lactam antibiotic, and
more preferably, the medicament against drug-resistant bacteria is a medicament against β-lactam antibiotic-resistant bacteria, and/or the antibiotic is selected from the group consisting of meropenem, imipenem, cefepime, and a combination thereof.
